(19) Europäisches Patentamt
European Patent Office
Office européen des brevets

(11) Publication number: **0 446 798 B1**

# (12) EUROPEAN PATENT SPECIFICATION

(45) Date of publication of patent specification: **23.11.94**

(51) Int. Cl.5: **C07D 213/28**, C07D 213/38, C07D 213/40, C07D 213/61, C07D 333/20, C07D 333/22, C07D 333/24, C07D 213/48, C07D 213/55, C07D 213/56, C07D 261/08

(21) Application number: **91103556.6**

(22) Date of filing: **08.03.91**

(54) **1-alkyl-, 1-alkenyl- and 1-alkynylaryl-2-amino-1, 3-propanediols and related compounds, a process and intermediates for their preparation and their use as medicaments.**

(30) Priority: **13.03.90 US 492200**
**12.10.90 US 596448**
**24.12.90 US 632910**

(43) Date of publication of application:
**18.09.91 Bulletin 91/38**

(45) Publication of the grant of the patent:
**23.11.94 Bulletin 94/47**

(84) Designated Contracting States:
**AT BE CH DE DK ES FR GB GR IT LI LU NL SE**

(56) References cited:
**EP-A- 0 185 814**
**US-A- 3 871 958**

**Chemical Abstracts, vol. 92, no. 23, June 9, 1980, Columbus, Ohio, USA ; MIYOSHI M. et al. : "Beta-Hydro-xyaminacids", p. 725, col. 2, abstract-no. 198778a & JP-A-79141723**

(73) Proprietor: **HOECHST-ROUSSEL PHARMACEUTICALS INCORPORATED**
**Route 202-206 North**
**Somerville New Jersey 08876 (US)**

(72) Inventor: **Tegeler, John Joseph**
**40 Highland Avenue**
**Bridgewater, NJ 08807 (US)**
Inventor: **Rauckman, Barbara Seavey**
**1 Rowland Road**
**Flemington, NJ 08822 (US)**
Inventor: **Hamer, Russell Richard Lee**
**108A Cokesbury Road**
**Lebanon, NJ 08833 (US)**
Inventor: **Freed, Brian Scott**
**112G Easton Avenue**
**Somerset, NJ 08873 (US)**

EP 0 446 798 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Chemical Abstracts, vol. 89, no. 7, August 14, 1978, Columbus, Ohio, USA ; ARENA F. et al. : "Hydroxyiminomethylalkylpyridine methiodides", p. 568, col. 1, abstract-no. 59826g

(74) Representative: **Losert, Wolfgang Dr. et al**
**HOECHST AKTIENGESELLSCHAFT,**
**Zentrale Patentabteilung,**
**Gebäude F 821**
**D-65926 Frankfurt am Main (DE)**

## Description

The present invention relates to 1-alkyl-, 1-alkenyl-, and 1-alkylnylaryl-2-amino-1,3-propanediols of formula 1

$RCH(OR^1)CH(NR^2R^3)R^4$   1

wherein R is

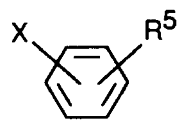 , 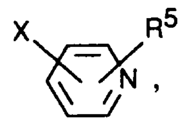 ,

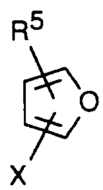 , 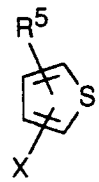 , 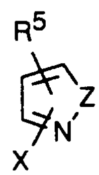 , and 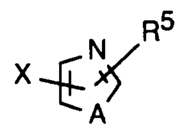

wherein $R^5$ is $CH_3(CH_2)_mC{\equiv}C$, $CH_3(CH_2)_mCH=CH$,$CH_3(CH_2)_mCH_2CH_2$,

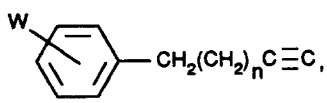

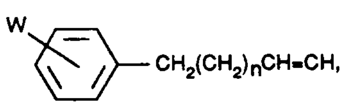 or 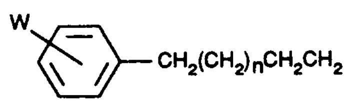

wherein m is 3 to 15, n is 0 to 12, and W and X are independently hydrogen, alkyl, alkoxy, halogen, or trifluoromethyl, Z is S, O, or C=O; and A is S or O; $R^1$ is hydrogen or

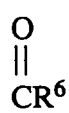

wherein $R^6$ is hydrogen, alkyl, alkoxy, or

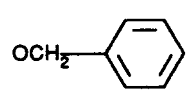 ;

$R^2$ is hydrogen or alkyl; $R^3$ is hydrogen, alkyl or

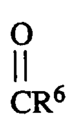

wherein $R^6$ is as above; $R^4$ is

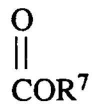

wherein $R^7$ is hydrogen or alkyl, or $CH_2OR^8$ wherein $R^8$ is hydrogen or

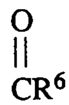

wherein $R^6$ is as above; $R^1$ and $R^8$ taken together with the oxygen to which they are attached form a group of the formula

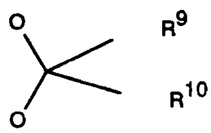

wherein $R^9$ and $R^{10}$ are independently hydrogen or alkyl; the optical isomers thereof, or the pharmaceutically acceptable salts thereof, which are useful for reducing inflammation by virtue of their ability to inhibit protein kinase C and thus indicated for the treatment of psoriasis and other skin disorders, for inhibiting tumor or neoplastic cell growth by virtue of their ability to reduce cell proliferation and thus indicated in cancer therapy, and relieving memory dysfunction and thus indicated in the treatment of Alzheimer's disease, and as antibacterial and antifungal agents, alone or in combination with adjuvants.

Preferred 2-amino-1,3-propanediols of the present invention are those wherein R is

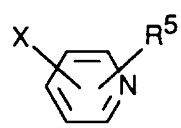 ;

$R^1$, $R^2$, and $R^3$ are hydrogen and $R^5$ is $CH_3(CH_2)_mCH_2CH_2$ or

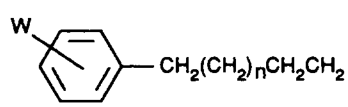 .

Also preferred are compounds wherein R is

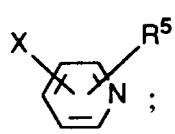 ;

$R^1$ and $R^2$ are hydrogen; $R^4$ is

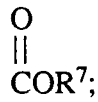

and $R^5$ is $CH_3(CH_2)_mC{\equiv}C$.

The present invention also relates to compounds of the formula

R'CHO 1a

wherein R' is

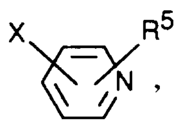 , 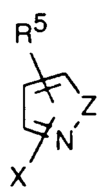 , or 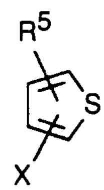

wherein $R^5$ is $CH_3(CH_2)_mC{\equiv}C$, $CH_3(CH_2)_mCH=CH$,$CH_3(CH_2)_mCH_2CH_2$, or

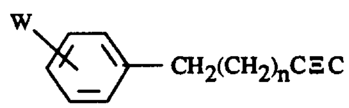

wherein m is 3 to 15, n is 0 to 12, W and X are independently hydrogen, alkyl, alkoxy, halogen, or trifluoromethyl and Z is O, which are useful as intermediates for the preparation of the present 2-amino-1,3-propanediols.

As used through the specification and appended claims, the term "alkyl" refers to a straight or branched chain hydrocarbon radical containing no unsaturation and Examples of alkyl groups are methyl, ethyl, 1-propyl, 2-propyl, 1-butyl, 1-pentyl, 3-hexyl, 4-heptyl and 2-octyl. The term "alkanol" refers to a compound formed by a combination of an alkyl group and hydroxy radical. Examples of alkanols are methanol, ethanol, 1- and 2-propanol, 2,2-dimethylethanol, hexanol and octanol. The term "alkanoic acid" refers to a compound formed by combination of a carboxyl group with a hydrogen atom or alkyl group. Examples of alkanoic acids are formic acid, acetic acid, propanoic acid, 2,2-dimethylacetic acid, hexanoic acid and octanoic acid. The term "halogen" refers to a member of the family fluorine, chlorine, bromine, or iodine. The term "alkanoyl" refers to the radical formed by removal of the hydroxyl function from an alkanoic acid. Examples of alkanoyl groups are formyl, acetyl, propionyl, 2,2-dimethylacetyl, hexanoyl and octanoyl. Any of the aforementioned groups having a carbon skeleton are "lower" ones containing up to and including 8 carbon atoms.

The compounds of the present invention which lack an element of symmetry exist as optical antipodes and as the racemic forms thereof. The optical antipodes may be prepared from the corresponding racemic forms by standard optical resolution techniques, involving, for example, the separation of diastereomeric salts of those instant compounds characterized by the presence of a basic amino group and an optically active acid, those instant compounds characterized by the presence of a carboxylic acid group and an optically active base, or by synthesis from optically active precursors.

The present invention comprehends all optical isomers and racemic forms thereof and all geometric isomers of the compounds disclosed and claimed herein. The formulas of the compounds shown herein are intended to encompass all possible geometric and optical isomers of the compounds so depicted.

The compounds of the present invention that have adjacent chiral centers exist as diastereomers and are distinguished as the erythro- and threo-isomers. The erythro diastereomers are those that become meso, i.e., optically inactive, by virtue of having an element of symmetry in one of the possible conformations, when one of the dissimilar substituents is replaced by the other. The threo diastereomers are those that remain enantromeric, i.e., optically active, by virtue of lacking an element of symmetry in one

of the possible conformations, when one of the dissimilar substitutents is replaced by the other. For example, replacement of the amino group of an erythro-2-amino-1,3-propanediol 9a of the present invention by a hydroxyl group creates a meso- 1,2,3-propanetriol 9b, having a plane of symmetry through the carbon backbone of the molecule, as shown below,

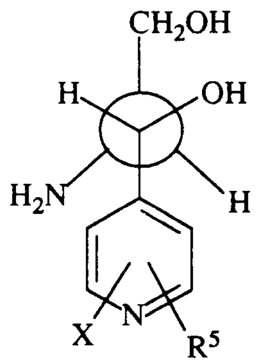

9a

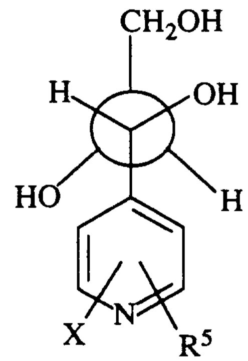

9b

and replacement of the amino group of a threo-2-amino-1,3-propanediol 9c of the present invention by a hydroxy group creates an enantiomer 9d, lacking an element of symmetry in all conformations, one of which is 9d.

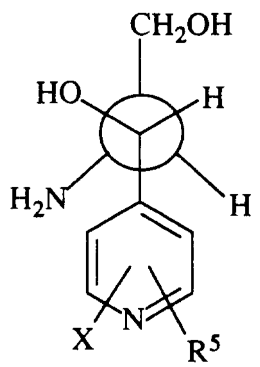

9c

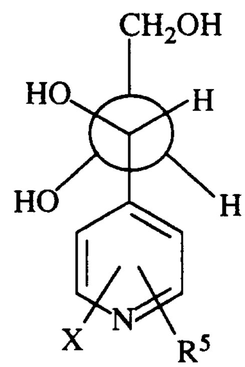

9d

The novel 1-alkyl-, 1-alkenyl-, and 1-alkynylaryl-2-amino-1,3-propanediols of the present invention are prepared by the processes illustrated in Reaction Schemes A, B, and C for the pyridine series, having an aralkyl side-chain. The transformations shown therein are applicable to the preparation of compounds of the invention wherein the aryl group is, among others, substituted and unsubstituted phenyl, furyl, thienyl, isoxazolyl, isothiazolyl, and pyrrolyl, thiazolyl, and oxazolyl, having a 1-alkyl, 1-alkenyl, or 1-alkynyl-side chain.

To prepare a 1-alkynylpyridinyl-2-amino-1,3-propanediol 7 wherein W and X are hydrogen, alkyl, alkoxy, halogen, or trifluoromethyl, a pyridinylcarboxaldehyde 2 wherein W and X are as above and Y is halogen is condensed with an amidomalonic acid ester 3 wherein $R^{11}$ and $R^{12}$ are alkyl to provide an alkyl pyridinylpropionate 4 wherein $R^{11}$, $R^{12}$, X, and Y are as above, which is alkynylated to alkynylpyridine 5 wherein $R^{11}$, $R^{12}$, W and X are as above and n is 3 to 15 and, in turn, reduced to pyridinyl-1,3-propanediol 6 wherein $R^{12}$, W, and X are as above and hydrolyzed to 7.

The condensation of carboxaldehyde 2 and malonate 3 is conducted in an ethereal solvent in the presence of a tertiary amine. Among ethereal solvents there may be mentioned diethyl ether, 1,2-dimethyoxyethane, 2-methoxyethyl ether, dioxane, and tetrahydrofuran. Among tertiary amines there may be mentioned pyridines (pyridine, picoline, lutidine, and collidine) and trialkylamines (trimethylamine, triethylamine, and tripropylamine). Tetrahydrofuran and triethylamine are the preferred solvent and tertiary

amine, respectively, While the condensation temperature is not critical, the reaction is preferably performed at about ambient temperature (25 °C), although reduced temperatures (about 0 °C to about 25 °C) or elevated temperatures (about 25 °C to the boiling point of the reaction mixture) may be employed.

The alkynylation is performed by treating a halopyridine 4 with an alkyne 13

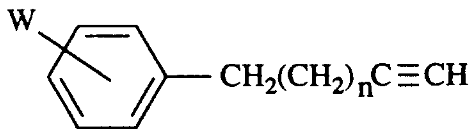

13

wherein W and n are as above in an acid acceptor, e.g., a di- or trialkylamine, such as, diethylamine, dipropylamine, trimethylamine, triethylamine, or tripropylamine, in the presence of bis(triphenylphosphine)-palladium dichloride/cuprous iodide at a temperature of about 0° to about 75 °C. Triethylamine is the preferred acceptor. A temperature of about 50° to 60 °C is the preferred alkynylation temperature. An ethereal solvent may be employed. Ethereal solvents include diethyl ether, 1,2-dimethoxyethane, 2-methoxyethyl-ether, dioxane, and tetrahydrofuran. Tetrahydrofuran is the preferred solvent.

The reduction of an alkyl pyridinylpropionate 5 to a propanediol 6 is accomplished by means of an alkali borohydride in an ethereal solvent at a reduction temperature within the range of about 0° to about 50 °C. Included among alkali borohydrides are calcium borohydride, lithium borohydride, potassium borohydride, and sodium borohydride. Included among ethereal solvents are diethyl ether, 1,2-dimethoxyethane, 2-methoxyethyl ether, dioxane, and tetrahydrofuran. A reducing system of lithium borohydride or calcium borohydride in tetrahydrofuran at a temperature of from about 0° to 25 °C is preferred.

The hydrolysis of a carboxamide 6 to an aminodiol 7 may be carried out by conventional hydrolysis techniques. For example, carboxamide 6 may be hydrolyzed by an alkali metal hydroxide, i.e., lithium hydroxide, sodium hydroxide, or potassium hydroxide, in an aqueous alkanol, i.e., methanol, ethanol, or 1- or 2-propanol, at a hydrolysis temperature of about 0 °C to about 100 °C.

To prepare a 1-alkylpyridinyl-2-amino-1,3-propanediol 9 wherein W, X, and m are as hereinbeforedescribed, a 1-alkynylpyridinyl-2-amido-1,3-propanediol 6 is hydrogenated to a 1-alkylpyridinyl-2-amido-1,3-propanediol 8, which is converted to a 1-alkylpyridinyl-2-aminol-1,3-propanediol 9.

The hydrogenation is effected by treating an alkyne 6 with hydrogen at about atmospheric pressure to about 60 psi, a pressure of about 40 psi being preferred, in the presence of a metal catalyst, e.g., platinum, palladium, rhodium, or ruthenium, unsupported or supported on carbon or calcium carbonate, palladium-on-carbon being preferred, in an alkanol, e.g., methanol, ethanol, or 1- or 2-propanol, ethanol being preferred, at a hydrogenation temperature of about 25° to about 50 °C, a temperature of about 25 °C being preferred.

The conversion of pyridinylamidodiol 8 to pyridinylaminodiol 9, i.e., the hydrazinolysis of 8, is conducted with hydrazine, free or in its hydrated form, in an alkanol such as, for example, methanol, ethanol, or 1- or 2-propanol, at a temperature of from about 25 °C to the reflux temperature of the reaction mixture. Ethanol is the preferred solvent. A hydrazinolysis temperature of about the reflux temperature of the reaction mixture is also preferred.

Alternatively, entry into the 1-alkynyl- and 1-alkylpyridinyl-2-amino-1,3-propanediol systems, i.e., systems of formulas 7 and 9, respectively, wherein W, X, and m are as hereinbeforedescibed may be achieved by alkynylation of pyridinylcarboxaldehyde 2 wherein W, X, and Y are as above to alkynylpyridinylcarboxaldehyde 10 wherein W, X, and m are as above followed by conversion of pyridinylcarboxaldehyde 10 to alkyl pyridinylpropionate 5 wherein $R^{11}$, $R^{12}$, W, X, and m are as above and hydrogenation of an alkynylpyridine 5 wherein $R^{11}$, $R^{12}$, W, X, and m are as above to 11 wherein $R^{11}$, $R^{12}$, W, X, and m are as above. The alkynylation, conversion, and hydrogenation, i.e., the transformations of 2 to 5 and 11, via 10, are accomplished by processes substantially similar to the corresponding transformations of 4 to 5, 2 to 4, and 6 to 8.

Alkyl 1-alkylpyridinylpropionate 11 wherein $R^{11}$, $R^{12}$, W, X, and m are as above may be reduced to 1-alkyl pyridinylpropanediol 8 by the process essentially the same as that employed for the reduction of alkyl pyridinylpropionate 5 to propanediol 6.

Entry into the 1-alkynylpyridinyl-2-amino-1,3-propanediol series, i.e., the series encompassing compounds of formulas 5, 6, and 7, is also attained by reducing an alkyl pyridinylpropionate 4 wherein $R^{11}$, $R^{12}$, W, X and Y are as hereinbeforedescribed to a pyridinylpropanediol 12 and alkynylating a pyridinyldiol 12, so obtained, to alkynylpyridinyldiol 6. As described above, amidopropanediol 6 is converted to amino

propanediol 7 by hydrolysis. Similarly, the reduction of 4 to 12 and the alkynylation of 12 to 6 are performed by processes substantially the same as those utilized for the conversion of 5 to 6 and 4 to 5.

Derivatives of an alkynylpyridinyl-2-amino-1,3-diol 7 are prepared from amidopropanediol 6 by acylation of 6 wherein $R^{12}$, W, X, and m are as hereinbeforedescribed to an amidodiacyloxypropane 15 wherein $R^{12}$, $R^{13}$, $R^{14}$, W, and m are as hereinbeforedescribed with, for example, an alkanoic acid anhydride such as acetic anhydride in the presence of triethylamine and 4-dimethylaminopyridine to 15, and dioxanylation of 6 to amidodioxane 14 wherein $R^{12}$, $R^{15}$, $R^{16}$, W, X, and m are as hereinbeforedescribed with, for example, 2,2-dimethoxypropane in the presence of para-toluene sulfuric acid. Hydrolysis of 15 as described for the conversion of 6 to 7 provides aminopropanediol 7. An amidodiacyloxypropane 15 is selectively hydrolyzed to an amidodihydroxy propane 20 by, for example, an alkali metal carbonate such as lithium, sodium, or potassium carbonate in an alkanol such as methanol, ethanol, or 2-propanol. Potassium carbonate in methanol is the preferred hydrolysis medium. The hydrolysis proceeds readily at ambient temperature. Elevated temperatures to the reflux temperature of the hydrolysis medium may be employed.

Acyl derivatives of amidopropanediol 12 wherein $R^{12}$, X, and Y are as hereinbeforedescribed are prepared by treating 12 with an alkanoic acid anhydride under the conditions for the conversion of 6 to 15.

To prepare a 1-alkenyl-2-amino-1,3-propanediol 17 wherein W, X, and m are as hereinbeforedescribed a 1-alkynyl-2-amino-1,3-propanediol 6 wherein $R^{12}$, W, X, and m are as above is hydrogenated to a 1-alkenyl-2-amino-1,3-propanediol 16 wherein $R^{12}$, W, X, and m are as above and the configuation of the hydrogen atoms of the carbon-to-carbon double bond is cis, which is hydrolyzed to 17 wherein W, X, and m are also as above.

To fabricate an N,O,O-tribenzyloxycarbonyl-2-amino-1,3-propane 18 wherein $R^{15}$ is

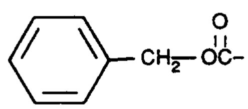 ,

an 2-amino-1,3-propanediol 9 is treated with N-benzyloxycarbonyloxysuccinimide 20

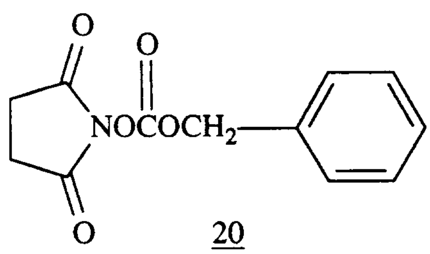

20

in the presence of a tertiary amine, e.g., triethyl amine in an ethereal solvent, e.g., tetrahydrofuran at about ambient temperature.

To synthesize a 2-amino-1,3-propanediol 19, a 1,3-diacyloxy-2-propanylacetamide 13 is hydrolyzed by hydrazine hydrate in the presence of ethanol according to the procedure for the conversion of 8 or 9.

Generally, the ultimate 1-alkylaryl-2-amino-1,3-propanediols of the present invention are prepared from 1-alkynylarylcarboxaldehydes. See Reaction Scheme A for the conversion of 10 to 9 in the pyridine series. In the isoxazole series, the ultimate 1-alkylisoxazolyl-2-aminol-1,3-propanediols may be prepared, for example, from a 5-(1-alkyl)-3-isoxazolecarboxaldehyde 21 wherein $R^5$ is dodecyl.

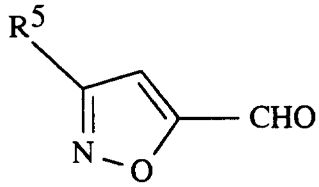

21

A 3-isoxazolecarboxaldehyde 21 wherein $R^5$ is dodecyl, in turn, is synthesized, for example, by condensing 1-nitrotridecane with O-trimethylsilylpropynol in the presence of phenylisocyanate and triethylamine followed tetrabutylammonium fluoride to afford isoxazolemethanol 22

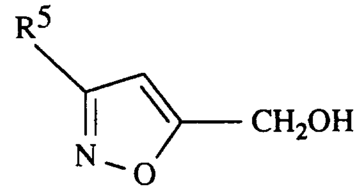

22

wherein $R^5$ is dodecyl, which is oxidized by oxalyl chloride:dimethylsulfoxide to 21.

To prepare a 2-alkoxycarbonylamino-1,3-propanediol, for example, 1-alkynyl-2-t-butyloxycarbonylamino-1,3-propanediol 6 wherein $R^{12}$ is $OC(CH_3)_3$, a 1-alkynyl-2-amino-1,3-propanediol 7 is acylated with di-t-butyldicarbonate in the presence of a base such as sodium bicarbonate in a halocarbon solvent such as chloroform at an elevated temperature of about 60 ° C.

To prepare a 2-dialkylamino-1,3-propanediol, for example, a 1-alkenyl-2-dimethylamino-1,3-propanediol 23, a 1-alkenyl-2-amino-1,3-propanediol 17 is reductively alkylated with formaldehyde such as formalin in the presence of a reducing agent such as sodium cyanoborohydride in a solvent such as acetonitrile at ambient temperature.

A 1-alkenyl-2-amino-1,3-propanediol, e.g., a 1-alkenylpyridinyl-2-amino-1,3-propanediol 17, is prepared by reduction of a 1-alkynylpyridinyl-2-acylamino-1,3-propanediol 6 via a 1-alkenylpyridinyl-2-acylamino-1,3-propanediol 16. See Reaction Scheme C. Alternatively, a 1-alkenyl-2-amino-1,3-propanediol, e.g., a 1-alkenylthienyl-2-amino-1,3-propanediol 27, is prepared by condensation of a halothiophenecarboxyaldehyde 25 wherein X is bromo with a tri-n-butyl-1-alkenylstannane 24 in the presence of 2,6-di-t-butyl-4-methylphenol and tetrakis(triphenylphosphine)palladium(O) in an aromatic solvent such as toluene at room temperature to a 1-alkenylthiophenecarboxaldehyde 26 (see Reaction Scheme D), which, in turn, is converted to a 2-amino-1,3-diol 27 and derivatives thereof by the processes outlined in Reaction Schemes A, B, and C.

The requisite tri-n-butyl-1-alkenylstannane 24 is prepared by reductive condensation of an alkyne 28 with tri-n-butyltinhydride in the presence of azobisisobutyronitrile.

The 1-alkyl-, 1-alkenyl-, and 1-alkynylaryl-2-amino-1,3-propanediols of the present invention are useful as agents for the relief of memory dysfunction, particularly dysfunctions associated with decreased cholinergic activity such as those found in Alzheimer's disease. Relief of memory dysfunction activity of the instant compounds is demonstrated in the dark avoidance assay, an assay for the determination of the reversal of the effects of scopolamine induced memory deficits associated with decreased levels of acetylcholine in the brain. In this assay, three groups of 15 male CFW mice were used--a vehicle/vehicle control group, a scopolamine/vehicle group, and a scopolamine/drug group. Thirty minutes prior to training, the vehicle/vehicle control group received normal saline subcutaneously, and the scopolamine/vehicle and scopolamine/drug groups received scopolamine subcutaneously (3.0 mg/kg, administered as scopolamine hydrobromide). Five minutes prior to training, the vehicle/vehicle control and scopolamine/vehicle groups received distilled water and the scopolamine/drug group received the test compound in distilled water.

The training/testing apparatus consisted of a plexiglass box approximately 48 cm long, 30 cm high and tapering from 26 cm wide at the top to 3 cm wide at the bottom. The interior of the box was divided equally by a vertical barrier into a light compartment (illuminated by a 25-watt reflector lamp suspended 30 cm from the floor) and a dark compartment (covered). There was a hole at the bottom of the barrier 2.5 cm wide and 6 cm tall and a trap door which could be dropped to prevent an animal from passing between the two compartments. A Coulbourn Instruments small animal shocker was attached to two metal plates which ran the entire length of the apparatus, and a photocell was placed in the dark compartment 7.5 cm from the vertical barrier and 2 cm off the floor. The behavioral session was controlled by PDP 11/34 minicomputer.

At the end of the pretreatment interval, an animal was placed in the light chamber directly under the light fixture, facing away from the door to the dark chamber. The apparatus was then covered and the system activated. If the mouse passed through the barrier to the dark compartment and broke the photocell beam within 180 seconds, the trap door dropped to block escape to the light compartment and an electric shock was administered at an intensity of 0.4 milliamps for three seconds. The animal was then

immediately removed from the dark compartment and placed in its home cage. If the animal failed to break the photocell beam within 180 seconds, it was discarded. The latency is seconds for each mouse was recorded.

Twenty-four hours later, the animals were again tested in the same apparatus except that no injections were made and the mice did not receive a shock. The test day latency in seconds for each animal was recorded and the animals were then discarded.

The high degree of variability (due to season of the year, housing conditions, and handling) found in one trial passive avoidance paradigm is well known. To control for this fact, individual cutoff (CO) values were determined for each test, compensating for interest variability. Additionally, it was found that 5 to 7% of the mice in the scopolamine/vehicle control groups were insensitive to scopolamine at 3 mg/kg, sc. Thus, the CO value was defined as the second highest latency time in the control group to more accurately reflect the 1/15 expected control responders in each test group. Experiments with a variety of standards repeated under a number of environmental conditions led to the development of the following empirical criteria: for a valid test, the CO value had to be less than 120 sec and the vehicle/vehicle control group had to have at least 5/15 animals with latencies greater than CO. For a compound to be considered active the scopolamine/compound group had to have at least 3/15 mice with latencies greater than CO.

The results of the dark avoidance test are expressed as the number of animals per group (%) in which this scopolamine induced memory deficit is blocked as measured by an increase in the latency period. Relief of memory dysfunction activity for representative compounds of the present invention is presented in Table 1.

TABLE 1

| Compound | Dose (mg/kg, sc) | Percent of Animals with Scopolamine Induced Memory Deficit Reversal |
|---|---|---|
| ethyl erythro-2-acetamido-3-[6-(1-decynyl)-2-pyridinyl]-3-hydroxypropionate | 3.0 | 27 |
| erythro-N-{1-[6-(1-decynyl)-2-pyridinyl]-1,3-dihydroxy--2-propanyl}acetamide | 3.0 | 33 |
| physostigmine | 0.31 | 20 |

Scopolamine induced memory deficit reversal is achieved when the present 1-alkyl-, 1-alkenyl-, and 1-alkynylaryl-2-amino-1,3-propanediol, and related compounds are administered to a subject requiring such treatment as an effective oral, parenteral or intravenous dose of from 0.01 to 100 mg/kg of body weight per day. A particularly effective amount is about 25 mg/kg of body weight per day. It is to be understood, however, that for any particular subject, specific dosage regimens should be adjusted according to the individual need and the professional judgment of the person administering or supervising the administration of the aforesaid compound. It is to be further understood that the dosages set forth herein are exemplary only and that they do not, to any extent, limit the scope or practice of the invention.

The 1-alkyl-, 1-alkenyl-, and 1-alkynylaryl-2-amino-1,3-propanediols of the present invention are also useful as antiiflammatory agents due to their ability to reduce inflammation in mammals. The antiinflammatory activity is demonstrated in the TPA-induced ear edema assay and the arachidonic acid-induced ear edema test (see J. M. Young, et al., Journal Investigative Dermatology, 80, 48 (1983)).

In the TPA-induced ear edema assay, TPA (12-O-tetradecanoylphorbol-13-acetate) was dissolved in 30/70 propylene glycol/ethanol and was applied to the right ear of groups of 6 female Swiss Webster mice, which were housed together in a cage under standard conditions for 1 week prior to use with food and water ad lib, at a volume of 20 µl so that a total of 10 µg of TPA is delivered to the inner and outer surfaces of the ear. The test compound was dissolved in the vehicle and was applied to the right ear (the inner and outer surface) at a volume of 20 µl so that a total of 10 µg of the compound was delivered to the ear. After about 5 hours, the animals were sacrificed, a 4 mm diameter plug was taken from each ear and weighed. The difference between the right and left ear plug weights for each animal was determined. The antiinflammatory activity of the test compound is expressed as the mean percent change in the ear plug weight of the treated animals compared to the mean percent change in the plug weight of the control animals. Antiinflammatory activity of representative compounds of the instant invention as determined in this

assay are presented below in Table 2.

TABLE 2

| Compound | Antiinflammatory Activity Percent Decrease in Ear Plug Weight at 10 μg/ear |
|---|---|
| erythro-2-amino-1-(6-decyl-2-pyridinyl)-1,3-dihydroxypropane | 66 |
| ethyl erythro-2-acetamido-3-[6-(1-dodecynyl)-2-pyridinyl]-3-hydroxypropionate | 50 |
| erythro-N-{1-[6-(1-dodecynyl)-2-pyridinyl]-1,3-dihydroxy-2-propanyl}acetamide | 59 |
| erythro-2-amino-1-(6-dodecyl-2-pyridinyl)-1,3-propanediol | 24 |
| threo-2-amino-1-(6-decyl)-2-pyridinyl)-1,3-propanediol | 30 |
| D-erythro-sphingosine | 46 |

In the arachidonic acid-induced ear edema assay, the test compound was dissolved in 30/70 propylene glycol/ethanol and was applied to both ears of groups of 6 female Swiss Webster mice, which were housed together in a cage under standard conditions for 1 week prior to use with food and water ad lib, at a volume of 20 μl so that a total of 1.0 mg of test compound was delivered to each ear over the inner and outer surfaces. The same volume (20 μl) of vehicle was applied to each ear of a control group of mice. After 30 minutes, arachidonic acid was applied to the right ear of each mouse of each group in the amount of 4 mg/ear. Vehicle was applied to the left ear of each mouse of each group at a volume of 20 μl/ear. After an additional hour, the mice were sacrificed and a 4 mm plug was taken from each ear and weighed. The difference between the right and left ear plugs was determined for each animal. The antiinflammatory activity of the test compound is expressed as the mean percent change in the ear plug weight of the treated animals relative to the mean percent change in weights of control animals' ear. Antiinflammatory activity of representative compounds of the present invention as determined in this assay are presented below in Table 3.

TABLE 3

| Compound | Antiinflammatory Activity Percent Decrease in Ear Plug Weight at 1 mg/ear |
|---|---|
| erythro-2-amino-1-(6-decyl-2-pyridinyl)-1,3-dihydroxypropane | 32 |
| D-erythro-sphingosine | +9 |

Inflammation reduction is achieved when the present 1-alkyl-, 1-alkenyl-, and 1-alkynylaryl-2-amino-1,3-propanediols are administered topically, including ophthalmic administration, to a subject requiring such treatment as an effective topical dose of from 0.001 to 100 mg/kg of body weight per day. A particularly effective amount is about 25 mg/kg of body weight per day. It is to be understood however, that for any particular subject, specific dosage regimens should be adjusted according to the individual need and the professional judgment of the person administering or supervising the administration of the aforesaid compound. It is to be further understood that the dosages set forth herein are exemplary only and that they do not, to any extent, limit the scope or practice of the invention.

The 1-alkyl-, 1-alkenyl-, and 1-alkynyl-2-amino-1,3-propanediols of the present invention are also useful as inhibitors of tumor or neoplastic cell growth by virtue of their ability to reduce cell proliferation as demonstrated in the protein kinase C assay. (see U. Kikkawa, et al., Biochemical and Biophysical Research Communications, 135, 636 (1986) and R. M. Bell, et al. "Methods in Enzymology, Hormone Action," Part J, P.M. Conn, Ed., Academic Press, Inc., New York, NY 1986, page 353).

Protein kinase C enzyme extract was prepared from the brain of male Wistar rats weighing 180 to 200 g and purified by the method of U. Kikkawa, et al., ibid. 636. The purified extract was stored at -80 °C, and aliquots were used in the protein kinase C assay performed by a modification of the method of R. M. Bell, et al., ibid. al 354.

To perform the assay, duplicate aliquots of duplicate samples are employed. Basal or unstimulated protein kinase C, phosphatidylserine/diacylglycerol stimulated protein kinase C, and test samples are run in each assay. Protein kinase C extract (1-5 μg of protein; 10 μl); an 8 μl solution of N-2-hydroxyethylpiperazine-N'-2-ethylsulforic acid (500 mM), magnesium chloride (40 mM), and ethylenediaminoetetraacetic acid (10 mM); dithiothreitol (20 mM; 8 µl), Type III histone (12 µg; 8 µl), and calcium chloride (11 mM; 8 µl) was added to each unstimulated protein kinase C sample assay tube, chilled in ice. Phosphatidylserine/diacylglycerol (4 µg; 8 µl) was added to each stimulated protein kinase sample assay tube, chilled in ice. The test compound ($10^{-4}$ to $10^{-12}$M in 4 µl dimethylsulfoxide) was added to the test sample tubes, chilled in ice. The volume for all sample tubes was brought to 72 µl with distilled water (18 µl for stimulated samples; 26 µl for unstimulated samples without 8 µl of phosphatidylserine/diacylglycerol). The assay tubes were allowed to warm to 25°C and an 8 µl mixture of adenosine 5'-triphosphate (100 µM) and $^{32}$P-adenosine triphosphate (1 to 2 x $10^5$ counts per minute) was added to each tube for a final volume of 80 µl per tube. After 2 min, the reaction (the incorporation of phosphorous into Type III histone) was terminated by spotting the assay mixture on phosphocellulose paper. The spots are cut out of the paper and the radioactivity (counts per min) of each spot was determined in a scintillation counter. Percent protein kinase C inhibitory activity, i.e., the percent inhibition of the incorporation of $^{32}$posphorous from $^{32}$P-adenosine triphosphate into Type III histone, is calculated as follows:

$$\frac{\text{radioactively of test sample (cpm)}}{\text{radioactivity of stimulated sample (cpm) - radioactivity of unstimulated sample (cpm)}} \times 100\%$$

Protein kinase C inhibitory activity of representative compounds of the present invention expressed as the calculated concentration of test compound effecting a 50% inhibition of phosphorous uptake ($IC_{50}$) is presented below in Table 4.

TABLE 4

| Compound | Protein Kinase Inhibitory Activity $IC_{50}$ (µM) |
|---|---|
| ethyl erythro-2-acetamido-3-[6-(1-dodecynyl)-2-pyridinyl]-3-hydroxypropionate | 29 |
| cis-erythro-N-{1-[6-(1-dodecenyl)-2-pyridinyl]-1,3-dihydroxy-2-propanyl}-acetamide | 66%@ 100 µM |
| erythro-2-amino-1-(6-dodecyl-2-pyridinyl)-1,3-propanediol | 8.5 |
| erythro-2-amino-1-(6-decyl-2-pyridinyl)-1,3-dihydroxypropane | 48 |
| threo-2-amino- 1-(6-decyl-2-pyridinyl)-1,3-dihydroxypropane | 25 |
| D-erythro-sphingosine | 6.7 |

Protein kinase C inhibition is achieved when the present 1-alkyl-, 1-alkenyl-, and 1-alkynylaryl-2-amino-1,3-propanediols, and related compounds are administered to a subject requiring such treatment as an effective oral, parenteral, intravenous, or topical dose of from 0.001 or 0.01 to 100 mg/kg of body weight per day. A particularly effective amount is about 25 mg/kg of body weight per day. It is to be understood, however, that for any particular subject, specific dosage regimens should be adjusted according to the individual need and the professional judgment of the person administering or supervising the administration of the aforesaid compound. It is to he further understood that the dosages set forth herein are exemplary only and that they do not, to any extent, limit the scope or practice of the invention.

The 1-alkyl-, 1-alkenyl-, and 1-alkynylaryl-2-amino-1,3-propanediols of the present invention are also useful as antibacterial and antifungal agents due to their ability to inhibit bacterial and fungal growth in mammals. Antibacterial and antifungal activity are demonstrated in conventional antimicrobial assays assays (see D. J. Bibel, et al., The Journal of Investigative Dermatology, 92, 632 (1989).

In the aerobic antibacterial assay, the sensitivity of aerobic bacteria was tested by means of the agar dilution test in Mueller-Hinton agar. Plates were inoculated with a multipoint inoculator which delivered 5 x $10^4$ CFU/spot of stationary, freshing diluted cultures of the strains concerned. The minimun inhibitory concentration (MIC) was taken as the lowest concentration at which no visible growth could be detected after 24 hours at 37°C.

In the anerobic assay, the susceptibility of obligate gram-positive and gram-negative anaerobes was tested using the agar dilution test on Wilkins-Chalgren agar. Overnight cultures of the appropriate test

strains diluted 1:10 in fresh thioglycollate medium were used as the inoculum. The MICs of the antibiotics were determined after the plates had been incubated in anaerobic jars for 48 hours at 37 °C.

Antibacterial activity of representative compounds of the instant invention as determined in this assay is presented below in Tables 5 and 6.

In the antifungal assay, utilizing a microtitration technique (U-shaped, 96 well-plate), the test compound (10 mg) is dissolved in a suitable solvent (10 ml dist. water, or 1 ml org. solvent + 9 ml dist. water).

The microtiter plate is prepared as follows: The wells are each filled (2 rows/strain) with 50 µl neopeptone-dextrose broth (12-channel pipette). In addition, one row/strain is coated with 50 µl yeast-nitrogen base/well for yeasts and moulds. Subsequently, 50 µl compound solution are added to each well in the first row, mixed and diluted further by transferral of 50 µl respectively in the ratio 1:2. All wells are then inoculated with 150 µl standardized organism suspension (yeasts: 1 x $10^3$ organisms/ml suspension; cutaneous fungi and moulds: 1.6 x $10^5$ organisms/ml suspension); the total volume is 200 µl per well.

There is also a growth control (inoculated, not medicated), a solvent control (inoculated, not medicated, containing solvent as in medicated rows) and a negative

TABLE 5

Antibacterial activity (MIC, mg/l)

| Aerobic Bacteria Strain | | erythro-2-amino-1-(6-decyl-2-pyridinyl)-1,3-dihydroxypropane | D-erythro-sphingosine |
|---|---|---|---|
| Staph. aureus | SG511 | 12.50 | 25.0 |
| | 285 | 12.50 | 25.0 |
| | 503 | 6.25 | 25.0 |
| Strept. pyogenes | 308 A | 6.25 | 12.5 |
| | 77 A | 6.25 | 25.0 |
| Strept. faecium | D | 12.50 | 25.0 |
| E. coli | O 78 | 12.50 | > 100.0 |
| | TEM | 25.00 | > 100.0 |
| | 1507E | 12.50 | > 100.0 |
| | DC0 | 12.50 | > 100.0 |
| | DC2 | 6.25 | 25.0 |
| S. typhimurium | | 12.50 | > 100.0 |
| Klebsiella spp. | 1082E | 12.50 | 25.0 |
| | 1522E | 12.50 | > 100.0 |
| E. cloacae | P99 | 12.50 | > 100.0 |
| | 1321E | 12.50 | 50.0 |

TABLE 6

Antibacterial activity

(MIC, mg/l)

| Anerobic Bacteria Strain | | Erythro-2-amino-1-(6-decyl-2-pyridinyl)-1,3-dihydroxypropane | Erythro-N-{1-[6-(1-dodecynyl)-2-pyridinyl]-1,3-dihydroxy-2-propanyl}acetamide | Erythro-2-amino-1-[5-(1-dodecynyl)-2-thienyl]-1,3-propanediol | D-erythro-sphingosine |
|---|---|---|---|---|---|
| Bact. fragilis | 312 | 12.50 | 3.13 | 6.25 | 25.0 |
| | 960 | 6.25 | 3.13 | 6.25 | 12.5 |
| | 1313 | 12.50 | 6.25 | 6.25 | 25.0 |
| | 17390 | 12.50 | 6.25 | 3.13 | 25.0 |
| | 18125 | 12.50 | 3.13 | 3.13 | 25.0 |
| | 19016 | 12.50 | 6.25 | 6.25 | 25.0 |
| Bact. ovatus | 103 | 6.25 | 3.13 | 3.13 | 12.5 |
| Bact. vulgatus | 1446 | 12.50 | 3.13 | 3.13 | 25.0 |
| Bact. thetaiotaom. | 123 | 6.26 | 3.13 | 3.13 | 25.0 |
| | 1428 | 12.50 | 6.25 | 6.25 | 25.0 |
| | 1445 | 12.50 | 6.25 | 3.13 | 25.0 |
| Bact. distasonis | 1366 | 12.50 | 6.25 | 3.13 | 12.5 |
| Fusobact. varium | 5262 | 12.50 | 6.25 | 3.13 | 25.0 |
| | 3085 | 12.50 | 6.25 | 6.25 | 25.0 |
| Spaeroph. freundii | 1369 | 12.50 | 3.13 | 1.56 | |
| Peptostr. anaerobius | 932 | 12.50 | 6.25 | 6.25 | 12.5 |
| Propionibact. acnes | 6919 | 12.50 | 6.25 | 3.13 | 12.5 |
| | 6922 | 12.50 | 6.25 | 1.56 | 12.5 |
| Clost. tetani | 19406 | 12.50 | 12.50 | 6.25 | 50.0 |
| Clost. perfringens | 194 | 12.50 | 12.00 | 6.25 | 6.25 |

control (not inoculated, not medicated).

Incubation for 5 days at 30 °C is followed by photometric evaluation. The obtained measurements are checked visually (macroscopically and microscopically) and corrected where necessary.

Criteria for Evaluation of the Antimycotic Effect

a. Photometric measurements (matrix method)
b. Growth, macroscopic evaluation
c. Growth, microscopic evalucation (inversion light microscope, magn. 64x).

Antifungal activity of representative compounds of the instant invention as determined in the microtiter assay is presented below in Table 7.

TABLE 7

**erythro-N-{1-[6-(l-Dodecynyl)-2-pyridinyl]-1,3-dihydroxy-2-propanyl}acetamide**

| Pathogen | Strain | Antifungal Activity MIC [µg/ml] |
|---|---|---|
| Trichophyton Mentagrophytes | 100/25 | 7.810 |
| Trichophyton Rubrum | 101/85 | 31.250 |
| Microsporum Canis | 150/353 | 0.970 |
| Candida Albicans | 200/175 | 125.000 |
| Aspergillus Niger | 500/284 | 125.000 |
| Trichophyton Vaginalis | 111/216 | 15.625 |
| **erythro-2-Amino-1-(6-decyl-2-pyridinyl)-1,3-dihydroxypropane** | | |
| Trichophyton Mentagrophytes | 100/25 | 31.250 |
| Trichophyton Rubrum | 101/85 | 31.250 |
| Microsporum Canis | 150/353 | 31.250 |
| Candida. Albicans | 200/175 | 31.250 |
| Aspergillus Niger | 500/284 | 31.250 |
| Trichophyton Vaginalis | 111/216 | 15.625 |

**erythro-2-Amino-1-[5-(1-dodecynyl)-2-thienyl]-1,3-propanediol**

| | | |
|---|---|---|
| Trichophyton Mentagrophytes | 100/25 | 3.900 |
| Trichophyton Rubrum | 101/85 | 15.625 |
| Microsporum Canis | 150/353 | 3.900 |
| Candida Albicans | 200/175 | 1.950 |
| Aspergillus Niger | 500/284 | 3.900 |
| Trichophyton Vaginalis | 111/216 | 15.625 |
| **Cyclopirox** | | |
| Trichophyton Mentagrophytes | 100/25 | 1.950 |
| Trichophyton Rubrum | 101/85 | 1.950 |
| Microsporum Canis | 150/353 | 1.950 |
| Candida Albicans | 200/175 | 1.950 |
| Aspergillus Niger | 500/284 | 0.970 |
| Trichophyton Vaginalis | 111/216 | 15.625 |
| **Clotrimazol** | | |
| Trichophyton Mentagrophytes | 100/25 | 0.970 |
| Trichophyton Rubrum | 101/85 | 0.240 |
| Microsporum Canis | 150/353 | 0.060 |
| Candida Albicans | 200/175 | 3.900 |
| Aspergillus Niger | 500/284 | 1.950 |
| Trichophyton Vaginalis | 111/216 | 62.500 |

Bacterial and fungal growth inhibition is achieved when the present 1 -alkyl-, 1-alkenyl-, and 1-alkynylaryl-2-amino-1,3-propanediols, and related compounds are administered to a subject requiring such treatment as an effective oral, parenteral, intravenous, or topical, including ophthalimic administration, dose of from 0.01 to 100 mg/kg of body weight per day. A particularly effective amount is about 25 mg/kg of body weight per day. It is to be understood, however, that for any particular subject, specific dosage regimens should be adjusted according to the individual need and the professional judgment of the person administering or supervising the administration of the aforesaid compound. It is to be further understood that the dosages set for herein are exemplary only and that they do not, to any extent, limit the scope or practice of the invention.

Compounds of the present invention include:

a. erythro-2-amino-1-(5-decyl-2-furyl)-1,3-dihydroxypropane;
b. erythro-2-amino-1-(5-decyl-3-isothiazolyl)-1,3-dihydroxypropane;
c. threo-2-amino-1-[5-decyl-3-(2-oxopyrrolyl)]-1,3-dihydroxypropane;
d. erythro-2-amino-1-[6-decyl-2-(4-methylpyridinyl)]-1,3-dihydroxypropane;
e. threo-2-amino-1-[6-decyl-2-(4-methoxypyridinyl)]-1,3-dihydroxypropane;
f. erythro-2-amino-1-[6-decyl-2-(5-chloropyridinyl)]-1,3-dihydroxypropane;

g. threo-2-amino-1-[6-decyl-2-(4-trifluoromethyl-pyridinyl)]-1,3-dihydroxypropane;
h. erythro-2-amino-1-[6-(5-phenylpentyl-2-pyridinyl)-1,3-dihydroxypropane;
i. erythro-2-amino-1-(2-decyl-4-thiazolyl)-1,3-dihydroxypropane;
j. erythro-2-amino-1-(2-decyl-4-oxazolyl)-1,3-dihydroxypropane;
k. erythro-2-methylamino-1-(5-decyl-2-thienyl)-1,3-dihydroxypropane
l. erythro-2-dimethylamino-1-(3-decyl)phenyl-1,3-dihydroxypropane;
m. erythro-2-(1,1-dimethylethoxy)carbonylamino-1-(2-dodecynyl-6-pyridinyl)-1,3-dihydroxypropane;
n. erythro-2-amino-1-(3-(1-decenyl)phenyl)-1,3-dihydroxypropane;
o. ethyl erythro-2-methoxycarbonylamino-3-(2-dodecynyl-6-pyridinyl)-3-hydroxypropionate;
p. erythro-2-amino-1-(3-(1-decynyl)phenyl)-1,3-dihydroxypropane;
q. erythro-2-amino-1-(3-(1-undecynyl)phenyl)-1,3-dihydroxypropane;
r. erythro-2-amino-1-(4-(1-nonyl)-2-thienyl)-1,3-dihydroxypropane;
s. erythro-2-amino-1-(4-(1-dodecynyl)-2-thienyl)-1,3-dihydroxypropane;
t. erythro-2-amino-1-(4-(1-decyl)-2-thienyl)-1,3-dihydroxypropane;
u. erythro-2-amino-1-(5-nonyl-2-thienyl)-1,3-dihydroxypropane;
v. erythro-2-amino-1-(3-dodecyl-5-isoxazolyl)-1,3-dihydroxypropane;
w. erythro-2-amino-1-(3-decyl-5-isoxazolyl)-1,3-dihydroxypropane;
x. erythro-2-amino-1-(6-(1-dodecenyl)-2-pyridinyl)-1,3-dihydroxypropane;
y. erythro-2-amino-1-(3-(6-phenyl-1-hexynyl)phenyl)-1,3-dihydroxypropane; and
z. erythro-2-amino-1-(5-(6-phenylhexyl)-2-thienyl)-1,3-dihydroxypropane.

Effective amounts of the compounds of the present invention may be administered topically to a subject in the form of sterile solutions, suspensions, ointments, creams, aerosols, or salves. The 1-alkyl-, 1-alkenyl, and 1-alkynylaryl-2-amino-1,3-propanediols of the present invention, while effective themselves, may be formulated and administered in the form of their pharmaceutically acceptable acid or base addition salts for purposes of stability, convenience or crystallization, increased solubility and the like.

Preferred pharmaceutically acceptable acid addition salts include salts of mineral acids, for example, hydrochloric acid, sulfuric acid, nitric acid and the like, salts of monobasic carboxylic acids such as, for example, acetic acid, propionic acid and the like, salts of dibasic carboxylic acids such as, for example, maleic acid, fumaric acid and the like, and salts of tribasic carboxylic acids such as, for example, carboxysuccinic acid, citric acid and the like. Preferred pharmaceutically acceptable base addition salts include salts of alkali metals, e.g. sodium or potassium, alkaline earth metals, e.g. calcium or magnesium; or complex salts such as ammonium or substituted ammonium salts such as a mono-, di- or trialkylammonium salts or a mono, di- or trihydroxyalkylammonium salts.

For the purpose of topical administration, the active compounds of the invention may be incorporated into a solution, suspension, ointment, cream, gel, aerosol, or salve. These preparations should contain at least 0.1% of active compound but may be varied to be between 0.05 and about 20% of the weight thereof. The amount of active compound in such compositions is such that a suitable dosage will be obtained. Preferred topically administered preparations should contain between 0.1 and 10% of active compound.

The topical compositions may also include the following components: water, fixed oils, polyethylene, glycols, glycerol, petroleum, stearic acid, beeswax, other synthetic solvents or mixtures thereof; antibacterial agents such as benzyl alcohol or methyl paraben; antioxidants such as $\alpha$-tocopherol acetate; chelating agents such as ethylenediaminetetraacetic acid; buffers such as acetates, citrates or phosphates; emulsifying agents such as polyoxyethylene monooleate and coloring materials and adjuvants such as ferric oxide or talc. The topical preparation can be enclosed in tubes, bottles, or jars made of metal, glass or plastic.

The active compounds of the present invention may also be administered orally, for example, with an inert diluent or with an edible carrier. They may be enclosed in gelatin capsules or compressed into tablets. For the purpose of oral therapeutic administration, the aforesaid compounds may be incorporated with excipients and used in the form of tablets, troches, capsules, elixirs, suspensions, syrups, wafers, chewing gums and the like. These preparations should contain at least 0.5% of active compound, but may be varied depending upon the particular form and may conveniently be between 4% to about 75% of the weight of the unit. The amount of present compound in such composition is such that a suitable dosage will be obtained. Preferred compositions and preparations according to the present invention are prepared so that an oral dosage unit form contains between 1.0-300 mgs of active compound.

The tablets, pills, capsules, troches and the like may also contain the following ingredients: a binder such as microcrystalline cellulose, gum tragacanth or gelatin; an excipient such as starch or lactose, a disintegrating agent such as alginic acid, Primogel, corn starch and the like; a lubricant such as magnesium stearate or Sterotes; a glidant such as colloidal silicon dioxide; and a sweetening agent such as sucrose or

saccharin or a flavoring agent such as peppermint, methyl salicylate, or orange flavoring may be added. When the dosage unit is a capsule, it may contain, in addition to materials of the above type, a liquid carrier such as a fatty oil. Other dosage unit forms may contain other various materials which modify the physical form of the dosage unit, for example, as coatings. Thus tablets or pills may be coated with sugar, shellac, or other enteric coating agents. A syrup may contain, in addition to the active compounds, sucrose as a sweetening agent and certain preservatives, dyes and colorings and flavors. Materials used in preparing these various compositions should be pharmaceutically pure and non-toxic in the amounts used.

For the purposes of parenteral therapeutic administration, the active compounds of the invention may be incorporated into a solution or suspension. These preparations should contain at least 0.1% of the aforesaid compound, but may be varied between 0.5 and about 50% of the weight thereof. The amount of active compound in such compositions is such that a suitable dosage will be obtained. Preferred compositions and preparations according to the present invention are prepared so that a parenteral dosage unit contains between 0.5 to 100 mgs of the active compound.

The oral solutions or suspensions may also include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite;
chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic.

The following Examples are for illustrative purposes only and are not to be construed as limiting the invention.

## EXAMPLE 1

### 6-(1-Dodecynyl)-2-pyridinecarboxaldehyde

To a solution of 6-bromo-2-pyridinecarboxaldehyde (3.0 g) in tetrahydrofuran (10 ml), was added sequentially bis(triphenylphosphine)palladium(II) chloride (0.178 g), copper(I)iodide (0.024 g), 1-dodecyne (3.25 ml), and triethylamine (2.12 ml). The solution was stirred at 40°C overnight. The reaction mixture was cooled to room temperature, charged again with bis(triphenylphosphine)palladium (II) chloride (0.024 g), copper (I) iodide (0.024 g), and triethylamine (2.12 ml), and 1-dodecyne (3.25 ml), and tetrahydrofuran (5.0 ml), and heated at 40°C for 5 hrs. The reaction mixture was again cooled and recharged as above, and heated at 40°C for 24 hrs. The cooled mixture was concentrated, taken up in ethyl acetate (100 ml), washed with water and saturated sodium chloride solution, dried over anhydrous magnesium sulfate, filtered, and the filtrate was concentrated. The residue was taken up in ethyl acetate (100 ml) and filtered. The filtrate was combined with material from a similar reaction run 1.43 g of the carboxaldehyde and proportionate amounts of the catalysts, 1-dodecyne, and solvent. The filtrate was concentrated. The residue was purified by flash chromatography using 1.5% ethyl acetate/hexane followed by 1% ethyl acetate/hexane as eluents. The appropriate fractions were collected and concentrated to yield 2.24 g (29%) of product, as an oil.

| Analysis: | | | |
|---|---|---|---|
| Calculated for $C_{18}H_{25}NO$: | 79.66%C | 9.28%H | 5.16%N |
| Found: | 79.54%C | 9.29%H | 4.98%N |

## EXAMPLE 2

### erythro-N-{1-[6-(1-Decynyl)-2-pyridinyl]1,3-dihydroxy-2-propanyl}acetamide

Ethyl erythro-2-acetamido-3-[6-(1-decynyl)-2-pyridinyl]-3-hydroxypropionate (5.71 g) in dry tetrahydrofuran (75 ml) was slowly added to 2.0 M lithium borohydride/tetrahydrofuran (7.6 ml) at 0° under nitrogen, and the mixture was stirred at room temperature overnight. The reaction mixture was chilled, and 1:1 methanol:water (50 ml) was added slowly followed by glacial acetic acid (0.5ml) until pH 6.5 was obtained. The reaction mixture was concentrated, and the residue azeotroped with methanol (4 x 40 ml). The residue was slurried with 7.5% sodium bicarbonate solution (15 ml) (pH 8.5), extracted into 3:1-trichloromethane:isopropanol and concentrated. The appropriate fractions were collected and concentrated. The residue was purified by flash chromatography on silica gel eluting with 49:1-ethyl acetate:methanol. The appropriate fractions were collected and concentrated to give 4.74 g (93%) of product, mp 85-87 °C.

| Analysis: | | | |
|---|---|---|---|
| Calculated for $C_{20}H_{30}N_2O_3$: | 69.33%C | 8.73%H | 8.09%N |
| Found: | 69.44%C | 8.84%H | 8.07%N |

## EXAMPLE 3

**erythro-N-{1-[6-(1-Decynyl)-2-pyridinyl]-1,3-diacetyloxy-2-propanyl}acetamide**

N-{1-[6-(1-Decynyl)-2-pyridinyl]-1,3-dihydroxy-2-propanyl}acetamide (4.35 g), acetic anhydride (7.45 ml), triethylamine (16 ml), and 4-dimethylaminopyridine (0.24 g) in dry tetrahydrofuran (80 ml) was stirred at room temperature for 3 days. The reaction mixture was evaporated, and the residue was warmed with methanol for 20 mins, reevaporated, and the residue was azeotroped with toluene. The residue was taken up in trichloromethane, and 7.5% sodium bicarbonate solution was added until pH 8.5 was obtained. The mixture was extracted with trichloromethane, dried over anhydrous magnesium sulfate, filtered, and the filtrate was concentrated. The residue was combined with the residue (1.0 g) from a reaction, starting with 0.829 g of acetamide, and purified by flash chromatography on silica gel eluting with 1:1-hexane:ethyl acetate. The appropriate fractions were collected and concentrated to yield 1.61 g (25%) of product.

| Analysis: | | | |
|---|---|---|---|
| Calculated for $C_{24}H_{34}N_2O_5$: | 66.95%C | 7.96%H | 6.51%N |
| Found: | 66.65%C | 8.04%H | 6.36%N |

## EXAMPLE 4

**threo-N{1-[6-(1-Decynyl)-2-pyridinyl]-1,3-diacetyloxy-2-propanyl}acetamide**

N-{1-[6-(1-Decynyl)-2-pyridinyl]-1,3-dihydroxy-2-propanyl}acetamide (4.35 g), acetic anhydride (7.45 ml), triethylamine (16 ml), and 4-dimethylaminopyridine (0.24 g) in dry tetrahydrofuran (80 ml) was stirred at room temperature for 3 days. The reaction mixture was evaporated, and the residue was warmed with methanol for 20 min, reevaporated and azeotroped with toluene. The residue was taken up in trichloromethane, and 7.5% sodium bicarbonate solution was added until pH 8.5 was obtained. The mixture was extracted with trichloromethane, dried over anhydrous magnesium sulfate, filtered, and concentrated. The residue was combined with 1.0 g of the residue from another reaction (0.829 g of acetamide), and purified by flash chromatography on silica gel, eluting with 1:1-hexane:ethyl acetate to yield 1.02 g (15.9%) of product, mp 59-61 °C.

| Analysis: | | | |
|---|---|---|---|
| Calculated for $C_{24}H_{34}N_2O_5$: | 66.95%C | 7.96%H | 6.51%N |
| Found: | 67.21%C | 7.83%H | 5.92%N |

## EXAMPLE 5

**Ethyl erythro-2-acetamido-3-[6-(1-decynyl)-2-pyridinyl]-3-hydroxypropionate**

A 2:1-erythro:threo mixture of ethyl 2-acetamido-3-(6-bromo-2-pyridinyl)-3-hydroxypropionate (10.0 g), 1-decyne (5.01 g), bis(triphenylphosphine)palladium chloride (0.42 g) and cuprous iodide (0.06 g) in triethylamine (50 ml) was heated at 50-60 °C for 2.5 hrs. under nitrogen, and then at room temperature

overnight. The reaction mixture was evaporated, water was added, and the mixture was extracted with ethyl acetate. The organic extract was purified by flash chromatography on silica gel, eluting with 1:1 hexane:ethyl acetate and collecting the appropriate fractions. The appropriate fractions were evaporated. Recrystallization of the residue from ethyl acetate gave 7.8 g (66%) of the product, mp 97-99 °C.

| Analysis: | | | |
|---|---|---|---|
| Calculated for $C_{22}H_{32}N_2O_4$:<br>Found: | 68.01%C<br>68.23%C | 8.30%H<br>8.28%H | 7.21%N<br>7.22%N |

**EXAMPLE 6**

**Ethyl threo-2-acetamido-3-[6-(1-decynyl-2-pyridinyl]-3-hydroxypropionate**

A mixture of ethyl 2-acetamido-3-(6-bromo-2-pyridinyl)-3-hydroxypropionate (17.3 g, 97% erythro), 1-decyne (8.67 g), bis(triphenylphosphine)palladium chloride (0.73 g), cuprous iodide (0.10 g), and triethylamine (13.2 g) in tetrahydrofuran (90 ml) was heated overnight at 50-55 °C, under nitrogen. The reaction mixture was evaporated, water was added, and the mixture was extracted with ethyl acetate, and concentrated. The residue was purified by flash chromatography on silica gel, eluting with 1:1-hexane:ethyl acetate. The appropriate fractions were collected and evaporated. Recrystallization of the residue from 1:2-hexane:ethyl acetate gave 14.7 g of 19:1-mixture erythro:threo-compounds, and from the mother liquors, 4.66 g of an 8:3 of mixture erythro:threo compounds. Flash chromatography of 2.63 g of the threo-enriched material on silica gel, eluting with 1:1-hexane:ethyl acetate, yielded 0.39 g (3.4%) of product, mp 97-99.5 °C.

| Analysis: | | | |
|---|---|---|---|
| Calculated for $C_{22}H_{32}N_2O_4$:<br>Found: | 68.01%C<br>67.89%C | 8.30%H<br>8.26%H | 7.21%N<br>7.10%N |

**EXAMPLE 7**

**erythro-N-{4-[6-(1-Decynyl)-2-pyridinyl]-2,2-dimethyl-1,3-dioxan-5-yl}acetamide**

N-{1-[6-(1-Decynyl)-2-pyridinyl]-1,3-dihydroxy-2-propanyl}acetamide (6.1 g, 3:1/erythro:threo mixture), p-toluenesulfonic acid (3.7 g), and 2,2-dimethoxypropane (43 ml) in dichloromethane (115 ml) were stirred at room temperature overnight, under nitrogen. The reaction mixture was extracted with 0.5 M sodium bicarbonate solution and water, dried over anhydrous magnesium sulfate, filtered, and the filtrate was evaporated. The residue was chromatographed on silica gel, eluting with 2:1-hexane:ethyl acetate to give 2.4 g (35%) of product, as an oil.

| Analysis: | | | |
|---|---|---|---|
| Calculated for $C_{23}H_{34}N_2O_3$:<br>Found: | 71.47%C<br>71.14%C | 8.87%H<br>9.12%H | 7.25%N<br>7.13%N |

**EXAMPLE 8**

**threo-N-{4-[6-(1-Decynyl)-2-pyridinyl]-2,2-dimethyl-1,3-dioxan-5-yl}acetamide**

N-{1-[6-(1-Decynyl)-2-pyridinyl]-1,3-dihydroxy-2-propanyl}acetamide (6.1 g, 3:1-erythro:threo mixture), p-toluenesulfonic acid (3.7 g), and 2,2-dimethoxypropane (43 ml) in dichloromethane (115 ml) were stirred at room temperature overnight, under nitrogen. The reaction mixture was washed with 0.5 M sodium bicarbonate solution and water, dried over anhydrous magnesium sulfate, filtered, and the filtrate was

evaporated. The residue was chromatographed twice on silica gel, eluting with 2:1-hexane:ethyl acetate to 1:1-hexane:ethyl acetate to give 0.76 g (11%) of product, as an oil.

| Analysis: | | | |
|---|---|---|---|
| Calculated for $C_{23}H_{34}N_2O_3$: | 71.47%C | 8.87%H | 7.25%N |
| Found: | 71.15%C | 8.91%H | 7.06%N |

## EXAMPLE 9

**Ethyl erythro-2-acetamido-3-[6-(1-dodecynyl)-2-pyridinyl]-3-hydroxypropionate**

A solution of 6-(1-dodecynyl)-2-pyridinecarboxaldehyde (5.51 g), acetamidomalonic acid monoethyl ester (3.78 g), and triethylamine (2.8 ml) in dry tetrahydrofuran (30 ml) was stirred at room temperature overnight, under nitrogen. The reaction mixture was evaporated, and the residue was purified on a silica gel column eluting with 1:1-hexane:ethyl acetate to give 7.46 g (89.6%) of product (10:1-erythro:threo mixture). This product was combined with 7.40 g from a prior reaction run on the same scale, and the combined material was recrystallized from 2:1-ethyl acetate:hexane to give 9.62 g (57.7%) of the analytically pure product, mp 86-87.5 ° C.

| Analysis: | | | |
|---|---|---|---|
| Calculated for $C_{24}H_{36}N_2O_4$: | 69.20%C | 8.71%H | 6.72%N |
| Found: | 69.40%C | 8.72%H | 6.68%N |

## EXAMPLE 10

**Ethyl erythro-2-acetamido-3-[6-(1-hexynyl)-2-pyridinyl]-3-hydroxypropionate**

An 11:1-erythro:threo mixture of ethyl 2-acetamido-3-(6-bromo-2-pyridinyl)-3-hydroxypropionate (24.9 g), 1-hexyne (7.39 g), triethylamine (19.0 g), bis(triphenylphosphine)palladium chloride (1.05 g) and cuprous iodide (0.14 g) in dry tetrahydrofuran (100 ml) was heated at 55 ° C for 6 hrs, under nitrogen. Additional 1-hexyne (6.2 g), triethylamine (7.6 g), bis(triphenylphosphine)palladium chloride (0.53 g), and cuprous iodide (0.07 g) were added at room temperature and the reaction mixture was heated an additional 5.5 hrs. The mixture was evaporated, water was added, and the mixture was extracted with ethyl acetate. The extract was flash chromatographed (silica gel, 1:1-hexane:ethyl acetate). The appropriate fractions were collected and evaporated. Recrystallization of the residue from 1:1-hexane:ethyl acetate provided 3.8 g (15%) of product, 87-88 ° C.

| Analysis: | | | |
|---|---|---|---|
| Calculated for $C_{18}H_{24}N_2O_4$: | 65.04%C | 7.28%H | 8.43%N |
| Found: | 65.19%C | 7.31%H | 8.37%N |

## EXAMPLE 11

**erythro-N-{1,3-Diacetyloxy-1-[6-(1-hexynyl)-2-pyridinyl]-2-propanyl}acetamide**

To ethyl erythro-2-acetamido-3-[6-(1-hexynyl)-2-pyridinyl}-3-hydroxypropionate (16.0 g) in dry tetrahydrofuran (140 ml) was added 2.0 M lithium borohydride:tetrahydrofuran (24 ml) at 0 ° C, with stirring, under nitrogen. After the addition was complete, the mixture was allowed to warm to room temperture and was stirred overnight. The reaction mixture was chilled and 1:1-methanol water (80 ml) was added slowly followed by acetic acid (2.8 ml) until a pH of 6.8 was obtained. The reaction mixture was stirred for 1 hr and evaporated. The residue was azeotroped several times with methanol. A 7.5% sodium bicarbonate solution

was added to the residue until a pH of 8.5 was obtained, and the mixture was extracted with 3:1 trichloromethane:isopropanol and concentrated. The residue was flash chromatographed on silica gel eluting with 1% methanol:ethyl acetate to give 13.2 g (94%) of erythro-N-{1-[6-(1-hexynyl)-2-pyridinyl]-1,3-dihydroxy-2-propanyl}acetamide.

erythro-N-{1-[6-(1-hexynyl)-2-pyridinyl]-1,3-dihydroxy-2-propanyl}acetamide(10.3 g), acetic anhydride (21.8 g), triethylamine (32.4 g), and 4-dimethylaminopyridine (0.44 g) in tetrahydrofuran (150 ml) was stirred at room temperature overnight. The reaction mixture was evaporated, methanol was added to the residue, and the solution was warmed at 50 ° C for 15 min. The mixture was evaporated. The residue was dissolved in chloroform, and 7.5% sodium bicarbonate solution was added until a pH 8 was obtained. The mixture was extracted with chloroform. The extract was dried over anhydrous magnesium sulfate, filtered, and the filtrate was concentrated. The residue was flash chromatographed, eluting with 1:1-hexane:ethyl acetate to yield 7.3 (55%) of product, mp 97-99 ° C.

| Analysis: | | | |
|---|---|---|---|
| Calculated for $C_{20}H_{26}N_2O_5$: | 64.16%C | 7.00%H | 7.48%N |
| Found: | 64.17%C | 7.00%H | 7.44%N |

## EXAMPLE 12

**erythro-N-{1-[6-(1-Hexynyl)-2-pyridinyl]-1,3-dihydroxy-2-propanyl}acetamide**

erythro-N-{1,3-Diacetyloxy-1-[6-(1-hexynyl)-2-pyridinyl]-2-propanyl}acetamide (6.7 g) and potassium carbonate (3.3 g) in methanol (100 ml) was stirred for 40 min. The precipitate was collected, and the filtrate was evaporated. A 7.5% sodium bicarbonate solution was added to pH 8.5, and the mixture was extracted with 3:1-trichloromethane:2-propanol, dried over anhydrous magnesium sulfate, filtered, and the filtrate was evaporated. Recrystallization of the residue from 1:1-hexane:ethyl acetate gave 4.6 g (88%) of product, mp 75-77 ° C.

| Analysis: | | | |
|---|---|---|---|
| Calculated for $C_{16}H_{22}N_2O_3$: | 66.19%C | 7.64%H | 9.65%N |
| Found: | 65.99%C | 7.55%H | 9.65%N |

## EXAMPLE 13

**Ethyl erythro-2-acetamido-3-hydroxy-3-[6-(1-octynyl)-2-pyridinyl]propionate**

A mixture of an 11:1-erythro:threo mixture of ethyl 2-acetamido-3-(6-bromo-2-pyridinyl)-3-hydroxypropionate (24.9 g), 1-octyne (9.9 g), triethylamine (19.0 g), bis(triphenylphosphine)palladium chloride (1.05 g) and cuprous iodide (0.14 g) in dry tetrahydrofuran (100 ml) was heated at 55 ° C for 6 hrs, under nitrogen. Additional 1-octyne (4.1 g), triethylamine (3.8 g), bis(triphenylphosphine)palladium chloride (0.53 g), and cuprous iodide (0.07 g) were added at room temperature, and the reaction mixture was heated an additional 4 hrs. The mixture was evaporated, water was added, and the mixture was extracted with ethyl acetate. The solution was flash chromatographed on silica gel eluting with 1:1-hexane:ethyl acetate. The fractions, enriched in the erythro isomer, were evaporated and the residue was recrystallized from 1:1-isopropanol:water to give 3.2 g (12%) of product, mp 81-83 ° C.

| Analysis: | | | |
|---|---|---|---|
| Calculated for $C_{20}H_{28}N_2O_4$: | 66.64%C | 7.83%H | 7.77%N |
| Found: | 66.62%C | 7.77%H | 7.75%N |

## EXAMPLE 14

**erythro-N-{1,3-Dihydroxy-1-[6-(1-octynyl)-2-pyridinyl]-2-propanyl}acetamide**

To ethyl erythro-2-acetamido-3-hydroxy-3-[6-(1-octynyl)-2-pyridinyl]-propionate (9.02 g) in dry tetrahydrofuran (80 ml) was added slowly 2.0 M lithium borohydride:tetrahydrofuran (12.5 ml) at 0°, under nitrogen. The mixture was stirred at room temperature overnight, chilled, and 1:1-methanol:water was added slowly followed by glacial acetic acid (1.5 ml) in 1:1-methanol:water (15 ml) until pH 6.8 was obtained. The solution was stirred at room temperature for 1.5 hrs, evaporated, and the residue was azeotroped with methanol (4 x 40 ml). The residue was slurried with 7.5% sodium bicarbonate solution (25 ml) (pH 8.5), saturated sodium chloride solution (25 ml), extracted with 3:1-trichloromethane:2-propanol, and concentrated. The residue was flash chromatographed on silica gel, eluting with ethyl acetate:0.5% methanol. The appropriate fractions were collected and evaporated. The residue was recrystallized (3 times) from ethyl acetate to give 1.24 g (15.6%) of product, mp 81-83°C.

| Analysis: | | | |
|---|---|---|---|
| Calculated for $C_{18}H_{26}N_2O_3$: | 67.90%C | 8.23%H | 8.80%N |
| Found: | 68.03%C | 7.97%H | 8.70%N |

## EXAMPLE 15

**Ethyl erythro-2-acetamido-3-[6-(1-hexadecynyl)-2-pyridinyl]-3-hydroxypropionate**

A solution of 6-(1-hexadecynyl)-2-pyridinecarboxaldehyde (17.4 g), acetamidomalonic acid monoethyl ester (10.6 g), and triethylamine (5.4 ml) in dry tetrahydrofuran (85 ml) was stirred at room temperature for 3 days, under nitrogen. The reaction mixture was evaporated and the residue was dissolved in ethyl acetate. The solution was washed with half-saturated sodium chloride solution, dried over anhydrous magnesium sulfate, filtered, and the filtrate was evaporated. The residue was purified on a silica gel column, eluting with 2:1- to 1:1-hexane:ethyl acetate. The appropriate fractions were collected and evaporated. The residue was recrystallized from ethanol and then 85% ethanol to give 12.8 g (50.9%) of product, mp 82.5-84°C.

| Analysis: | | | |
|---|---|---|---|
| Calculated for $C_{28}H_{44}N_2O_4$: | 71.15%C | 9.38%H | 5.93%N |
| Found: | 70.86%C | 9.18%H | 5.82%N |

## EXAMPLE 16

**erythro-N-{1-[6-(1-Dodecynyl)-2-pyridinyl]-1,3-dihydroxy-2-propanyl}acetamide**

To a solution of ethyl erythro-2-acetamido-3-[6-(1-dodecynyl)-2-pyridinyl]-3-hydroxypropionate (20.3 g) in dry tetrahydrofuran (250 ml) 2.0 M lithium borohydride:tetrahydrofuran (30 ml) was added at 0°, under nitrogen. The reaction mixture was stirred at room temperature overnight. The mixture was chilled and 1:1-methanol:water (100 ml) was added slowly followed by glacial acetic acid (3.5 ml) in 1:1-methanol:water (50 ml) until a pH of 6.5 was obtained. The solution was stirred at room temperature for 2 hrs, the solvents were evaporated, and the residue was azeotroped with methanol (5 x 100 ml). The residue was slurried with 7.5% sodium bicarbonate solution (65 ml) (pH 8.5), extracted into 3:1-chloroform:2-propanol, and concentrated. The residue was flash chromatographed on silica gel, eluting with 0.5% methanol:ethyl acetate. The appropriate fractions were collected and evaporated. Recrystallization of the residue from hexane:ethyl acetate/1:1 gave 15.5 g (85.0%) of product, mp 86-88°C.

| Analysis: | | | |
|---|---|---|---|
| Calculated for $C_{22}H_{34}N_2O_3$: | 70.55%C | 9.15%H | 7.48%N |
| Found: | 70.78%C | 9.35%H | 7.49%N |

**EXAMPLE 17**

**Ethyl erythro-2-acetamido-3-(6-decyl-2-pyridinyl)-3-hydroxypropionate**

Ethyl erythro-2-acetamido-3-[6-(1-decynyl)-2-pyridinyl]-3-hydroxypropionate (2.7 g) in ethanol (65 ml) was reduced using 5% palladium-on-charcoal (0.7 g) in a Parr hydrogenator at 40 psi of hydrogen. After 2.5 hrs, the catalyst was collected, the filtrate was evaporated, and the residue was recrystallized from ethyl acetate to give 2.11 g (77.6%) of product, mp 67-68.5°C.

| Analysis: | | | |
|---|---|---|---|
| Calculated for $C_{22}H_{36}N_2O_4$: | 67.32%C | 9.24%H | 7.14%N |
| Found: | 66.96%C | 9.13%H | 7.08%N |

**EXAMPLE 18**

**erythro-N-[1-(6-Decyl-2-pyridinyl)-1,3-dihydroxy-2-propanyl]acetamide**

erythro-N-{1-[6-(1-Decynyl)-2-pyridinyl]-1,3-dihydroxy-2-propanyl}-acetamide (4.0 g) in ethanol (100 ml) was reduced using 5% palladium-on-charcoal (0.1 g) in a Parr hydrogenator at 40 psi of hydrogen. After two hrs, the catalyst was collected, the solvent was evaporated, and the residue was recrystallized from ethyl acetate to give 3.69 g (91%) of product, mp 94-96°C.

| Analysis: | | | |
|---|---|---|---|
| Calculated for $C_{20}H_{34}N_2O_3$: | 68.54%C | 9.78%H | 7.99%N |
| Found: | 68.36%C | 9.72%H | 7.94%N |

**EXAMPLE 19**

**threo-2-Amino-1-(6-decyl-2-pyridinyl)-1,3-dihydroxypropane**

threo-N-{1-[6-(1-Decynyl)-2-pyridinyl]-1,3-diacetyloxy-2-propanyl}acetamide (1.0 g) in ethanol (55 ml) was reduced using 5% palladium-on-charcoal (0.06 g) in a Parr hydrogenator at 40 psi of hydrogen. After two hrs, the catalyst was collected, and the solvent was evaporated to give 0.95 g (94%) of threo-N-[3-(6-decyl-2-pyridinyl)-1,3-diacetyloxy-2-propanyl]acetamide.

The acetamide (0.95 g), hydrazine hydrate (40 ml), and ethanol (20 ml) was heated under reflux for 25 hrs, under nitrogen. The reaction mixture was cooled, water (30 ml) was added, and the mixture was extracted with ethyl acetate. The ethyl acetate layer was washed with saturated sodium chloride solution, dried over anhydrous magnesium sulfate, filtered, and the filtrate was evaporated. The residue was chromatographed on silica gel eluting with 980:20:2- to 970:30:2-trichloromethane:methanol:2N ammonium hydroxide. The appropriate fractions were collected and evaporated. The residue was dissolved in ethyl acetate, washed with half-saturated sodium chloride solution, dried, filtered, and the filtrate was evaporated to give 0.50 g (72%) of product, mp 76-78°C.

| Analysis: | | | |
|---|---|---|---|
| Calculated for $C_{18}H_{32}N_2O_2$:<br>Found: | 70.09%C<br>70.02%C | 10.46%H<br>10.63%H | 9.08%N<br>8.85%N |

**EXAMPLE 20**

**erythro-2-Amino-1-(6-decyl-2-pyridinyl)-1,3-dihydroxypropane**

erythro-N-[1-(6-Decyl-2-pyridinyl)-1,3-dihydroxy-2-propanyl]acetamide (6.0 g), hydrazine hydrate (60 ml), and ethanol (15 ml) were refluxed for 20 hrs., under nitrogen. The reaction mixture was cooled, water (75 ml) was added, and the mixture was extracted with ethyl acetate. The ethyl acetate layers were washed with saturated sodium chloride solution, dried over anhydrous magnesium sulfate, filtered, and the filtrate was evaporated. The residue was combined with 1.44 g from two other experiments, and was chromatographed on silica gel eluting with 950:50:3- to 900:100:5- trichloromethane:methanol:2N ammonium hydroxide. The appropriate fractions were collected and evaporated. The residue was dissolved in ethyl acetate (150 ml) and the solution was washed with half-saturated sodium chloride solution, dried over anhydrous magnesium sulfate, filtered, and the filtrate was evaporated to give 5.60 g (79%) of product, mp 52-55 °C.

| Analysis: | | | |
|---|---|---|---|
| Calculated for $C_{18}H_{32}N_2O_2$:<br>Found: | 70.09%C<br>69.63%C | 10.46%H<br>10.29%H | 9.08%N<br>8.87%N |

**EXAMPLE 21**

**Ethyl erythro-2-acetamido-3-(6-dodecyl-2-pyridinyl)-3-hydroxypropionate**

Ethyl erythro-2-acetamido-3-[6-(1-dodecynyl)-2-pyridinyl]-3-hydroxypropionate (2.0 g) in ethanol (80 ml) containing of 5% palladium-on-carbon (0.06 g) was reduced in a Parr hydrogenator at 40 psi of hydrogen. After two hrs, the catalyst was filtered, the filtrate was evaporated, and the residue was recrystallized from ethyl acetate to give 1.42 g (70.3%) of product, mp 71-73 °C.

| Analysis: | | | |
|---|---|---|---|
| Calculated for $C_{24}H_{40}N_2O_4$:<br>Found: | 68.54%C<br>68.74%C | 9.59%H<br>9.46%H | 6.66%N<br>6.69%N |

**EXAMPLE 22**

**erythro-N-[1-(6-Dodecyl-2-pyridinyl)-1,3-dihydroxy-2-propanyl]acetamide**

erythro-N-{1-[6-(1-Dodecynyl)-2-pyridinyl]-1,3-dihydroxy-2-propanyl}acetamide (6.05 g) in ethanol (120 ml) containing 5% palladium-on-carbon (0.15 g) was reduced in a Parr hydrogenator at 30 psi of hydrogen. After two hrs, the catalyst was filtered, the filtrate was evaporated, and the residue was recrystallized from ethyl acetate to give 5.90 g (96.4%) of product, mp 99-100.5 °C.

| Analysis: | | | |
|---|---|---|---|
| Calculated for $C_{22}H_{38}N_2O_3$:<br>Found: | 69.80%C<br>69.71%C | 10.12%H<br>10.37%H | 7.40%N<br>7.34%N |

## EXAMPLE 23

**erythro-2-Amino-1-(6-dodecyl-2-pyridinyl)-1,3-propanediol**

erythro-N-[1-(6-Dodecyl-2-pyridinyl)-1,3-dihydroxy-2-propanyl]acetamide (3.8 g), hydrazine hydrate (35 ml), and ethanol (20 ml) were refluxed under nitrogen for 24 hrs. The reaction mixture was cooled, water (50 ml) was added, and the mixture was extracted with chloroform (3 x 65 ml). The extracts were washed with saturated sodium chloride solution, dried over anhydrous magnesium sulfate, filtered, and the filtrate was evaporated. The residue was chromatographed on silica gel, eluting with 950:50:3-chloroform:methanol:2N ammonium hydroxide. The residue was dissolved in ethyl acetate and the solution was washed with half-saturated sodium chloride solution, dried over anhydrous magnesium sulfate, filtered, and the filtrate was evaporated to give 2.10 g (62%) of product, mp 61-64 °C .

| Analysis: | | | |
|---|---|---|---|
| Calculated for $C_{20}H_{36}N_2O_2$: | 71.38%C | 10.78%H | 8.32%N |
| Found: | 71.04%C | 10.95%H | 8.07%N |

## EXAMPLE 24

**erythro-N-1-(6-Hexyl-2-pyridinyl)-1,3-dihydroxy-2-propanyl]acetamide**

erythro-N-{1-[6-(1-Hexynyl)-2-pyridinyl]-1,3-dihydroxy-2-propanyl}acetamide (5.80 g) in ethanol (125 ml) was hydrogenated using 0.15 g of 5% palladium-on-carbon in a Parr system at 40 psi. After 2.5 hours, the catalyst was collected, the filtrate was evaporated, and the residue was recrystallised from ethyl acetate to give 5.2 g (88.6%) of product, mp 75-76.5 °C.

| Analysis: | | | |
|---|---|---|---|
| Calculated for $C_{16}H_{26}N_2O_3$: | 65.28%C | 8.90%H | 9.52%N |
| Found: | 65.18%C | 8.78%H | 9.50%N |

## EXAMPLE 25

**N,O,O-Tribenzyloxycarbonyl-erythro-2-amino-1-(6-decyl-2-pyridinyl)-1,3-propanediol**

erythro-2-Amino-1-(6-decyl-2-pyridinyl)-1,3-propanediol (1.50 g), N-benzyloxycarbonyloxysuccinimide (4.00 g) and triethylamine (2.23 ml) in dry tetrahydrofuran (60 ml) was stirred at room temperature for 9 days, under nitrogen. Additonal N-benzyloxycarbonyloxysuccinimide (4.00 g) was added and stirring was continued for 3 days. The reaction mixture was evaporated. The residue was dissolved in ethyl acetate and the solution was washed with saturated sodium chloride solution, dried over anhydrous magnesium sulfate, filtered, and the filtrate was evaporated. The residue was purified by flash chromatography (silica gel, 9:1 hexane:ethyl acetate). The appropriate fractions were collected and evaporated to give 1.87 g (54%) of product.

| Analysis: | | | |
|---|---|---|---|
| Calculated for $C_{42}H_{50}N_2O_8$: | 70.96%C | 7.09%H | 3.94%N |
| Found: | 71.00%C | 6.92%H | 3.77%N |

## EXAMPLE 26

**Ethyl erythro-2-acetamido-3-hydroxy-3-[3-(1-undecynyl)phenyl]propionate**

To a solution of 3-bromobenzaldehyde (30.3 g) and 1-undecyne (29.5 g) in triethylamine (120 ml) was added bis(triphenylphosphine)palladium(II) chloride (1.9 g) followed by copper(I) iodide (0.25 g). The mixture was stirred in the dark at 55°C for 6 hrs, under nitrogen. After cooling to room temperature, the reaction mixture was diluted with ethyl acetate and filtered. The filtrate was washed with water and saturated sodium chloride solution, dried over anhydrous magnesium sulfate, filtered, and the filtrate was concentrated to yield 45.8 g of 3-(1-undecynyl)benzaldehyde, as an oil.

A solution of 3-(1-undecynyl)benzaldehyde (23.0 g), acetamidomalonic acid monoethyl ester (15.1 g), and triethylamine (11.2 ml) in dry tetrahydrofuran (150 ml) was stirred at room temperature for 48 hrs, under nitrogen. Additional acetamidomalonic acid monoethyl ester (7.6 g) and triethylamine (5.6 ml) were added and stirring was continued for 72 hrs. The reaction mixture was evaporated and the residue was purified on a silica gel column, eluting with 2:1-hexane:ethyl acetate to give 13.0 g (41%) of product. The product was dissolved in warm 3:2-ethanol:water and cooled. The precipitate was collected. The filtrate was concentrated and the residue was recrystallized from cyclohexane to give the analytical sample, mp 69-71°C

| Analysis: | | | |
|---|---|---|---|
| Calculated for $C_{24}H_{35}NO_4$: | 71.79%C | 8.79%H | 3.49%N |
| Found: | 71.81%C | 8.72%H | 3.51%N |

## EXAMPLE 27

**Ethyl erythro-2-acetamido-3-[3-(1-dodecynyl)phenyl]-3-hydroxypropionate**

To a solution of 3-bromobenzaldehyde (26.5 g) and (25.0 g) 1-dodecyne in triethylamine (105 ml) was added bis(triphenylphosphine)palladium(II) chloride (1.73 g) followed by copper(I) iodide (0.24 g). The resultant mixture was stirred in the dark at 55°C for 7 hrs, under nitrogen. After cooling to room temperature, the reaction mixture was diluted with ethyl acetate and filtered. The filtrate was washed with water and saturated sodium chloride solution, dried over anhydrous magnesium sulfate, filtered, and concentrated to yield 37.7 g 3-(1-dodecynyl)benzaldehyde. The filtrate was an oil.

A solution of 3-(1-dodecynyl)benzaldehyde (7.6 g), acetamidomalonic acid monoethyl ester (5.1 g), and triethylamine (3.8 ml) in dry tetrahydrofuran (35 ml) was stirred at room temperature for 48 hrs, under nitrogen. Additional acetamidomalonic acid monoethyl ester (2.6 g) and triethylamine (1.9 ml) were added and stirring for 72 hr. The mixture was evaporated, and the residue was purified on a silica gel column, eluting with 2:1-hexane:ethyl acetate to give 4.8 g (43%) of product. The product was dissolved in warm 3:2-ethanol:water and cooled. The precipitate was collected. The filtrate was concentrated and the residue was recrystallized from 1:2-ethyl acetate:hexane to provide the analytical sample, mp 80-82°C.

| Analysis: | | | |
|---|---|---|---|
| Calculated for $C_{25}H_{37}NO_4$: | 72.26%C | 8.97%H | 3.37%N |
| Found: | 72.34%C | 8.74%H | 3.38%N |

## EXAMPLE 28

**cis-erythro-N-{1-[6-(1-Dodecenyl)-2-pyridinyl]-1,3-dihydroxy-2-propanyl}acetamide**

erythro-N-{1-[6-(1-Dodecynyl)-2-pyridinyl]-1,3-dihydroxy-2-propanyl}acetamide (2.05 g) in ethanol (55 ml), 5% palladium-on-barium sulfate (0.02 g), and 0.04 g of quinoline was hydrogenated at atmospheric pressure until one equivalent of hydrogen (ca. 123 ml) was taken up. The catalyst was filtered, the filtrate was evaporated, and the residue (2.1 g) was combined with residue (3.5 g) from similar reactions. The combined residues were chromatographed on silica gel eluting with 1:2-to 1:4-hexane:ethyl acetate to give

1.58 g (28%) of product, mp 96-98 °C.

| Analysis: | | | |
|---|---|---|---|
| Calculated for $C_{22}H_{36}N_2O_3$: | 70.18%C | 9.64%H | 7.44%N |
| Found: | 70.17%C | 9.67%H | 7.43%N |

**EXAMPLE 29**

**5-(1-Dodecynyl)-2-thiophenecarboxaldehyde**

A solution of 1-dodecyne (28.7 g), 5-bromo-2-thiophenecarboxaldehyde (30.0 g) and triethylamine (47.7 g) in dry tetrahydrofuran (75 ml) was degassed and stirred at room temperature under a nitrogen atmosphere. bis(Triphenylphosphine)palladium(II)chloride (two mole percent) followed by copper(I)iodide (one mole percent) was added to the mixture. The mixture was degassed again and stirred at room temperature for three hrs, under nitrogen. The precipitate was collected and washed with ethyl acetate, and the filtrate was evaporated. The residue was distilled in a kugelrohr (oven temp = 175 °C/0.1 mm Hg) to give 27.1 g (62%) of product, as a oil. A portion of the oil was purified by flash chromatography (silica; 7:3-hexane-dichloromethane) and dried at 50 °C under vacuum for three hrs to give the analytical sample

| Analysis: | | |
|---|---|---|
| Calculated for $C_{17}H_{24}OS$: | 73.86%C | 8.75%H |
| Found: | 73.86%C | 8.72%H |

**EXAMPLE 30**

**Ethyl erythro-2-acetamido-3-[5-(1-dodecynyl)-2-thienyl]-3-hydroxypropionate**

A slurry of 5-dodecynyl-2-thiophenecarboxaldehyde (31.8 g), acetamidomalonic acid monoethyl ester (21.7 g), and dry tetrahydrofuran (150 ml) was degassed and cooled to 0 °C. Triethylamine (5% excess) was added, the solution was degassed, and the reaction mixture was stirred at room temperature for 2 days, under nitrogen. Additional acetamidomalonic acid monoethyl ester (21.7 g) and triethylamine (5% excess) were added, and the reaction mixture was stirred at room temperature for 5 days, under nitrogen. The mixture was evaporated, and the residue was purified by flash chromatography (silica, 1:1-ethyl acetate:hexanes). The appropriate fractions were collected and evaporated. The residue was recrystallized from ether and from ethyl acetate-hexane to give 29.5 g (61%) of product, mp 81-83 °C.

| Analysis: | | | |
|---|---|---|---|
| Calculated for $C_{23}H_{35}NO_4S$: | 65.53%C | 8.37%H | 3.32%N |
| Found: | 65.36%C | 8.25%H | 3.30%N |

**EXAMPLE 31**

**erythro-N-[1-[5-(1-dodecynyl)-2-thienyl]-1,3-dihydroxy-2-propanyl]acetamide**

A solution of ethyl erythro-2-acetamido-3-[5-(1-dodecynyl)-2-thienyl]-3-hydroxypropionate (15.0 g) in dry tetrahydrofuran (150 ml) was stirred at 0 °C, under nitrogen, as 2M lithium borohydride in tetrahydrofuran (22.3 ml) was added dropwise. The reaction mixture was stirred at room temperature for 3 days, under nitrogen. The pH of the mixture was adjusted to 6 with glacial acetic acid, and the mixture was evaporated. The residue was diluted with water (100 ml) and extracted with ethyl acetate. The combined organic extracts were dried over anhydrous magnesium sulfate, filtered, and the filtrate was evaporated. The residue was purified by flash chromatography (silica, 1-5% methanol-ethyl acetate). The appropriate fractions were

collected and evaporated. The residue was recrystallized from ethyl acetate-hexane to give 9.6 g (71%) of product, mp 83-85 ° C.

| Analysis: | | | |
|---|---|---|---|
| Calculated for $C_{21}H_{33}NO_3S$: | 66.45%C | 8.76%H | 3.69%N |
| Found: | 66.47%C | 8.53%H | 3.75%N |

## EXAMPLE 32

**erythro-2-Amino-1-[5-(1-dodecynyl)-2-thienyl]-1,3-propanediol**

A solution of erythro-N-[1-[5-(1-dodecynyl)-2-thienyl]-1,3-dihydroxy2-propanyl]acetamide (3.00 g), 2N sodium hydroxide solution (100 ml) and 95% ethanol (50 ml) was stirred at 65 ° C overnight. After cooling to room temperature, the mixture was evaporated, and the residue was diluted with sodium bicarbonate solution (250 ml). The mixture was extracted with 3:1-chloroform:isopropanol and the combined organic layers were dried anhydrous sodium sulfate, filtered, and the filtrate was evaporated. The residue was purified by flash chromatography (silica gel, 90:9:1-dichloromethane: methanol:ammonium hydroxide). The appropriate fractions were collected and evaporated. The residue was crystallized from ethyl acetate:hexane to give 1.4 g (53%) of product, mp 77-78 ° C.

| Analysis: | | | |
|---|---|---|---|
| Calculated for $C_{19}H_{31}NO_2S$: | 67.61%C | 9.26%H | 4.15%N |
| Found: | 67.61%C | 8.63%H | 4.16%N |

## EXAMPLE 33

**erythro-N-[1-[5-(1-Dodecyl)-2-thienyl]-1,3-dihydroxy-2-propanyl]acetamide**

A mixture of erythro-N-[1-[5-(1-dodecynyl)-2-thienyl]-1,3-dihydroxy-2-propyl]acetamide (8.00 g), 5% palladium-on-carbon (400 mg), and absolute ethanol (500 ml) was shaken on a Parr hydrogenator under 50 psi of hydrogen for three hrs. The catalyst was collected. Fresh catalyst (400 mg) was added to the filtrate and the mixture was shaken under 50 psi of hydrogen overnight. The mixture was filtered through a bed of celite, and the filter cake washed with ethanol. The filtrate was evaporated, and the residue was recrystallized from ethyl acetate to give 7.3 g (90%) of product, m.p. 104-106 ° C.

| Analysis: | | | |
|---|---|---|---|
| Calculated for $C_{21}H_{37}NO_3S$: | 65.75%C | 9.72%H | 3.65%N |
| Found: | 65.45%C | 9.58%H | 3.67%N |

## EXAMPLE 34

**erythro-2-Amino-1-[5-(1-dodecyl)-2-thienyl]-1,3-propanediol**

A solution of erythro-N-[3-[5-(1-dodecyl)-2-thienyl]-1,3-dihydroxy-2-propyl]acetamide (3.00 g), hydrazine monohydrate (35 ml) and absolute ethanol (25 ml) was stirred at 70 ° C for 48 hrs, under nitrogen. The reaction mixture was cooled to room temperature, poured into 300 ml of dilute sodium bicarbonate solution (300 ml), and extracted with chloroform. The combined organic layers were dried over anhydrous sodium sulfate, filtered, and the filtrate was evaporated. The residue was purified by flash chromatography (silica; 90:9:1-dichloromethane:methanol:ammonium hydroxide). The appropriate fractions were collected and evaporated. The residue was recrystallized from ethyl acetate:hexane to give 1.4 g (52%) of product, m.p.89-90 ° C.

| Analysis: | | | |
|---|---|---|---|
| Calculated for $C_{19}H_{35}NO_2S$:<br>Found: | 66.81%C<br>66.48%C | 10.33%H<br>10.37%H | 4.10%N<br>4.11%N |

**EXAMPLE 35**

**Ethyl erythro-2-acetamido-3-(3-dodecyl-5-isoxazolyl)-3-hydroxypropionate**

A mixture of 3-dodecyl-5-isoxazolecarboxaldehyde (5.72 g) and acetaminomalonic acid monoethylester (4.06 g) in dry tetrahydrofuran (75 ml) was cooled to 0°, with stirring, and triethylamine (2.29 g) was added. The reaction mixture was allowed to warm to room temperature and was stirred for 16 hrs. The solution was evaporated and the residue was purified by flash chromatography (silica gel, 2:1-ethyl acetate:hexanes). The appropriate fractions were collected and evaporated. Recrystallization of the residue from ethyl acetate:hexane gave 4.65 g (52.6%) of product, mp 87-89°C.

| Analysis: | | | |
|---|---|---|---|
| Calculated for $C_{22}H_{38}N_2O_5$:<br>Found: | 64.36%C<br>64.55%C | 9.33%H<br>9.08%H | 6.82%N<br>6.76%N |

**EXAMPLE 36**

**erythro-N-[1-[3-(1-Dodecyl)-5-isoxazolyl]-1,3-dihydroxy-2-propyl]acetamide.**

To a solution of freshly prepared calcium borohydride (0.61 g) (from calcium hydride and borane-dimethyl sulfide) in dry tetrahydrofuran (70 ml) was added a solution of ethyl erythro-2-acetamido-3-(3-dodecyl-5-isoxazolyl)-3-hydroxypropionate (2.4 g) in dry tetrahydrofuran (20 ml). The reaction mixture was stirred at room temperature for 3 hrs. The reaction was quenched with 90:10:5-mixture of water, methanol, acetic acid, and extracted with chloroform. The solution was evaporated and the residue was recrystallized twice from ethyl acetate-hexane to give 1.23 g (57.1%) of product, mp 88-90°C.

| Analysis: | | | |
|---|---|---|---|
| Calculated for $C_{20}H_{36}N_2O_4$:<br>Found: | 65.19%C<br>65.17%C | 9.85%H<br>9.60%H | 7.60%N<br>7.60%N |

**EXAMPLE 37**

**Ethyl erythro-2-acetamido-3-(6-bromo-2-pyridinyl)-3-hydroxypropionate**

A solution of 6-bromo-2-pyridinecarboxaldehyde (5.6 g), acetamidomalonic acid monoethyl ester (5.67 g), and triethylamine (4.2 ml) in dry tetrahydrofuran (40 ml) was stirred at room temperature overnight, under nitrogen. The reaction mixture was evaporated and the mixture was purified on a silica gel column, eluting with 1:1-hexane:ethyl acetate to give 7.9 g (80%) of product as a mixture of two diastereomers. Recrystallization from toluene, followed by ethyl acetate gave 2.15 g (21.6%) of the erythro product, mp 98-100°C.

| Analysis: | | | |
|---|---|---|---|
| Calculated for $C_{12}H_{15}BrN_2O_4$:<br>Found: | 43.52%C<br>43.56%C | 4.57%H<br>4.53%H | 8.46%N<br>8.42%N |

## EXAMPLE 38

**erythro-N-[1-(6-Bromo-2-pyridinyl)-1,3-dihydroxy-2-propanyl]acetamide**

To a 2:1-erythro:threo mixture of ethyl 2-acetamido-3-(6-bromo-2-pyridinyl)-3-hydroxypropionate (11.4 g) in dry tetrahydrofuran (60 ml) was added slowly 2.0 M lithium borohydride/tetrahydrofuran (20.6 ml) at 0°, under nitrogen, and the mixture was stirred at room temperature overnight. The reaction mixture was chilled, and 1:1-methanol:water (100 ml) was added slowly followed by glacial acetic acid (2 ml) until pH 6.5 was obtained. The mixture was evaporated and the residue azeotroped with methanol (6 x 50 ml). The residue was slurried with 7.5% sodium bicarbonate solution (40 ml) (pH 8.5), extracted with 3:1-trichloromethane:isopropanol, filtered, and the filtrate was concentrated. The residue was flash chromatographed on silica gel eluting with 19:1-ethyl acetate:methanol to give 8.9(93.7%) of product. Recrystallization from ethanol gave the analytical sample of the erythro-diastereomer, mp 134.5-136.5°C.

| Analysis: | | | |
|---|---|---|---|
| Calculated for $C_{10}H_{13}BrN_2O_3$: | 41.54%C | 4.53%H | 9.69%N |
| Found: | 41.64%C | 4.54%H | 9.64%N |

## EXAMPLE 39

**6-(1-Hexadecynyl)-2-pyridinecarboxaldehyde**

A solution of 6-bromo-2-pyridinecarboxaldehyde (12.3 g), 1-hexadecyne (16.1 g), triethylamine (20.0 g), bis(triphenylphosphine)palladium(II)chloride (0.92 g), and copper(I)iodide (0.13 g) in dry tetrahydrofuran (55 ml) was heated at 50°C for 29 hrs, under nitrogen. The reaction mixture was cooled to room temperature, filtered, and the filter cake was washed with ethyl acetate. The filtrate was evaporated and the residue was taken up in ethyl acetate (100 ml). The mixture was washed with 1:1 -water:saturated sodium chloride solution, dried over anhydrous magnesium sulfate, filtered, and the filtrate was evaporated. The residue was flash chromatographed on silica gel, eluting with 1% ethyl acetate:hexanes. The appropriate fractions were collected and evaporated. The residue was dissolved in ethyl acetate and the solution was washed with half-saturated sodium chloride solution, dried over anhydrous magnesium sulfate, filtered, and the filtrate was evaporated to give 11.5 g (53.5%) of product, mp 38.5-40°C.

| Analysis: | | | |
|---|---|---|---|
| Calculated for $C_{22}H_{33}NO$: | 80.68%C | 10.16%H | 4.28%N |
| Found: | 80.44%C | 10.00%H | 4.30%N |

## EXAMPLE 40

**3-(1-Dodecyl)-5-isoxazolemethanol**

To a solution of 1-nitrotridecane (10.5 g) and O-trimethylsilyl-propynol (5.88 g) in dry benzene (100 ml) was added dropwise a solution of freshly distilled phenylisocyanate (10.9 g) and triethylamine (5.56 g) in dry benzene (40 ml) at 40 °C, with mechanical stirring. The mixture was heated to 60°C for 3.5 hr, cooled, and filtered. The filtrate was evaporated, taken up in tetrahydrofuran (300 ml) and 1.0M tetrabutylammonium fluoride (8 ml) was added. After 30 mins, the mixture was evaporated and the residue was purified by flash chromatography (silica gel, 2% methanol:dichloromethane). The appropriate fractions were collected and evaporated to give 10.5 g (86%) of product, mp 61-63°C.

| Analysis: | | | |
|---|---|---|---|
| Calculated for $C_{16}H_{29}NO_2$:<br>Found: | 71.87%C<br>71.92%C | 10.93%H<br>11.10%H | 5.24%N<br>5.19%N |

## EXAMPLE 41

### 3-(1-Dodecyl)-5-isoxazolecarboxaldehyde

A solution of oxalyl chloride (28.8 ml) in dry dichloromethane (100 ml) was cooled to -60°C and a solution of dimethylsulfoxide (8.9 ml) in dichloromethane (30 ml) was added, followed by a slurry of 3-(1-dodecyl)-5-isoxazolemethanol (14.0 g) in dry dichloromethane (200 ml). The mixture was stirred at -60°C for 1 hr, quenched with triethylamine (87 ml) and allowed to warm to room temperature. The solution was poured into water (300 ml) and extracted with dichloromethane. The organic phases were washed with dilute citric acid solution, dried, filtered, and the filtrate was evaporated. The residue was passed through a short pad of silica gel using dichloromethane as the eluent. The solution was evaporated and the residue was recrystallized from ether-hexane to give 11.5 g (78%) of product, mp 53-54°C.

| Analysis: | | | |
|---|---|---|---|
| Calculated for $C_{16}H_{27}NO_2$:<br>Found: | 72.41%C<br>72.22%C | 10.25%H<br>10.59%H | 5.28%N<br>5.24%N |

## EXAMPLE 42

### erythro-N-{1-[6-(1-Hexadecynyl)-2-pyridinyl]-1,3-dihydroxy-2-propanyl}acetamide

To ethyl erythro-2-acetamido-3-{6-(1-hexadecynyl)-2-pyridinyl]-3-hydrox ypropionate (11.1 g), in tetrahydrofuran (100 ml) dry was added slowly 2.0 M lithium borohydride/tetrahydrofuran (11.8 ml) at 0° under nitrogen. The reaction mixture was stirred at ambient temperature for 2.5 hrs, chilled, and 1:1 -methanol:water (60 ml) and glacial acetaic acid (1.4 ml) was added slowly until a pH of 6.5 was obtained. The mixture was then strirred at ambient temperature for 0.5 hr and evaporated. The residue was azeotroped with methanol (4 x 40 ml), slurried with 7.5% sodium bicarbonate solution (25 ml) (pH 8.5), and the mixture was extracted with 3:1-chloroform:2-propanol. The solution was concentrated and the residue was flash chromatographed on silica gel, eluting with 99:1-ethyl acetate:methanol. The appropriate fractions were collected and evaporated. Recrystailization gave 8.57 g (84.4%) of product, mp 88-90°C.

| Analysis: | | | |
|---|---|---|---|
| Calculated for $C_{26}H_{42}N_2O_3$:<br>Found: | 72.52%C<br>72.55%C | 9.83%H<br>9.46%H | 6.51%N<br>6.54%N |

## EXAMPLE 43

### erythro-N-[1,3-Diacetyloxy-1-(6-hexadecyl-2-pyridinyl)-2-propanyl]acetamide

A mixture of N-{1-[6-(1-hexadecynyl)-2-pyridinyl]-1,3-dihydroxy-2-propanyl }acetamide. (7.26 g, 5:2-erythro:threo mixture), acetic anhydride (10.5 g), triethylamine (15.5 g), and 4-dimethylaminopyridine (0.21 g), in tetrahydrofuran (100 ml) was stirred at ambient temperature overnight. The reaction mixture was evaporated, methanol was added, and the mixture was warmed at 50°C for 20 min, and then was concentrated. A solution of 7.5% sodium bicarbonate solution was added to the residue until a pH of 8.5 was obtained. The mixture was extracted with chloroform. The organic extract was dried over anhydrous magnesium sulfate, filtered and the filtrate was concentrated. The residue was flash chromatographed, eluting with 2:1 to 1:1-hexane:ethyl acetate. The appropriate fractions were collected and evaporated to

yield 5.54 g (64%) of erythro-N-[1,3-diacetyloxy-1-(6-hexadecynyl-2-pyridinyl)-2-propanyl]acetamide.

A mixture of erythro-N-[1,3-diacetyloxy-1-(6-hexadecynyl-2-pyridinyl-2-propanyl]acetamide (5.4 g) in ethanol (150 ml) and 5% palladium-on-carbon (0.20 g) was shaken on a Parr hydrogenator under 35 psi of hydrogen for 2.5 hrs. The catalyst was collected and the filtrate was evaporated. The residue was chromatographed to give 4.1 g (75%; 48% overall) of product, mp 79-80.5 °C.

| Analysis | | | |
|---|---|---|---|
| Calculated for $C_{30}H_{50}N_2O_5$: | 69.46%C | 9.72%H | 5.40%N |
| Found: | 69.81%C | 9.60%H | 5.41%N |

## EXAMPLE 44

### erythro-N-[1-(6-Hexadecyl-2-pyridinyl)-1,3-dihydroxy-2-propanyl]acetamide

A mixture of erythro-N-{1-[6-(1-hexadecynyl)-2-pyridinyl]-1,3-dihydroxy-2-propanyl}acetamide (3.9 g), ethanol (125 ml) and 5% palladium-on-carbon (0.20 g) was shaken on a Parr hydrogenator under 35 psi of hydrogen for 2 hrs. The catalyst was collected and the filtrate was evaporated. The residue was recrystallized from ethyl acetate to give 3.6 g (91%) of product, mp 98-101 °C.

| Analysis: | | | |
|---|---|---|---|
| Calculated for $C_{26}H_{46}N_2O_3$: | 71.85%C | 10.67%H | 6.44%N |
| Found: | 71.92%C | 10.75%H | 6.52%N |

## EXAMPLE 45

### erythro-2-Amino-(6-hexadecyl-2-pyridinyl)-1,3-propanediol

erythro-N-[1-(6-hexadecyl-2-pryidinyl)-1,3-dihydroxy-2-propanyl]acetamide (3.0 g), hydrazine hydrate (35 ml), and ethanol (25 ml) were heated under reflux, under nitrogen, for 28 hrs. The reaction mixture was cooled, water (40 ml) was added, and the mixture was extracted with chloroform. The combined extracts were washed with saturated sodium chloride solution, dried over anhydrous magnesium sulfate, filtered, and the filtrate was evaporated. The residue was chromatographed on silica gel, eluting with 950:50:3-chloroform:methanol:2N ammonium hydroxide. The appropriate fractions were collected and concentrated. The residue was dissolved in ethyl acetate and the solution was washed with half-saturated sodium chloride solution, dried, over anhydrous magnesium sulfate, filtered, and the filtrate was evaporated. The residue was azeotroped with toluene to give 1.37 g (50%) of product, mp 67-69 °C.

| Analysis: | | | |
|---|---|---|---|
| Calculated for $C_{24}H_{44}N_2O_2$: | 73.42%C | 11.30%H | 7.13%N |
| Found: | 73.25%C | 11.14%H | 7.04%N |

## EXAMPLE 46

### Ethyl threo-2-acetamido-3-(6-bromo-2-pyridlnyl)-3-hydroxypropionate

A solution of 6-bromo-2-pyridinecarboxaldehyde (30.3 g), acetamidomalonic acid monoethyl ester (34.1 g), and triethylamine (16.6 g) in dry tetrahydrofuran (170 ml) was stirred under nitrogen at ambient temperature overnight. The reaction mixture was concentrated and the residue was azeotroped three times with ethyl acetate, and then recrystallized from ethyl acetate to remove 36.4 g of ethyl erythro-2-acetamido-3-(6-bromo-2-pyridinyl)-3-hydroxyproprionate. The filtrate was chromatographed on a silica gel column, eluting with 3:2 to 1:1-hexane:ethyl acetate. The appropriate fractions were collected and concentrated. The

residue was recrystallized from ethyl acetate to give 1.0 g (2.0%) of product, mp 144-146 °C.

| Analysis: | | | |
|---|---|---|---|
| Calculated for $C_{12}H_{15}BrN_2O_4$: | 43.52%C | 4.57%H | 8.46%N |
| Found: | 44.02%C | 4.52%H | 8.39%N |

## EXAMPLE 47

**6-(1-Undecynyl)pyridine-2-carboxaldehyde**

A solution of 6-bromopyridine-2-caboxaldehyde (15.0 g), 1-undecyne (12.9 g), triethylamine (24.5 g), bis(triphenylphosphine)palladium(II)chloride (1.1g, 2%), and copper(I)iodide (0.15g, 1%) in dry tetrahydrofuran (60 ml) was heated under nitorgen at 55 °C for 10 hrs. The reaction mixture was filtered, the filter cake was washed with ethyl acetate, and the filtrate was evaporated. The residue was dissolved in ethyl acetate, and the solution was washed with half-saturated sodium chloride solution, dried over anhydrous magnesium sulfate, filtered, and the filtrate was concentrated. The residue was flash chromatographed on silica gel, eluting with 0%-2%-ethyl acetate:hexane. The appropriate fractions were collected and evaporated to yield 17.5 g (84.0%) of product.

| Analysis: | | | |
|---|---|---|---|
| Calculated for $C_{17}H_{23}NO$: | 79.33%C | 9.01%H | 5.44%N |
| Found: | 79.03%C | 9.36%H | 5.14%N |

## EXAMPLE 48

**Ethyl erythro-2-acetamido-3-hydroxy-3-[6-(1-undecynyl)-2-pyridinyl]propionate**

A mixture of ethyl erythro-2-acetamido-3-(6-bromo-2-pyridinyl)-3-hydroxypropionate (20.8 g), 1-undecyne (1 1.5 g), triethylamine (12.7 g), bis(triphenylphosphine)palladium chloride (0.88 g), and cuprous iodide (0.12 g) in dry tetrahydrofuran (100 ml) was heated at 55 °C for 4 hrs under nitrogen. Additional 1-undecyne (2.9 g), triethylamine (3.2 g), bis(triphenylphosphine)palladium chloride (0.44 g), and cuprous iodide (0.06 g) were added at ambient temperature, and the reaction mixture was heated an additional 5 hrs. The reaction mixture was evaporated and the residue was dissolved in ethyl acetate. The solution was washed with half-saturated sodium chloride solution and the organic phase was flash chromatographed on silica gel, eluting with 3:2 to 1:1-hexane:ethyl acetate. The appropriate fractions were collected and evaporated. Recrystallization of the residue from ethyl acetate save 16.0 g (63.4%) of product, mp 92-93 °C.

| Analysis: | | | |
|---|---|---|---|
| Calculated for $C_{23}H_{34}N_2O_4$: | 68.63%C | 8.51%H | 6.96%N |
| Found: | 68.58%C | 8.94%H | 6.94%N |

## EXAMPLE 49

**erythro-N-{1,3-Dihydroxy-1-[6-(1-undecynyl)-2-pyridinyl]-2-propanyl}acetamide**

To ethyl erythro-2-acetamido-3-hydroxy-3-[6-(-1-undecynyl)-2-pyridinyl]propionate (17.9 g), in dry tetrahydrofuran (150 ml) was added 2.0 M lithium borohydride/tetrahydrofuran (22 ml) at 0 °C under nitrogen. The reaction mixture was chilled, stirred at ambient temperature overnight, and 1:1-methanol:water (30 ml) was added followed by of glacial acetic acid (2.8 ml) in 1:1-methanol:water (30 ml) until a pH of 6.4 was obtained. The solution was stirred at ambient temperature for 1 hr, evaporated, and the residue was azeotroped with methanol. The residue was slurried with 75% sodium bicarbonate solution (pH 8.5),

extracted with 3:1-chloroform:2-propanol, and concentrated. The residue was flash chromatographed on silica gel, eluting with 0.5%-1% methanol:ethyl acetate. The appropriate fractions were collected and concentrated. The residue was recrystallized from 1:1-hexane:ethyl acetate to give 12.1 g (75.4%) of product, mp 95-96.5 ° C.

| Analysis: | | | |
|---|---|---|---|
| Calculated for $C_{21}H_{32}N_2O_3$: | 69.97%C | 8.95%H | 7.77%N |
| Found: | 69.84%C | 8.87%H | 7.71%N |

**EXAMPLE 50**

**(Z)-erythro-N-{1,3-Dihydroxy-1-{6-(1-octenyl)-2-pyridinyl]-2-propanyl}acetamide**

A solution of ethyl erythro-2-acetamido-3-[6-(1-octynyl)-2-pyridinyl]-3-hydroxypropionate (9.0 g), in dry tetrahydrofuran (80 ml) and 2.0 M lithium borohydride/tetrahydrofuran (12.5 ml), was stirred under nitrogen at $0^0$C. The reaction mixture was stirred at ambient temperature overnight, chilled , and 1:1-methanol:water (40 ml) and acetic acid (1.5 ml) was added until a pH of 6.8 was obtained. The reaction mixture was stirred 1 hr, evaporated, and the residue was azeotroped with methanol. 7.5% Sodium bicarbonate solution was added until a pH of 8.5 was obtained. The mixture was extracted with 3:1-chloroform:-2-propanol and concentrated. The residue was flash chromatographed on silica gel, eluting with 1% methanol/ethyl acetate to give 7.6 g of material.

Part of the above material (5.0 g) and 8.35 g from a similar experiment, acetic anhydride (25.7 g), triethylamine (38.2 g), and 4-dimethylaminopyridine (0.51 g) in tetrahydrofuran (180 ml ) was stirred at ambient temperature for 3 hrs. The reaction mixture was evaporated, methanol was added, and the mixture was warmed at 50 ° C for 20 min, and evaporated. A 7.5% sodium bicarbonate solution was added until a pH of 8.5 was obtained and the mixture was extracted with chloroform. The extract was dried over anhydrous magnesium sulfate, filtered, and the filtrate was concentrated. The residue was flash chromatographed, eluting with 2:1-hexane:ethyl acetate. The appropriate fractions were collected and concentrated to yield 1.1 g of (Z)-erythro-N-{1,3-diacetyloxy-1[6-(1-octenyl)-2-pyridinyl]-2-propanyl}acetamide.

A solution of (Z)-erythro-N-{1,3-diacetyloxy-1-[6-(1-octenyl)-2-pyridinyl]-2-propanyl}acetamide (1.1 g), potassium carbonate (0.44 g) in methanol (18 ml) was stirred for 1 hr. The reaction mixture was filtered and the filtrate was concentrated. The resiude was chromatographed on silica gel, eluting with 0.5% methanol/ethyl acetate. The appropriate fractions were collected and concentrated to give 0.5 g (3.7% overall) of product.

| Analysis: | | | |
|---|---|---|---|
| Calculated for $C_{18}H_{28}N_2O_3$: | 67.47%C | 8.81%H | 8.74%N |
| Found: | 67.60%C | 8.96%H | 8.72%N |

**EXAMPLE 51**

**erythro-N-[1,3-Diacetyloxy-1-(6-octyl-2-pyridinyl)-2-propanyl]acetamide**

A solution of erythro-N-(1,3-dihydroxy-1-1-{[6-(1-octynyl)-2-pyridinyl]-2-propanyl} acetamide (13.4 g), acetic anhydride (25.7 g), triethylamine (38.2 g), and 4-dimethylaminopyridine (0.51 g) in tetrahydrofuran (180 ml) was stirred at ambient temperature for 3 hrs. The reaction mixture was evaporated, methanol was added to the residue, and the solution was warmed at 50 ° C for 20 min and evaporated. A solution of 7.5% sodium bicarbonate solution was added until a pH of 8.5 was obtained, and the mixture was extracted with chloroform. The extract was dried over anhydrous magnesium sulfate, filtered, and the filtrate was concentrated. The residue was flash chromatographed, eluting with 2:1 to 1:1-hexane:ethyl acetate. The appropriate fractions were collected and evaporated to yield 9.17 g of erythro-N-[1,3-diacetyloxy-1-(6-octynyl-2-pyridinyl)-2-propanyl]acetamide.

A portion of erythro-N-[1,3-diacetyloxy-1-(6-octynyl-2-pyridinyl)-2-propanyl]acetamide (7.5 g) in ethanol (200 ml) and 5% palladium-on-carbon (0.25 g) was shaken on a Parr hydrogenator at 40 psi of hydrogen. After 1.5 hrs, the catalyst was collected and the filtrate was evaporated. The residue was chromatographed to give 6.0 g (78.5%, 43% overall) of product, mp 53-56 °C.

| Analysis: | | | |
|---|---|---|---|
| Calculated for $C_{22}H_{34}N_2O_5$: | 65.00%C | 8.43%H | 6.89%N |
| Found: | 65.07%C | 8.32%H | 6.88%N |

**EXAMPLE 52**

**erythro-N-[1,3-Dihydroxy-1-(6-octyl-2-pyridinyl)-2-propanyl]acetamide.**

A solution of erythro-N-[1,3-diacetyloxy-1-(6-octyl-2-pyridinyl-2-propanyl]-acetamide (5.2 g), and potassium carbonate (0.88 g) in methanol (75 ml) was stirred for 1 hr. The precipitate was collected, and the filtrate was evaporated. 7.5% Sodium bicarbonate solution and 1 N hydrochloric acid was added until a pH of 8.5 was obtained. The mixture was extracted with 3:1-chloroform:2-propanol. The extract was dried over anhydrous magnesium sulfate, filtered, and the filtrate was evaporated. Recrystallization of the residue from ethyl acetate gave 3.1 g (75.6%) of product, mp 85-86.5 °C.

| Analysis: | | | |
|---|---|---|---|
| Calculated for $C_{18}H_{30}N_2O_3$: | 67.05% | 9.38%H | 8.69%N |
| Found: | 66.98%C | 9.74%H | 8.65%N |

**EXAMPLE 53**

**erythro-2-Amino-1-(6-octyl-2-pyridinyl)1,3-propanediol**

A solution of erythro-N-[1,3-dihydroxy-1-(6-octyl-2-pyridinyl)-2-propanyl]-acetamide. (3.8 g), hydrazine hydrate (35 ml), and ethanol (25 ml) was heated under reflux, under nitrogen, for 26 hrs. The reaction mixture was cooled, water (50 ml) was added, and the mixture was extracted with chloroform. The extract was washed with saturated sodium chloride solution, dried over anhydrous magnesium sulfate, filtered, and the filtrate was evaporated. The residue was chromatographed on silica gel, eluting with 950:50:3-chloroform:methanol:2N ammonium hydroxide. The appropriate fractions were collected and evaporated. The residue was dissolved in ethyl acetate and the solution was washed with half-saturated sodium chloride solution dried over anhydrous magnesium sulfate, filtered, and the filtrate was evaporated. The residue was azeotroped with toluene to give 2.47 g (75%) of product mp 38-40 °C.

| Analysis: | | | |
|---|---|---|---|
| Calculated for $C_{16}H_{28}N_2O_2 \cdot 0.1H_2O$: | 68.10%C | 10.07%H | 9.93%N |
| Found | 67.88%C | 10.18%H | 9.74%N |

**EXAMPLE 54**

**threo-2-Amino-1-(6-octyl-2-pyridinyl)-1,3-propanediol**

A mixture of erythro-and threo-N-{1,3-dihydroxy-1-[6-(1-octynyl)-2-pyridinyl]-2-propanyl}acetamide (13.4 g), acetic anhydride (25.7 g), triethylamine (38.2 g), and 4-dimethylaminopyridine (0.5 g) in tetrahydrofuran (180 ml) was stirred at ambient temperature for 3 hrs. The reaction mixture was evaporated, the residue was warmed with methanol (80 ml) for 20 min, and the mixture was evaporated. 7.5% Sodium bicarbonate solution was added to a pH of 8.5, and the solution extracted with chloroform. The extracts

were dried, filtered, and the filtrate was evaporated. The residue was flash chromatographed. The appropriate fractions collected and evaporated to yield 2.4 g (14 %) of threo-N-{1,3-diacetyloxy-1-[6-(1-octynyl)-2-pyridinyl]-2-propanyl}acetamide.

threo-N-{1,3-Diacetyloxy-1-[6-(1-octynyl)-2-pyridinyl]-2-propanyl}acetamide (2.4 g) in ethanol (75 ml) containing 0.12 g of 5% palladium-on-carbon was shaken on a Parr hydrogenator at 40 psi of hydrogen. After 3 hrs, the reaction mixture was filtered, and the filtrate was evaporated. The residue was flash chromatographed. The appropriate fractions were collected and evaporated to give 2.2 g (92%) of threo-N-[1,3-diacetyloxy-1-(6-octyl-2-pyridinyl)-2-propanyl]acetamide.

A solution of threo-N-{1,3-diacetyloxy-1-[6-(1-octyl)-2-pyridinyl]-2-propanyl}acetamide (2.17 g) was stirred with potassium carbonate (70 mg), and methanol (25 ml) was stirred for 1 hr at ambient temperature, filtered, and the filtrate was evaporated. Water was added, the pH was adjusted to 8.5, and the mixture was extracted with 3:1 -chloroform:i-2-propanol. The extract was concentrated to give 1.6 g (94%) of threo-N-[1,3-diacetyloxy-1-(6-octyl-2-pyridinyl)-2-propanyl]acetamide, mp 71.5-74 °C.

A solution of threo-N-[1,3-diacetyloxy-1-(6-octyl-2-pyridinyl)-2-propanyl]acetamide (1.6 g), hydrazine hydrate (15 ml), and ethanol (15 ml) was heated winder reflux, under nitrogen for 23 hrs. The reaction mixture was cooled, water was added, and the mixture was extracted with chloroform. The extracts were washed with saturated sodium chloride solution, dried over anhydrous magnesium sulfate, filtered, and the filtrate was evaporated. The residue was chromatographed on silica gel, eluting with 960:40:3-chloroform:methanol:2N ammonium hydroxide. the appropriate fractions were collected and evaporated to give 0.78 g (56.5%, 7.0% overall) of product, mp 74-77 °C.

| Analysis: | | | |
|---|---|---|---|
| Calculated for $C_{16}H_{28}N_2O_2$: | 68.53%C | 10.06%H | 9.99%N |
| Found: | 68.48%C | 10.15%H | 9.96%N |

## EXAMPLE 55

### erythro-2-Amino-1-(6-hexyl-2-pyridinyl)-1,3-propanediol

A solution of erythro-2-[1-(6-hexyl-2-pyridinyl)-1,3-dihydroxy-2-propanyl]-acetamide (3.6 g), hydrazine hydrate (35 ml) and ethanol (25 ml) was heated under reflux, under nitrogen for 29 hrs. The reaction mixture was cooled, water (50 ml) was added, and the mixture was extracted with ethyl acetate. The extract was washed with saturated sodium chloride solution, dried over anhydrous magnesium sulfate, filtered and the filtrate was evaporated. The residue was chromatographed on silica gel, eluting with 950:50:3-chloroform:methanol:2N ammonium hydroxide. The appropriate fractions were collected and evaporated. The residue was dissolved in ethyl acetate (80 ml), and the solution was washed with half-saturated sodium chloride solution, dried, filtered, and the filtrate was evaporated to give 2.02 g, (66%) of product.

| Analysis: | | | |
|---|---|---|---|
| Calculated for $C_{14}H_{24}N_2O_2$: | 66.63%C | 9.59%H | 11.10%N |
| Found: | 65.91%C | 9.42%H | 10.82%N |

## EXAMPLE 56

### 6-(7-Phenyl-1-heptynyl)pyridine-2-carboxaldehyde

A solution of 6-bromopyridine-2-caboxaldehyde (30.0 g), 7-phenyl-1-heptyne (26.8 g), triethylamine (48.9 g), bis(triphenylphosphine)palladium(II)chloride (2.3 g, 2%), and copper(I)iodide (0.31 g, 1%) in dry tetrahydrofuran (100 ml) was heated under nitrogen at 55 °C for 70 hrs. The reaction mixture was cooled to ambient temperature, filtered, and the filtrate was washed with ethyl acetate. The filtrate was evaporated. The residue was dissolved in ethyl acetate, the solution washed with half-saturated sodium chloride solution, dried over anhydrous magnesium sulfate, and the filtrate was concentrated. The residue was flash chromatographed on silica gel, eluting with 0.5% to 2%-ethyl acetate:hexane. The appropriate fractions

were collected and concentrated to yield 29.5 g of the product.

| Analysis: | | | |
|---|---|---|---|
| Calculated for $C_{19}H_{19}NO$: | 82.28%C | 6.90%H | 5.05%N |
| Found: | 82.00%C | 6.94%H | 5.02%N |

## EXAMPLE 57

**Ethyl erythro-2-acetamido-3-hydroxy-3-[6-(7-phenyl-1-heptynyl)-2-pyridinyl]propionate**

A solution of 6-(7-phenyl-1-heptynyl)pyridine-2-carboxaldehyde (26.5 g), acetamidomalonic acid mono-oethyl ester (19.2 g), triethylamine (14.6 ml) in dry tetrahydrofuran (125 ml) was stirred at ambient temperature for 3 days, under nitrogen. The reaction mixture was evaporated, the residue was dissolved in ethyl acetate and the solution was washed with half-saturated sodium chloride solution, dried over anhydrous magnesium sulfate, filtered, and evaporated. The residue was chromatographed on a silica gel column, eluting with 1:1 -hexane:ethyl acetate to give 32.9 g (82.0%) of a mixture of erythro- and threo-isomers. The mixture was recrystallized from 1:1 -hexane:ethyl acetate to give 4.7 g (11.5%) of product, mp 79.0-81 °C.

| Analysis: | | | |
|---|---|---|---|
| Calculated for $C_{25}H_{30}N_2O_4$: | 71.07%C | 7.16%H | 6.63%N |
| Found: | 71.27%C | 6.89%H | 6.63%N |

## EXAMPLE 58

**erythro-N-{1,3-Dihydroxy-1-[6-(7-phenyl-1-heptynyl)-2-pyridinyl]-2-propanyl}-acetamide**

To a solution of ethyl erythro-2-acetamido-3-hydroxy-3-[6-(7-phenyl-1-heptynyl)-2-pyridinyl]propionate (23.4 g) in dry tetrahydrofuran (200 ml) was added 2.0 M lithium borohydride/tetrahydrofuran (22 ml) at 0 °C under nitrogen. The reaction mixture was stirred at ambient temperature overnight, chilled, and 1:1 -methanol:water (50 ml) was added followed by glacial acetic acid (2.5 ml) in 1:1 -methanol:water (30 ml) until a pH of 6.4 was obtained. The solution was stirred at ambient temperature for 1 hr, evaporated, and the residue was azeotroped with methanol. The residue was slurried with sodium bicarbonate (40 ml) (pH 8.5), saturated sodium chloride solution (40 ml) was added, and the mixture was extracted with 3:1 -chloroform:2-propanol. The extracts were concentrated. The residue was flash chromatographed on silica gel, eluting with 0.5% to 5% methanol:ethyl acetate. The appropriate fractions were collected and evaporated. Recrystallization of the residue from 1:1 -hexane:ethyl acetate gave 2.64 g (34%) of product, mp 83-85 °C.

| Analysis: | | | |
|---|---|---|---|
| Calculated for $C_{23}H_{28}N_2O_3$: | 72.61%C | 7.42%H | 7.36%N |
| Found: | 72.76%C | 7.66%H | 7.31%N |

## EXAMPLE 59

**Ethyl erythro-2-acetamido-3-hydroxy-3-[6-(5-phenyl-1-pentynyl)-2-pyridinyl]propionate**

A mixture of ethyl erythro-2-acetamido-3-(6-bromo-2-pyridinyl)-3-hydroxypropionate (23.2 g), 5-phenyl-1-pentyne (12.1 g), triethylamine (10.6 g), bis(triphenylphosphine)palladium chloride (0.98 g), and cuprous iodide (0.13 g) in dry tetrahydrofuran (100 ml) was heated at 55 °C for 1.5 hrs under nitrogen. Additional 5-phenyl-1-pentyne (6.1 g), triethylamine (7.1 g), bis(triphenylphosphine)palladium chloride (0.49 g), and cuprous iodide (0.07 g) were added, and the reaction mixture was heated overnight. The reaction mixture

was filtered and the filtrate was evaporated. The residue was redissolved in ethyl acetate, washed with half-saturated sodium chloride solution, and flash chromatographed on silica gel, eluting with 1:1 -hexane:ethyl acetate. The appropriate fractions were collected. The residue was rechromatographed, eluting with 1%-methanol:chloroform to give 14.2 g (51.6%) of product.

| Analysis: | | | |
|---|---|---|---|
| Calculated for $C_{23}H_{26}N_2O_4$: | 70.03%C | 6.64%H | 7.10%N |
| Found: | 69.30%C | 6.75%H | 6.94%N |

**EXAMPLE 60**

**erythro-N-{1,3-Dihydroxy-1-[6-(5-phenyl-1-pentynyl)-2-pyridinyl]-2-propanyl}-acetamide**

To a solution of ethyl erythro-2-acetamido-3-hydroxy-3-[6-(5-phenyl-1-pentynyl)-2-pyridinyl]propionate (12.3 g) in dry tetrahydrofuran (100 ml) was added 2.0 M lithium borohydride/tetrahydrofuran (15.5 ml) at 0°C, under nitrogen. The reaction mixture was stirred at ambient temperature overnight, chilled, and 1:1-methanol:water (45 ml) and glacial acetic acid (1.8 ml) were added until a pH of 6.5 was obtained. The mixture was stirred at ambient temperature for 80 min. The reaction mixture was evaporated, the residue was azeotroped with methanol and slurried with 7.5% sodium bicarbonate solution (25 ml) (pH 8.5). The mixture was extracted with 3:1-chloroform:2-propanol, filtered, and the filtrate was concentrated. The residue was flash chromatographed on silica gel, eluting with 0.5% to 1%-methanol:ethyl acetate. The appropriate fractions were collected and concentrated to give 6.0 g (54.6%) of product, mp 91-95°C.

| Analysis: | | | |
|---|---|---|---|
| Calculated for $C_{12}H_{24}N_2O_3$: | 71.57%C | 6.86%H | 7.95%N |
| Found: | 71.48%C | 6.75%H | 7.92%N |

**EXAMPLE 61**

**erythro-N-{1,3-Dihydroxy-1-[6-(5-phenylpentyl)-2-pyridinyl]-2-propanyl}acetamide hydrate**

erythro-N-{1,3-Dihydroxy-1-[6-(5-phenyl-1-pentynyl-2-pyridinyl]-2-propanyl} acetamide (5.45 g) in ethanol (150 ml) containing 5% palladium-on-carbon (0.20 g) was shaken on a Parr hydrogenator at 40 psi of hydrogen. After 1.5 hrs, the catalyst was collected. The filtrate was evaporated, and the residue was chromatographed on silica gel, eluting with 0.5%-1% methanol acetate to give 2.8 g (50%) of product.

| Analysis: | | | |
|---|---|---|---|
| Calculated for $C_{21}H_{28}N_2O_3 \cdot H_2O$: | 67.36%C | 8.07%H | 7.48%N |
| Found: | 67.95%C | 8.02%H | 7.53%N |

**EXAMPLE 62**

**erythro-2-Amino-1-[6-(5-phenylpentyl)-2-pyridinyl]-1,3-propanediol hemihydrate**

A solution of erythro-N-{1,3-dihydroxy-1-[6-(5-phenylpentyl)-2-pyridinyl]-2--propanyl}acetamide (3.5 g), hydrazine hydrate (32 ml), and ethanol (25 ml) was heated under reflux, under nitrogen, for 26 hrs. The reaction mixture was cooled, water (40 ml) was added, and the mixture was extracted with chloroform. The extracts were washed with saturated sodium chloride solution, dried over anhydrous magnesium sulfate, filtered, and the filtrate was evaporated. A mixture of the residue and 0.25 g from a similar experiment was chromatographed on silica gel, eluting with 970:30:2 to 950:50:3-chloroform:methanol:2N ammonium hydroxide. The appropriate fractions were collected and evaporated. The residue was dissolved in chloroform

(100 ml), and the solution was washed with saturated sodium chloride solution, dried over anhydrous magnesium sulfate, filtered, and the filtrate was evaporated. The residue was dried at 70°C under high vacuum to give 2.23 g (64%) of product.

| Analysis: | | | |
|---|---|---|---|
| Calculated for $C_{19}H_{26}N_2O_2 \cdot 0.5H_2O$: | 70.56%C | 8.41%H | 8.66%N |
| Found: | 70.77%C | 8.23%H | 8.62%N |

## EXAMPLE 63

**erythro-N-{1,3-Dihydroxy-1-[3-(1-undecynyl)phenyl]-2-propanyl}acetamide**

To ethyl erythro-2-acetamido-3-hydroxy-3-[3-(1-undecynyl)phenyl]propionate (7.3 g) in dry tetrahydrofuran (75 ml) was added 2.0 M lithium borohydride/tetrahydrofuran (11.4 ml) at 0°C, under nitrogen. The reaction mixture was chilled, stirred at ambient temperature overnight, and a mixture of glacial acetic acid (1.3 ml), methanol (25 ml) and water (25 ml) was added dropwise to a final pH of 6. The solution was concentrated and the residue was extracted with ethyl acetate. The extracts were dried over anhydrous magnesium sulfate, filtered, and the filtrate was concentrated. Trituration of the residue with 2:3-ether:hexane and recrystallization of the residue from ethyl acetate gave 1.9 g (29%) of product, mp 99-101°C.

| Analysis: | | | |
|---|---|---|---|
| Calculated for $C_{22}H_{33}NO_3$: | 73.50%C | 9.25%H | 3.90%N |
| Found: | 73.56%C | 9.05%H | 3.93%N |

## EXAMPLE 64

**erythro-N-{1-[3-(1-Dodecynyl)phenyl]-1,3 dihydroxy-2-propanyl}acetamide**

To ethyl erythro-2-acetamido-3-[3-(1-dodecynyl)phenyl]-3-hydroxypropionate (24.0 g) in tetrahydrofuran (220 ml) was added 2 N lithium borohydride/tetrahydrofuran (29 ml) at 2°C, under nitrogen. The reaction mixture was chilled, stirred at ambient temperature for 2.5 hrs. and 1:1-methanol:water (100 ml) and glacial acetic acid (3.3 ml) was added slowly until a pH of 6.5 was obtained. The solution was stirred at ambient temperature for 0.5 hr, evaporated, and the residue was azeotroped with methanol. The residue was slurried with 7.5% sodium bicarbonate solution (50 ml) (pH 8.5),and the mixture was extracted with 3:1-chloroform:propanol. The extract was concentrated. The residue was flash chromatographed on silica gel, eluting with ethyl acetate. The appropriate fractions were collected and evaporated. Recrystallization of the residue from ethyl acetate gave 9.13 g (42.4%) of product, mp 93-95°C.

| Analysis: | | | |
|---|---|---|---|
| Calculated for $C_{23}H_{35}NO_3$: | 73.96%C | 9.44%H | 3.75%N |
| Found: | 73.66%C | 9.14%H | 3.69%N |

## EXAMPLE 65

**erythro-N-[1-(3-Dodecylphenyl)-1,3-dihydroxy-2-propanyl]acetamide**

erythro-N-{1-(3-Dodecynyl)phenyl]-1,3-dihydroxy-2-propanyl}acetamide (4.4 g) in ethanol (100 ml) containing 5% palladium-on-carbon (0.02 g) was shaken on a Parr hydrogenator at 35 psi of hydrogen for 2.5 hrs. The catalyst was collected, the solvent evaporated, and the residue was recrystallized from ethyl acetate to give 4.1 g (90.6%) of product, mp 101-104°C.

| Analysis: | | | |
|---|---|---|---|
| Calculated for $C_{23}H_{39}NO_3$: | 73.17%C | 10.41%H | 3.71%N |
| Found: | 72.82%C | 10.36%H | 3.54%N |

**EXAMPLE 66**

**erythro-2-Amino-1-(3-dodecylphenyl)-1,3-propanediol**

erythro-N-[1-(3-Dodecylphenyl)-1,3-dihydroxy-2-propanyl]acetamide (2.9 g), hydrazine hydrate (25 ml), and ethanol (25 ml) were heated under reflux, under nitrogen for 22 hrs. The reaction mixture was washed with saturated sodium chloride solution, dried over anhydrous magnesium sulfate, filtered, and the filtrate was evaporated. The residue was chromatographed on silica gel, eluting with 950:50:3-chloroform:methanol:2N ammonium hydroxide. The appropriate fractions were collected and evaporated. The residue was dissolved in ethyl acetate (75 ml), and the solution was washed with half-saturated sodium chloride solution, dried over anhydrous magnesium sulfate, evaporated, and the residue was azeotroped with toluene to give 1.35 g (52%) of product, mp 36-40 ° C.

| Analysis: | | | |
|---|---|---|---|
| Calculated for $C_{21}H_{37}NO_2$: | 75.17%C | 11.12%H | 4.17%N |
| Found: | 75.14%C | 11.18%H | 4.12%N |

**EXAMPLE 67**

**erythro N-[1-[5-(1-Dodecyl)-2-thienyl]-1,3-diacetoxy-2-propyl]acetamide**

A solution of erythro N-[1-[5-(1-dodecyl)-2-thienyl]-1,3-dihydroxy-2-propyl]acetamide (12.0 g), acetic anhydride (19.2 g), triethylamine (28.4 g),and dimethylaminopyridine (0.4 g) in dry tetrahydrofuran (150 ml) was stirred at ambient temperature for 3 hrs. The reaction mixture was evaporated and the residue was dissolved in chloroform. The solution was washed with water, dried, filtered, and the filtrate was evaporated. The residue was dried to give 13.6 g (93.5%) of product, mp 105-107 ° C.

| Analysis: | | | |
|---|---|---|---|
| Calculated for $C_{25}H_{41}NO_5S$: | 64.21%C | 8.84%H | 2.99%N |
| Found: | 64.33%C | 8.92%H | 3.02%N |

**EXAMPLE 68**

**Ethyl erythro-2-acetamide-3-[5-(1-nonynyl)-2-thienyl]-3-hydroxypropionate**

A slurry of 5-nonynyl-2-thiophenecarboxaldehyde (40.0 g), acetamidomalonic acid monoethyl ester (32.3 g) and dry tetrahydrofuran (150 ml) was degassed and cooled to 0 ° C. Triethylamine (18.2 g) was added, the solution degassed, and the reaction mixture was stirred at room temperature under nitrogen for four days, Acetamidomalonic acid monoethyl ester (32.3 g) and triethylamine (18.2 g) were added, and the reaction mixture was stirred at room temperature under nitrogen for an additional four days. The reaction mixture was evaporated and the residue dried under vacuum. The residue was flash chromatographed (silica; 1:1-ethyl acetate:hexane). The appropriate fractions were collected and evaporated. The residue was crystallized from ether, then recrystallized twice from ethyl acetate to give 40.2 g (62%) of product, mp 104-106 ° c.

| Analysis: | | | |
|---|---|---|---|
| Calculated for $C_{20}H_{29}NO_4S$:<br>Found: | 63.30%C<br>63.30%C | 7.70%H<br>7.64%H | 3.69%N<br>3.71%N |

**EXAMPLE 69**

**erythro-N-[1-[5-(1-Nonynyl)-2-thienyl]-1,3-dihydroxy-2-propyl]acetamide**

A solution of ethyl erythro-2-acetamido-3-[5-(1-nonynyl)-2-thienyl]-3-hydroxypropionate (40.0 g) in dry tetrahydrofuran (150 ml) was stirred at 0°C under nitrogen as lithium borohydride (2.0 M in tetrahydrofuran, 68.5 ml) was added dropwise. The reaction mixture was stirred under a nitrogen atmosphere overnight, warming to room temperature. A solution of 50:50:8-methanol:water:acetic acid (108 ml) was added, with cooling in an ice-bath. The solution was neutralized with glacial acetic acid and evaporated. The residue was diluted with water and extracted with ethyl acetate. The combined organic extracts were dried over anhydrous sodium sulfate, filtered, and the filtrate was evaporated. The residue was flash chromatographed (silica; 2-4%-methanol-ethyl acetate). The appropriate fractions were collected and evaporated. The residue was recrystallized twice from ethyl acetate to give 29.9 g (84%) of product, mp 81-83°C.

| Analysis: | | | |
|---|---|---|---|
| Calculated for $C_{18}H_{27}NO_3S$:<br>Found: | 64.06%C<br>64.04%C | 8.06%H<br>8.17%H | 4.15%N<br>4.16%N |

**EXAMPLE 70**

**erythro-2-Amino-1-[5-(1-nonynyl)-2-thienyl]-1,3-propanediol**

A solution of erythro-N-[1-[5-(nonynyl)-2-thienyl]-1,3-dihydroxy-2-propyl]acetamide (16.4 g), 2N sodium hydroxide solution (150 ml), and 95% ethanol (75 ml) was stirred at 70°C overnight. The reaction mixture was cooled to room temperature and acidified with glacial acetic acid. The solution was diluted with water (200 ml), basified with sodium bicarbonate solution and chilled. The precipitate was collected, and the filtrate was extracted with 4:1-chloroform:2-propanol. The combined organic extracts were dried over anhydrous sodium sulfate, filtered, and the filtrate was evaporated. The residue was combined with the precipitate and flash chromatographed (silica, 90:9:1-dichloromethane:methanol:ammonium hydroxide) to give 11.8 g (82%) of product. A portion of product was crystallized from ether to give the analytical sample, mp 64-67°C.

| Analysis: | | | |
|---|---|---|---|
| Calculated for $C_{16}H_{25}NO_2S$:<br>Found: | 65.05%C<br>65.22%C | 8.53%H<br>8.56%H | 4.74%N<br>4.76%N |

**EXAMPLE 71**

**erythro-N-[1-[5-(1-Nonyl)-2-thienyl]-1,3-dihydroxy-2-propyl]acetamide**

A mixture of erythro-N-[1-[5(1-nonynyl)-2-thienyl]-1,3-dihydroxy-2-propyl]acetamide (8.00 g), 5% palladium-on-carbon (800 mg), and absolute ethanol (500 ml) was shaken under 50 psi of hydrogen overnight. The catalyst was filtered through a bed of celite, and the filtrate was washed with ethanol. The filtrate was evaporated, and the residue was recrystallized twice from ethyl acetate to give 7.0 g (86%) of product, mp 98-100°c.

| Analysis: | | | |
|---|---|---|---|
| Calculated for $C_{18}H_{31}NO_3S$: | 63.31%C | 9.15%H | 4.10%N |
| Found: | 63.03%C | 9.14%H | 4.01%N |

**EXAMPLE 72**

**5-(6-Phenyl-1-hexynyl)-2-thiophenecarboxaldehyde**

A solution of 6-phenyl-1-hexyne (30.6 g), 5-bromo-2-thiophenecarboxaldehyde (37.0 ), and triethylamine (58.7 g) in dry tetrahydrofuran (75 ml) was degassed and stirred at room temperature under a nitrogen atmosphere. Two mole percent of bis(triphenylphosphine)palladium(II)chloride (2.7 g) followed by one mole percent of copper(I)iodide (0.4 g) was added, The mixture was degassed and stirred at room temperature under nitrogen overnight. The precipitate was filtered and washed with ethyl acetate. The filtrate was evaporated, and the residue was distilled to give 47.0 g (91%) of product. A two gram-portion of product was flash chromatographed (silica, 1:1 -toluene:hexane). The appropriate fractions were collected and evaporated. The residue was distilled in a kugelruhr oven (170°C/0.07 mm mercury) to give the analytical sample, as an oil.

| Analysis: | | |
|---|---|---|
| Calculated for $C_{17}H_{16}OS$: | 76.08%C | 6.01%H |
| Found: | 75.54%C | 6.04%H |

**EXAMPLE 73**

**erythro-N-[1-[5-(6-Phenyl-1-hexynyl)-2-thienyl]-1,3-dihydroxy-2-propyl]acetamide**

A solution of ethyl erythro-2-acetamido-3-[5-(6-phenyl-1-hexynyl)-2-thienyl]-3-hydroxypropionate (41.6 g) in dry tetrahydrofuran (150 ml) was stirred at 0°C, under nitrogen, as a solution of lithium borohydride (2.0 M in tetrahydrofuran, 66 ml) was added dropwise. The reaction mixture was warmed to room temperature and stirred under a nitrogen atmosphere for four hrs. A solution of 50:50:8 methanol:water:acetic acid (108 ml) was added with cooling in an ice-bath. Glacial acetic acid was added, and the solution was evaporated. Water was added to the residue, and the solution was extracted with ethyl acetate. The combined organic extracts were dried over anhydrous sodium sulfate, filtered, and the filtrate was evaporated. The residue was flash chromatographed (silica; 90:9:1 dichloromethane:methanol:2N ammonium hydroxide). The appropriate fractions were collected and evaporated. Recrystallization of the residue from ethyl acetate gave 31.5 g (84%) of product, mp 131-133°C.

| Analysis: | | | |
|---|---|---|---|
| Calculated for $C_{21}H_{25}NO_3S$: | 67.90%C | 6.78%H | 3,77%N |
| Found: | 67.54%C | 6.88%H | 3.71%N |

**EXAMPLE 74**

**erythro-2-Amino-1-[5-(6-phenyl-1-hexynyl)-2-thienyl]-1,3-propanediol**

A solution of erythro-N-[1-[5-(6-phenyl-1-hexynyl)-2-thienyl]-1,3-dihydroxy-2-propyl]acetamide (10.0 g), 1N sodium hydroxide solution (125 ml) and 95% ethanol (75 ml) was stirred at 65°C overnight. The reaction mixture was evaporated, and the residue was neutralized with glacial acetic acid. The solution was diluted with water (200 ml) and extracted with 4:1-chloroform:2-propanol. The combined organic extracts were dried over anhydrous sodium sulfate, filtered, and the filtrate was evaporated. The residue was flash chromatographed (silica, 90:9:1-dichloromethane:methanol:2N ammonium hydroxide). The appropriate fractions were collected and evaporated to give 7.8 g (88%) of product. A portion was crystallized from ethyl acetate-hexane to give the analytical sample, mp 130-140 ° C (dec).

| Analysis: | | | |
|---|---|---|---|
| Calculated for $C_{19}H_{23}NO_2S$: | 69.27%C | 7.04%H | 4.25%N |
| Found: | 69.29%C | 7.14%H | 4.26%N |

**EXAMPLE 75**

**4-(1-Dodecynyl)-2-thiophenecarboxaldehyde**

A solution of 1-dodecyne (23.9 g), 5-bromo-2-thiophenecarboxaldhyde (25.0 g) and triethylamine (39.7 g) in dry tetrahydrofuran (75 ml) was degassed and stirred at room temperature under a nitrogen atmosphere. Two mole percent of bis(triphenylphosphine)palladium(II)chloride (1.8 g) followed by one mole percent of copper(I)iodide (0.25 g) was added. The mixture was degassed and stirred at room temperature under nitrogen for three days. The precipitate was filtered, and the filter cake was washed with ethyl acetate. The filtrate was evaporated, and the residue was flash chromatographed (silica, 7:3-Hexane:dichloromethane). The appropriate fractions were collected and evaporated. The residue was dried at 50 ° C under vacuum for three hrs to give 35.5 g (98%) of product.

| Analysis: | | |
|---|---|---|
| Calculate for $C_{17}H_{24}OS$: | 73.86%C | 8.75%H |
| Found: | 73.79%C | 9.08%H |

**EXAMPLE 76**

**Ethyl erythro-2-acetamide-3-[4-(1-dodecynyl)-2-thienyl]-3-hydroxypropionate**

A slurry of 4-(1-dodecynyl)-2-thiophenecarboxaldehyde (21.4 g), acetamidomalonic acid monoethyl ester (14.6 g), and dry tetrahydrofuran (100 ml) was degassed and cooled to 0 ° C. Triethylamine (8.23 g) was added, the solution degassed, and the reaction stirred at room temperature under nitrogen for five days. Additional acetamidomalonic acid monoethyl ester (14.6 g) and triethylantine (8.23 g) were added, and the reaction was stirred at room temperature under nitrogen for five days. The reaction mixture was evaporated, the residue dried under vacuum, and flash chromatographed (silica, 3:2-hexane:ethyl acetate). The appropriate fractions were collected and evaporated. The residue was recrystallized from ethyl acetate-hexane to 23.3 g (71%) of product, mp 79-81 ° C.

| Analysis: | | | |
|---|---|---|---|
| Calculated for $C_{23}H_{35}NO_4S$: | 65.53%C | 8.37%H | 3.32%N |
| Found: | 65.63%C | 7.96%H | 3.34%N |

**EXAMPLE 77**

**erythro-2-Amino-1-[4-(1-dodecyl)-2-thienyl]-1,3-propanediol**

A solution of erythro-N-[1-[4-(dodecyl)-2-thienyl]-1,3-dihydroxy-2-propyl]a-cetamide (6.35 g), 2N sodium hydroxide solution (100 ml) and 95% ethanol (50 ml) was stirred at 65 ° C overnight. The reaction mixture was cooled to room temperature and neutralized with glacial acetic acid, The solution was diluted with water (200 ml) and chilled. The precipitate was collected, and the filtrate was extracted with chloroform. The combined organic extracts were dried over anhydrous sodium sulfate, filtered, and the filtrate was evaporated. The residue was combined with the precipitate and flash chromatographed (silica, 90:9:1-

dichloromethane:methanol:2N ammonium hydroxide). The appropriate fractions were collected and evaporated. The residue was recrystallized from ethyl acetate to give 4.1 g (73%) of product, mp 99-100 ° C.

| Analysis: | | | |
|---|---|---|---|
| Calculated for $C_{19}H_{35}NO_2S$: | 66.81%C | 10.33%H | 4.10%N |
| Found: | 66.70%C | 10.40%H | 4.11%N |

## EXAMPLE 78

### Ethyl erythro-2-acetamido-3-[5-(6-phenyl-1-hexynyl)-2-thienyl]-3-hydroxypropionate

A slurry of 6-phenyl-1-hexynyl-2-thiophenecarboxaldehyde (45.0 g), acetamidomalonic acid monoethyl ester (31.7 g), and dry tetrahydrofuran (150 ml) was degassed and cooled to 0 ° C. Triethylamine (17.9 g) was added, the solution degassed, and the reaction mixture stirred at room temperature under nitrogen for two days. Additional acetamidomalonic acid monoethyl ester (31.7 g) and triethylamine (17.9 g) were added, and the reaction mixture was stirred at room temperature under nitrogen for an additional two days. The mixture was evaporated, the residue dried under vacuum, and flash chromatographed (silica, 1:1-ethyl acetate:hexane). The appropriate fractions were collected and evaporated. The residue was recrystallized from ethyl acetate-hexane to give 48.1 g (69%) of product, mp 90-92 ° C.

| Analysis: | | | |
|---|---|---|---|
| Calculated for $C_{23}H_{27}NO_4S$: | 66.80%C | 6.58%H | 3.39%N |
| Found: | 66.48%C | 6.59%H | 3.33%N |

## EXAMPLE 79

### erythro-2-Amino-1-[4-(1-dodecynyl)-2-thienyl]-1,3-propanediol] acetate

A solution of erythro-N-[1-[4-(dodecynyl)-2-thienyl]-1,3-dihydroxy-2-propyl]-acetamide (4.30 g), 2N sodium hydroxide solution (100 ml), and 95% ethanol (50 ml) was stirred at 65 ° C overnight. The reaction mixture was cooled to room temperature and acidified with glacial acetic acid. The solution was diluted with water (200 ml) and chilled in the refrigerator. The precipitate was collected and the filtrate extracted with chloroform. The combined organic extracts were dried over anhydrous sodium sulfate, filtered, and the filtrate was evaporated. The residue was combined with the precipitate and previously prepared material, and recrystallized twice from ethyl acetate to give 4.0 g (55%) of product, mp 120-122 ° C.

| Analysis: | | | |
|---|---|---|---|
| Calculated for $C_{21}H_{35}NO_2S$: | 63.44%C | 8.87%H | 3.52%N |
| Found: | 63.32%C | 8.71%H | 3.50%N |

## EXAMPLE 80

### erythro-N-[1-[4-(1-Dodecyl)-2-thienyl]1,3-dihydroxy-2-propyl]acetamide

A mixture of erythro-N-[1-[4-(1-dodecynyl)-2-thienyl]-1,3-dihydroxy-2-propyl]acetamide (8.00 g), 5% palladium-on-carbon (800 mg), and absolute ethanol (500 ml) was shaken under 50 psi of hydrogen overnight. The reaction mixture was filtered through a bed of celite, and the filter cake was washed with ethanol. The solvent was evaporated and the residue recrystallized twice from ethyl acetate to give 7.0 g (87%) of product, mp 92-94 ° C.

| Analysis: | | | |
|---|---|---|---|
| Calculated for $C_{21}H_{37}NO_3S$: | 65.75%C | 9.72%H | 3.65%N |
| Found: | 65.72%C | 9.82%H | 3.63%N |

**EXAMPLE 81**

**erythro-2-Amino-1-[3-(1-dodecyl)-5-isoxazolyl]-1,3-propanediol**

A solution of erythro-N-[1-[3-(1-dodecyl)-5-isoxazolyl]-1,3-dihydroxy-2-propyl]acetamide (4.0 g), degassed 2N sodium hydroxide solution (100 ml), and 95% ethanol (50 ml) was stirred at 60 °C for 3 hrs. The reaction mixture was concentrated, the residue diluted with sodium bicarbonate solution, and extracted with 4:1 -chloroform-2-propanol. The combined organic extracts were dried over anhydrous magnesium sulfate, filtered, and the filtrate was evaporated. The residue was recrystallized from ethylacetate-hexane. The precipitate was flash chromatographed (silica, 90:1 -dichloromethane:methanol). The appropriate fractions were collected and evaporated. The residue was recrystallized from ethyl acetate-hexane to give 2.3 g (65%) of product, mp 88-90 °C.

| Analysis: | | | |
|---|---|---|---|
| Calculated for $C_{18}H_{34}N_2O_3$: | 66.22%C | 10.50%H | 8.58%N |
| Found: | 65.55%C | 10.44%H | 8.51%N |

**EXAMPLE 82**

**3-(1-Decyl)-5-isoxazolemethanol**

To a solution of 1-nitroundecane (38.0 g) and O-trimethylsilylpropynol (24.1 g) in dry benzene (300 ml) was added dropwise a solution of freshly distilled phenylisocyanate (44.9 g) and triethylamine (22.4 g) in dry benzene (50 ml) at 40 °C, with stirring. The reaction mixture was heated at 60 °C for 3 hrs, cooled, and filtered. To the filtrate was added 1.0 M tetrabutylammonium fluoride (40 ml). After 30 mins, the solution was evaporated, and the residue was flash chromatographed (silica gel, 1% methanol-dichloromethane) to give 15.5 g (34%) of product, mp 55-56 °C.

| Analysis: | | | |
|---|---|---|---|
| Calculated for $C_{14}H_{25}NO_2$: | 70.25%C | 10.53%H | 5.85%N |
| Found: | 70.44%C | 10.42%H | 5.92%N |

**EXAMPLE 83**

**3-(1-Decyl)-5-isoxazolecarboxaldehyde**

To a solution of oxalyl chloride (31.7 ml) in dry dichloromethane (100 ml) cooled to -60 °C, was added a solution of dimethylsulfoxide (9.8 ml) in dichloromethane (30 ml) followed by a slurry of 3-(1-decyl)-5-isoxazolemethanol (13.8 g) in dry dichloromethane (100 ml). The reaction mixture was stirred at -60 °C for 0.5 hr, quenched with triethylamine (40 ml), and allowed to warm to ambient temperature. The solution was poured into water (300 ml) and extracted with dichloromethane. The organic phases were washed with dilute citric acid, dried, and evaporated. The residue was flash chromatographed (silica, 50:1-dichloromethane:methanol). The appropriate fraction were collected and evaporated. The residue was recrystallized from ether-hexane to give 11.2 g (82%) of product, mp 46-48 °C.

| Analysis: | | | |
|---|---|---|---|
| Calculated for $C_{14}H_{23}NO_2$:<br>Found: | 70.85%C<br>71.16%C | 9.77%H<br>9.93%H | 5.90%N<br>5.94%N |

**EXAMPLE 84**

**Ethyl erythro-2-acetamido-3-(3-(1-decyl)-5-isoxazolyl)-3-hydroxypropionate**

To a mixture of 3-(decyl)-5-isoxazolecarboxaldehyde (10 g), and acetamidomalonic acid monoethylester (7.9 g) in dry tetrahydrofuran (100 ml), cooled to 0°C, was added triethylamine (4.5 g), under nitrogen. The solution was allowed to warm to room temperature and stirred for 16 hrs. The solution was evaporated, and the residue was recrystallized twice from ethyl acetate-hexane to give 10.3 g (64%) of product, mp 83-85°C.

| Analysis: | | | |
|---|---|---|---|
| Calculated for $C_{20}H_{34}N_2O_5$:<br>Found: | 62.80%C<br>62.87%C | 8.96%H<br>9.30%H | 7.32%N<br>7.32%N |

**EXAMPLE 85**

**erythro-2-Amino-1-[3-(1-decyl)-5-isoxazolyl]-1,3-propanediol**

A solution of erythro-N-[1-[5-(decyl)-3-isoxazolyl]-1,3-dihydroxy-2-propyl]acetamide (9.30 g), degassed 2N sodium hydroxide solution (200 ml), and 95% ethanol (100 ml) was stirred at 80°C for 6 hrs. The reaction mixture was concentrated, the residue diluted with sodium bicarbonate solution, and extracted with 4:1-chloroform:2-propanol. The combined organic extracts were dried over anhydrous magnesium sulfate, filtered, and the filtrate was evaporated. The residue was flash chromatographed (silica; 9:1 dichloromethane:methanol). The appropriate fractions were collected and evaporated. The residue was recrystallized from ethyl acetate-hexane to give 6.1 g (75%) of product, mp 88-90°C.

| Analysis: | | | |
|---|---|---|---|
| Calculated for $C_{16}H_{30}N_2O_3$:<br>Found: | 64.40%C<br>64.37%C | 10.13%H<br>10.25%H | 9.39%N<br>9.42%N |

**EXAMPLE 86**

**5-(1-Undecynyl)-2-thiophenecarboxaldehyde**

A solution of 1-undecyne (62.7 g), 5-bromo-2-thiophenecarboxaldehyde (75.0 g), and triethylamine (119.2 g) in dry tetrahydrofuran (300 ml) was degassed and stirred at 0-5°C, under a nitrogen atmosphere. Two mole percent of bis(triphenylphosphine)palladium(II)chloride (5.51 g) followed by one mole percent of copper(I)iodide (0.75 g) was added and the mixture was degassed and stirred at room temperature overnight, under nitrogen. The precipitate was filtered and the filter cake was washed with ethyl acetate. The filtrate was evaporated and the residue purified by flash chromatography (silica; 5% ethyl acetate-hexane). The appropriate fractions were colllected and evaporated to give 88.1 g (85%) of product as an oil. The oil was dried at 50°C under high vacuum (approx. 0.01 mm mercury) for four hrs to provide the analytical sample.

| Analysis: | | |
|---|---|---|
| Calculated for $C_{16}H_{22}OS$: | 73.23%C | 8.45%H |
| Found: | 73.15%C | 8.50%H |

**EXAMPLE 87**

**Ethyl erythro-2-acetamido-3-[5-(1-undecynyl)-2-thienyl]-3-hydroxypropionate**

A slurry of 5-undecynyl-2-thiophenecarboxaldehyde (86. 1 g), acetamidomalonic acid monoethyl ester (62.0 g), and dry tetrahydrofuran (300 ml) was degassed and cooled to 0°C. Triethylamine (34.8 g) was added, the solution were degassed, and the reaction mixture was stirred at room temperature for four days, under nitrogen. Acetamidomalonic acid monoethyl ester (62.0 g) and triethylamine (34.8 g) were added, and the reaction mixture was stirred at room temperature for an additional two days, under nitrogen. The reaction mixture was evaporated, and the residue was dried under vacuum. The residue was purified by flash chromatography (silica; 1:1-ethyl acetate-hexane). The appropriate fractions were collected and evaporated, and the residue was recrystallized from ethyl acetate to give 86.5 g (65%) of product, mp 80-82°C.

| Analysis: | | | |
|---|---|---|---|
| Calculated for $C_{22}H_{33}NO_4S$: | 64.83%C | 8.16%H | 3.44%N |
| Found: | 64.65%C | 8.07%H | 3.45%N |

**EXAMPLE 88**

**erythro-N-[1-[5-(1-Undecynyl)-2-thienyl]-1,3-dihydroxy-2-propyl]acetamide**

A solution of ethyl erythro-2-acetamido-3-[5-(1-undecynyl)-2-thienyl]-3-hydroxypropionate (86.2 g) in dry tetrahydrofuran (200 ml) was stirred at 0°C, under nitrogen. Lithium borohydride (137.5 ml, 2.0 M in tetrahydrofuran) was added dropwise. The reaction mixture was allowed to warm to room temperature, and was stirred for four hrs, under nitrogen. The reaction mixture was cooled in an ice-bath. Methanol:water:acetic acid solution (50 ml:50 ml:10 ml) was added. The solution was neutralized with glacial acetic acid. The mixture was evaporated, and the residue was diluted with water (400 ml) and extracted with ethyl acetate. The combined organic extracts were dried over anhydrous sodium sulfate, filtered, and the filtrate was evaporated. The residue was purified by flash chromatography (silica; 7% methanol:dichloromethane). The appropriate fractions were collected and evaporated. Recrystallization of the residue from ethyl acetate gave 61.2 g (79%) of product, mp 88-90°C.

| Analysis: | | | |
|---|---|---|---|
| Calculated for $C_{20}H_{31}NO_3S$: | 65.72%C | 8.55%H | 3.83%N |
| Found: | 66.00%C | 8.71%H | 3.82%N |

**EXAMPLE 89**

**(4-trans)-N-[2,2-Dimethyl-4-[5-(1-undecynyl)-2-thienyl]-1,3-dioxan-5-yl]acetamide**

To a solution of erythro-N-[1-[5-(1-undecynyl)-2-thienyl]-1,3-dihydroxy-2-propyl]acetamide (25 g) in dry acetone (400 ml) was added 2,2-dimethoxypropane (45.3 g) and a catalytic amount of para-toluenesulfonic acid. The solution was stirred at room temperature for 7 hrs and then evaporated. The residue was purified by column chromatography (silica gel, 4:1 chloroform:ether). The appropriate fractions were collected and concentrated. Recrystallization from ether-hexane gave 20.9 g (73.8%) of product, mp 88-89°C.

| Analysis: | | | |
|---|---|---|---|
| Calculated for $C_{23}H_{35}NO_3S$: | 68.11%C | 8.70%H | 3.45%N |
| Found: | 68.26%C | 8.83%H | 3.39%N |

**EXAMPLE 90**

**erythro-N-[1-[5-(1-Nonynyl)-2-thienyl]-1,3-dihydroxy-2-propyl]-1,1-dimethylethylcarbamate**

To a slurry of erythro-2-amino-1-[5-(1-nonynyl)-2-thienyl]-1,3-propanediol (2.8 g) in saturated sodium bicarbonate solution (50 ml) was added a solution of di-tert-butyl dicarbonate (2.24 g) in chloroform (50 ml), over two mins. The mixture was stirred at 60 °C for 45 mins, the layers were separated, and the organic layer was dried and evaporated. The residue was purified by passage through a short pad of silica gel, and the filtrate was evaporated. The residue was crystallized from ether-hexane to give 2.9 g (77.6%) of product, mp 73-75 °C.

| Analysis: | | | |
|---|---|---|---|
| Calculated for $C_{21}H_{33}NO_4S$: | 63.77%C | 8.41%H | 3.54%N |
| Found: | 63.65%C | 8.31%H | 3.50%N |

**EXAMPLE 91**

**erythro-2-Amino-1-[5-(1-nonyl)-2-thienyl]-1,3-propanediol acetate**

A solution of erythro-N-[1-[5-(1-nonyl)-2-thienyl]-1,3-dihydroxy-2-propyl]acetamide (7.30 g), 2N sodium hydroxide solution (100 ml) and 95% ethanol (75 ml) was stirred at 65 °C overnight. The reaction mixture was concentrated, and the aqueous residue was neutralized with glacial acetic acid. The solution was diluted with water (200 ml) and extracted with 4:1-chloroform:isopropanol. The combined organic extracts were dried over anhydrous sodium sulfate, filtered, and the filtrate was evaporated. The residue was purified by flash chromatography (silica; 90:9:1-dichloromethane:methanol:ammonium hydroxide). The appropriate fractions were collected and concentrated. The residue was dissolved in ethanol, and excess glacial acetic acid was added. Ether and hexane were added. The precipitate was recrystallized from ethyl acetate to give 4.1 g (53%) of product, mp 109-111 °C.

| Analysis: | | | |
|---|---|---|---|
| Calculated for $C_{18}H_{33}NO_4S$: | 60.13%C | 9.25%H | 3.90%N |
| Found: | 60.02%C | 8.99%H | 3.90%N |

**EXAMPLE 92**

**erythro-2-Dimethylamino-1-[5-(6-phenyl-1-hexynyl)-2-thienyl]-1,3-propanediol**

To a mixture of erythro-2-amino-1-[5-(6-phenyl-1-hexynyl)-2-thienyl]-1,3-propanediol (4.0 g), 37% aqueous formaldehyde (9.1 ml), and acetonitrile (50 ml) was added sodium cyanoborohydride (2.29 g) in three portions, with stirring at room temperature. After stirring for 30 mins, glacial acetic acid (1 ml) was added dropwise, and stirring was continued for 30 mins. The mixture was neutralized with acetic acid and evaporated. 1N Sodium hydroxide (200 ml) was added to the residue, and the mixture was extracted with chloroform. The extracts were dried over anhydrous sodium sulfate, filtered, and the filtrate was evaporated. The residue was flash chromatographed on silica gel, using 10% methanol:dichloromethane as eluent. The appropriate fractions were collected and evaporated to give 1.3 g (30%) of product, as an oil.

## EXAMPLE 93

### 4-(1-Dodecenyl)-2-thiophenecarboxaldehyde

A solution of 1-dodecyne (50.0 g), tributyltinhydride (109.4 g), and azobisisobutyronitrile (100 mg) was stirred at 95°C for 3 hrs. The reaction mixture was cooled to 50°C and evaporated. The resiude was filtered through a column of silica, using hexane as the eluent. The appropriate fractions were collected and evaporated. The residue was dried under vacuum to give 131.7 g (96.0%) of tri-n-butyl-1-dodecenylstannane.

To a solution of 4-bromo-2-thiophenecarboxaldehyde (35.0), tetrakis(triphenylphosphine)palladium(0) (4.23 g), 2,6-di-*t*-butyl-4-methylphenol (a few milligrams) and dry toluene (150 ml) was added tri-*n*-butyl-1-dodecenylstannane (92.0 g), dropwise at room temperature, under nitrogen. The solution was heated under reflux for four hrs, with stirring. After cooling to room temperature, the solution was filtered through a bed of celite, and the filter cake was washed with ether. The filtrate was evaporated and the residue purified by flash chromatography (silica; 5% ethyl acetate-hexane). The appropriate fractions were collected and concentrated. The residue was distilled in a kugelrohr oven (170°C/0.3 mm mercury) to give 46.7 g (92%) of product, as an oil (ca. 4:1 -trans:cis).

| Analysis: | | |
|---|---|---|
| Calculated for $C_{17}H_{26}OS$: | 73.33%C | 9.41%H |
| Found: | 73.64%C | 9.62%H |

## EXAMPLE 94

### Ethyl erythro-2-acetamido-3-[5-(1-dodecenyl)-2-thienyl]-3-hydroxypropionate

A slurry of 5-dodecenyl-2-thiophenecarboxaldehyde (46.0 g, ca. 4:1-trans:cis), acetamidomalonic acid monoethyl ester (31.3 g), and dry tetrahydrofuran (300 ml) was degassed and cooled to 0°C. Triethylamine (17.6 g) was added, the solution was degassed, and the reaction mixture was stirred at room temperature for three days, under nitrogen. Acetamidomalonic acid monoethyl ester (46.0 g) and triethylamine (17.6 g) were added, and the reaction mixture was stirred at room temperature for an additional three days, under nitrogen. The reaction mixture was concentrated, and the residue was dried under vacuum. The residue was was purified by flash chromatography (silica; 2:3 ethyl acetate-hexane). The appropriate fractions were collected and concentrated. The residue was recrystallized from diethyl ether to give 36.7 g (53%) of product (ca. 4:1-trans:cis), mp 66-68°C.

| Analysis: | | | |
|---|---|---|---|
| Calculated for $C_{23}H_{37}NO_4S$: | 65.21%C | 8.80%H | 3.31%N |
| Found: | 65.36%C | 8.71%H | 3.32%N |

## EXAMPLE 95

### erythro-N-{1.3-Diacetyloxy-1-[6-(5-phenyl-1-pentynyl)-2-pyridinyl]-2-propanyl}acetamide

A diastereomeric mixture of N-{1,3-dihydroxy-1-[6-(5-phenyl-1-pentynyl)-2-pyridinyl]-2-propanyl}acetamide (18.4 g, 6:1-erythro:threo), acetic anhydride (31.9 g), triethylamine (47.5 g), and 4-dimethylaminopyridine (0.64 g) in tetrahydrofuran (200 ml) was stirred at room temperature overnight. The reaction mixture was concentrated, methanol was added, and the mixture was warmed at 50°C for 20 mins. The mixture was evaporated, and the residue azeotroped with toluene. A 7.5% sodium bicarbonate solution was added until pH 8.5, and the mixture was extracted into chloroform. The extract was dried over anhydrous magnesium sulfate, filtered, and the filtrate was concentrated. The residue and one (8.0 g) from a similar reaction (18.8 mmol) were combined and purified by flash chromatography, eluting with 1:1-yield hexane:ethyl acetate. The appropriate fractions were collected and concentrated to yield 5.06 g (16.3%

yield) of product, mp 104-106 °C.

| Analysis: | | | |
|---|---|---|---|
| Calculated for $C_{25}H_{28}N_2O_5$: | 68.79%C | 6.47%H | 6.42%N |
| Found: | 68.69%C | 6.47%H | 6.36%N |

## EXAMPLE 96

**erythro-2-Amino-1-[6-(5-phenyl-1-pentynyl)-2-pyridinyl]-1,3-propanediol hemihydrate**

erythro-N-{1,3-Dihydroxy-1-[6-(5-phenyl-1-pentynyl)-2-pyridinyl]-2-propanyl}acetamide (4.4 g), 2N sodium hydroxide solution (100 ml) and ethanol (50 ml) were heated at 60 °C for 13 hrs, under nitrogen. The reaction mixture was cooled, extracted with chloroform, and the extract was washed with half-saturated sodium chloride solution, dried over anhydrous magnesium sulfate, filtered, and the filtrate was evaporated. The residue was chromatographed twice on silica gel eluting with 950:50:3 to 920:80:5 chloroform:methanol:2N ammonium hydroxide. The appropriate fractions were collected and concentrated. The residue was dissolved in ethyl acetate, and the solution was washed with saturated sodium chloride solution, dried over anhydrous magnesium sulfate, filtered, and the filtrate was concentrated to give 1.13 g (36.5%) of product, as an oil (dried 60 °C under vacuum).

| Analysis: | | | |
|---|---|---|---|
| Calculated for $C_{19}H_{22}N_2O_2 0.5H_2O$: | 71.44%C | 7.26%H | 8.77%N |
| Found: | 71.87%C | 7.11%H | 8.67%N |

## EXAMPLE 97

**erythro-N-[1,3-Dihydroxy-1-(6-undecyl-2-pyridinyl)-2-propanyl]acetamide**

A mixture of erythro-N-{1,3-Dihydroxy-1-[6-(1-undecynyl)-2-pyridinyl]-2-propanyl}acetamide (5.4 g) in ethanol (125 ml), and 5% palladium-on-carbon (0.20 g) was hydrogenated on a Parr hydrogenator at 40 psi of hydrogen. After 2.5 hrs, the catalyst was filtered, and the filtrate was concentrated. Recrystallization of the residue from ethyl acetate gave 4.9 g (88.7%) of product, mp 97-98.5 °C.

| Analysis: | | | |
|---|---|---|---|
| Calculated for $C_{21}H_{36}N_2O_3$: | 69.19%C | 9.95%H | 7.68%N |
| Found : | 69.19%C | 9.91%H | 7.64%N |

## EXAMPLE 98

**erythro-2-Amino-1-(6-undecyl-2-pyridinyl)-1,3-propanediol**

erythro-N-[1,3-Dihydroxy-1-(6-undecyl-2-pyridinyl)-2-propanyl]acetamide (3.72 g), hydrazine hydrate (32 ml), and ethanol (25 ml) were refluxed for 24 hrs, under nitrogen. The reaction mixture was cooled, water was added, and the mixture was extracted with chloroform. The extract was washed with saturated sodium chloride solution, dried over anhydrous magnesium sulfate, filtered, and the filtrate was evaporated. The residue was chromatographed on silica gel, eluting with 950:50:3 chloroform:methanol:2N ammonium hydroxide. The appropriate fractions were collected and evaporated. The residue was dissolved in chloroform, the solution was washed with saturated sodium chloride solution, dried over anhydrous magnesium sulfate, filtered, and the filtrate was concentrated to give 1.45 g (44%) of product, mp 56-59 °C (dried at 55 °C under vacuum).

| Analysis: | | | |
|---|---|---|---|
| Calculated for $C_{19}H_{34}N_2O_2$: | 70.76%C | 10.63%H | 8.69%N |
| Found: | 70.20%C | 10.71%H | 8.48%N |

EXAMPLE 99

**erythro-2-Amino-1-[6-(1-undecynyl)-2-pyridinyl]-1,3-propanediol hemihydrate**

erythro-N-{1,3-Dihydroxy-1-[6-(1-undecynyl)-2-pyridinyl]-2-propanyl}acetamide (5.4 g), 2N sodium hydroxide (53 ml), and ethanol (35 ml) were heated at 60 °C for 19 hrs, under nitrogen. The reaction mixture was cooled, extracted with chloroform, and the extract was washed with half-saturated sodium chloride solution, dried over anhydrous magnesium sulfate, filtered, and the filtrate was concentrated. The residue was chromatographed twice on silica gel, eluting with 950:50:3 to 930:70:5 chloroform:methanol:2N ammonium hydroxide. The appropriate fractions were collected and concentrated to give 3.04 g (63.6% yield) of product, as an oil dried at 60 °C under vacuum.

| Analysis: | | | |
|---|---|---|---|
| Calculated for $C_{19}H_{30}N_2O_2 0.5H_2O$: | 69.69%C | 9.54%H | 8.55%N |
| Found: | 69.93%C | 9.37%H | 8.46%N |

EXAMPLE 100

**erythro-N-{1,3-Diacetyloxy-1-[6-(1-undecynyl)-2-pyridinyl]-2-propanyl}acetamide**

A diastereomeric mixture of N-{1,3-dihydroxy-1-[6-(1-undecynyl)-2-pyridinyl]-2-propanyl}acetamide (12.0 g, 4:1 erythro:threo), acetic anhydride (20.4 g), triethylamine (30.4 g), and 4-dimethylaminopyridine (0.41 g) in tetrahydrofuran (125 ml) was stirred at room temperature overnight. The reaction mixture was evaporated, methanol was added, and the solution was warmed at 50 °C for 20 min and evaporated. A 7.5% sodium bicarbonate solution was added until pH 8.5, and the mixture was extracted into chloroform. The extract was dried over anhydrous magnesium sulfate, filtered, and the filtrate was concentrated. The residue was purified by flash chromatography, eluting with 1:1-hexane:ethyl acetate. The appropriate fractions were combined and concentrated to yield 3.5 g (24%) of product, mp 69-71 °C.

| Analysis: | | | |
|---|---|---|---|
| Calculated for $C_{25}H_{36}N_2O_5$: | 67.54%C | 8.16%H | 6.30%N |
| Found: | 67.65%C | 8.23%H | 6.18%N |

EXAMPLE 101

**3-(1-Undecynyl)benzaldehyde**

A mixture of 3-bromobenzaldehyde (51.8 g), 1-undecyne (48.6 g), bis(triphenylphosphine)palladium(II)-chloride (3.37 g), copper iodide (457 mg), and triethylamine (196 ml) in dry tetrahydrofuran (300 ml) was stirred for 4 hrs at 55 °C. The reaction mixture was filtered, the filtrate was diluted with ethyl acetate, and the solution was washed with water and brine. The organic phase was dried over anhydrous magnesium sulfate, filtered, and the filtrate was concentrated in vacuo. The residue was chromatographed on 300 g of silica gel (1:4-ethyl acetate:hexane). The appropriate fractions were collected and evaporated. Distillation of a 40-g sample of the residue (81.3 g) gave 9.5 g (13%) of the product, bp 172-174 °C (0.5 mm mercury).

| Analysis: | | |
|---|---|---|
| Calculated for $C_{18}H_{24}O$:<br>Found : | 84.32%C<br>84.76%C | 9.44%H<br>9.57%H |

**EXAMPLE 102**

**erythro-2-Amino-1-(3-undecynylphenyl)-1,3-propanediol acetate**

A solution of erythro-N-{1,3-diacetyloxy-1-[3-(undecynylphenyl-2-propanyl} acetamide (2.76 g) and aqueous 2 N sodium hydroxide solution (62 ml) in ethanol (20 ml) was warmed to 60°C for 16 hr. The reaction mixture was allowed to cool to room temperature and extracted with chloroform. The extract was dried over anhydrous magnesium sulfate, filtered, and the filtrate was concentrated in vacuo. The residue was chromatographed on silica gel (eluted with 900:100:5 chloroform:methanol:ammonium hydroxide). The residue was rechromatographed (eluted with 9:1-chloroform:methanol) to afford 1.35 g (71%) of the free base.

To a solution of free base (1.05 g) in dichloromethane (10 ml) was added glacial acetic acid (2 ml) followed by hexane (50 ml). The mixture was concentrated in vacuo and the solid was recrystallized from ethyl acetate to afford 860 mg (69%) of product, mp 110-112°C.

| Analysis: | | | |
|---|---|---|---|
| Calculated for $C_{22}H_{35}NO_4$:<br>Found : | 69.99%C<br>70.36%C | 9.34%H<br>9.32%H | 3.71%N<br>3.69%N |

**EXAMPLE 103**

**erythro-2-Amino-1-[3-(1-dodecynyl)phenyl]-1,3-propanediol acetate**

erythro-N-{1,3-Dihydroxy-1-[3-(1-dodecynyl)-phenyl]-2-propanyl}acetamide (2.7 g), 2N sodium hydroxide solution (36 ml) and ethanol (20 ml) were heated at 60°C for 19 hrs, under nitrogen. The reaction mixture was cooled, and extracted with chloroform. The extract was washed with half-saturated sodium chloride solution, dried over anhydrous magnesium sulfate, filtered, and the filtrate was concentrated. The residue was chromatographed on silica gel, eluting with 950:50:3 chloroform:methanol:2N ammonium hydroxide. The appropriate fractions were collected and evaporated. The residue was dissolved in dichloromethane and treated with glacial acetic (0.27 ml). The mixture was concentrated in vacuo and the solid was recrystallized from ethyl acetate to give 1.72 g (61%) of product, mp 104-105.5°C.

| Analysis: | | | |
|---|---|---|---|
| Calculated for $C_{23}H_{37}NO_4$:<br>Found : | 70.55%C<br>70.62%C | 9.52%H<br>9.46%H | 3.58%N<br>3.54%N |

## REACTION SCHEME A

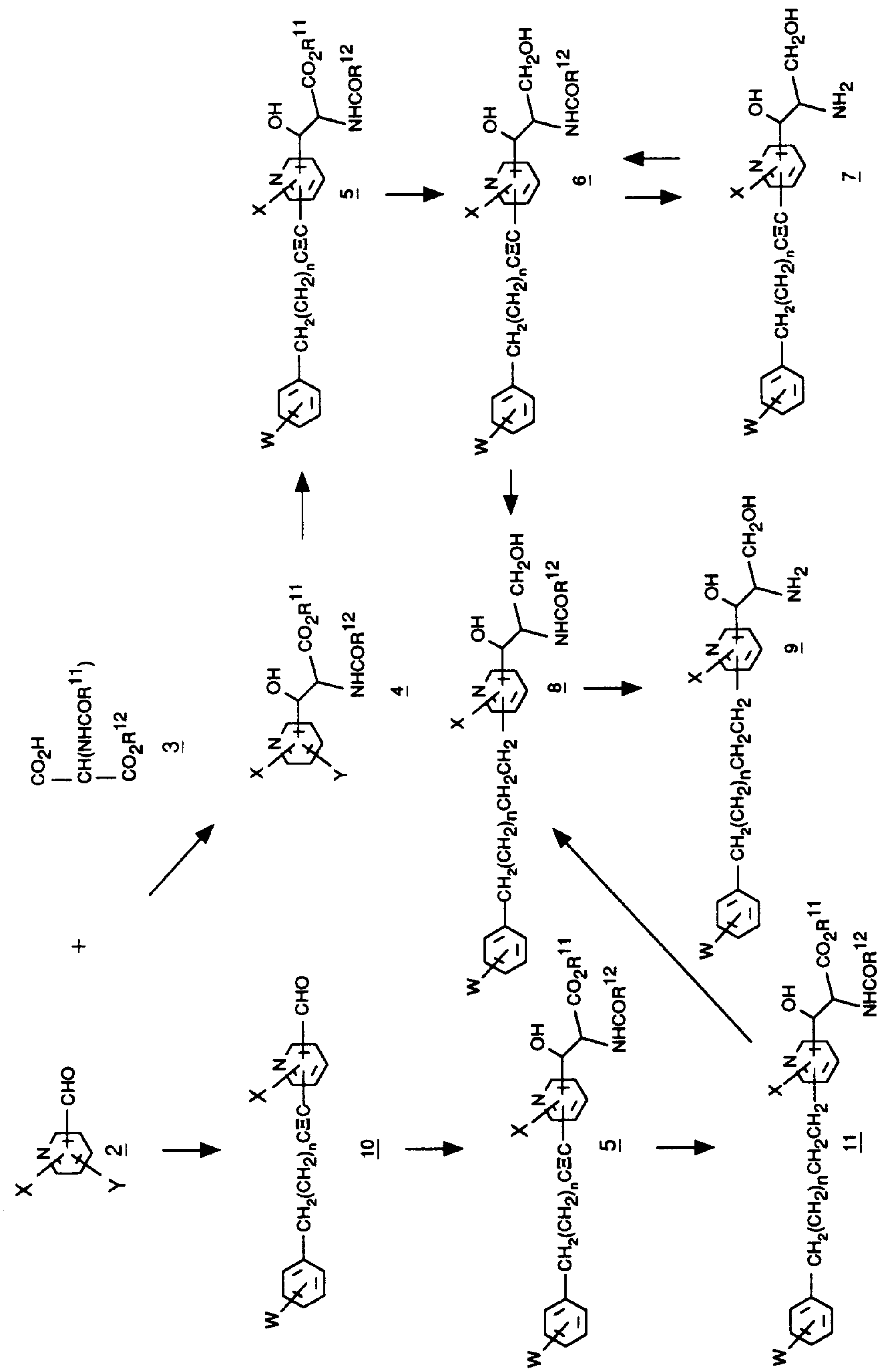

wherein $R^{11}$, $R^{12}$, X, W, Y, and n are as hereinbeforedescribed

## REACTION SCHEME B

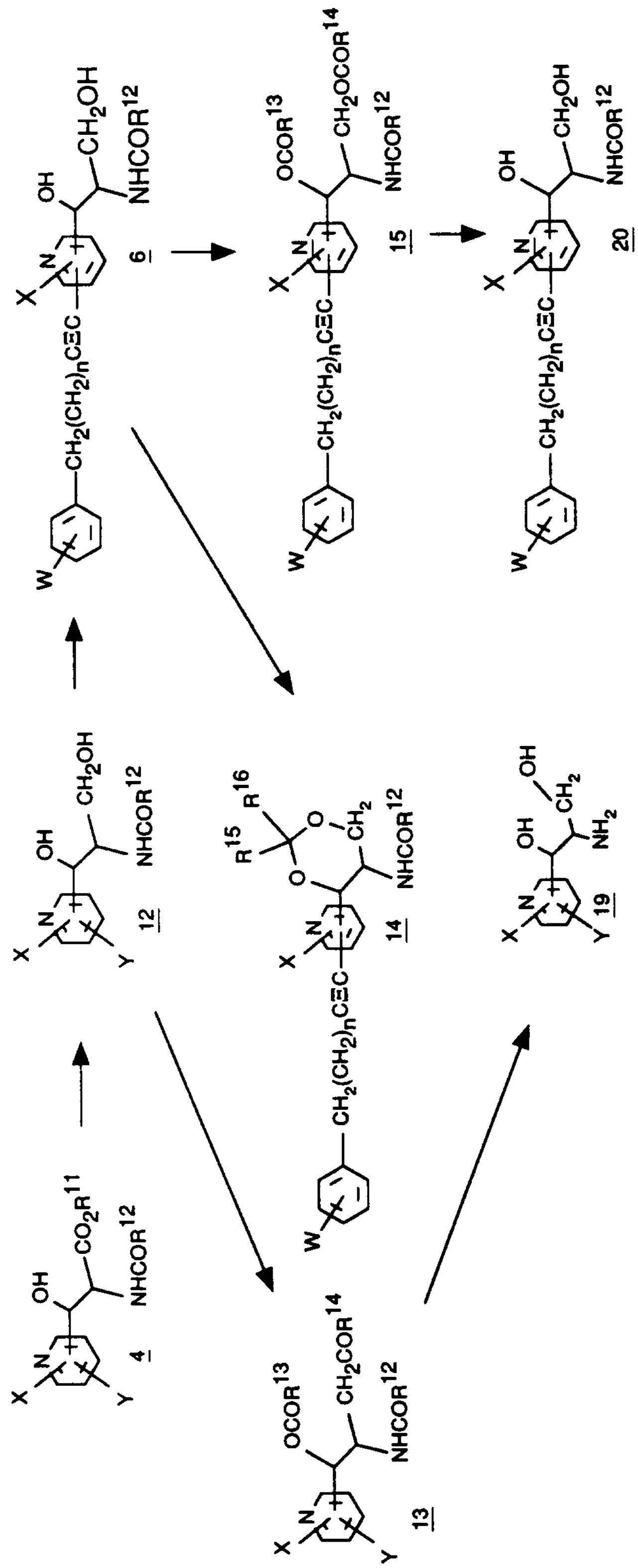

4 12 6 13 14 15 19 20

wherein $R^{11}$, $R^{12}$, $R^{13}$, $R^{14}$, X, Y, W, and n are as hereinbeforedescribed

## REACTION SCHEME C

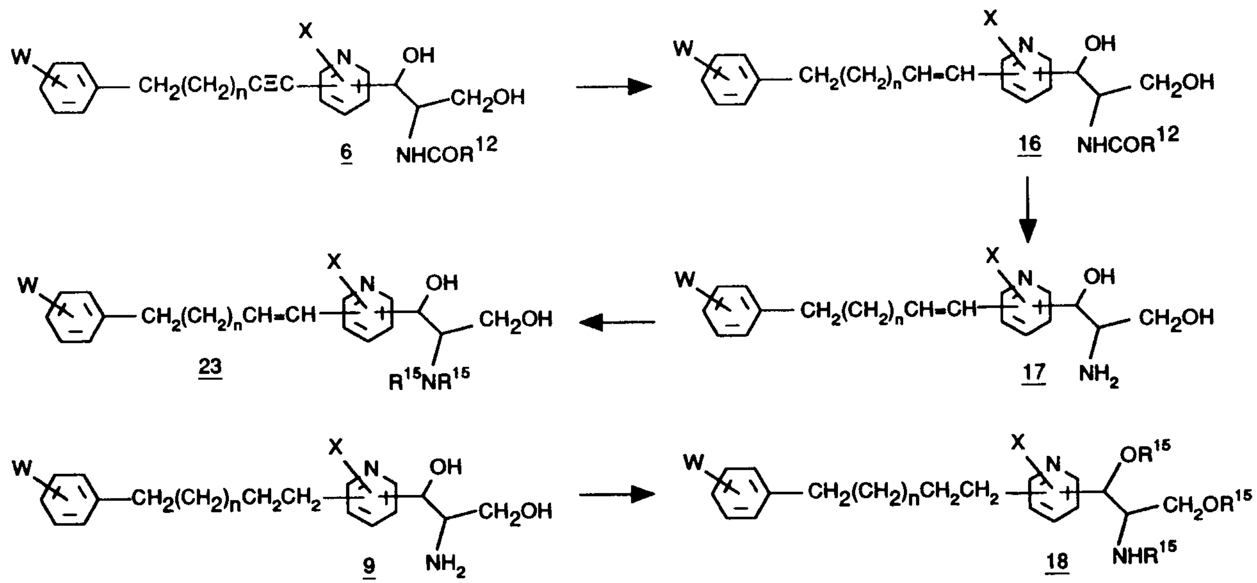


wherein W, X, $R^{12}$, $R^{15}$, and n are as hereinbeforedescribed

REACTION SCHEME D

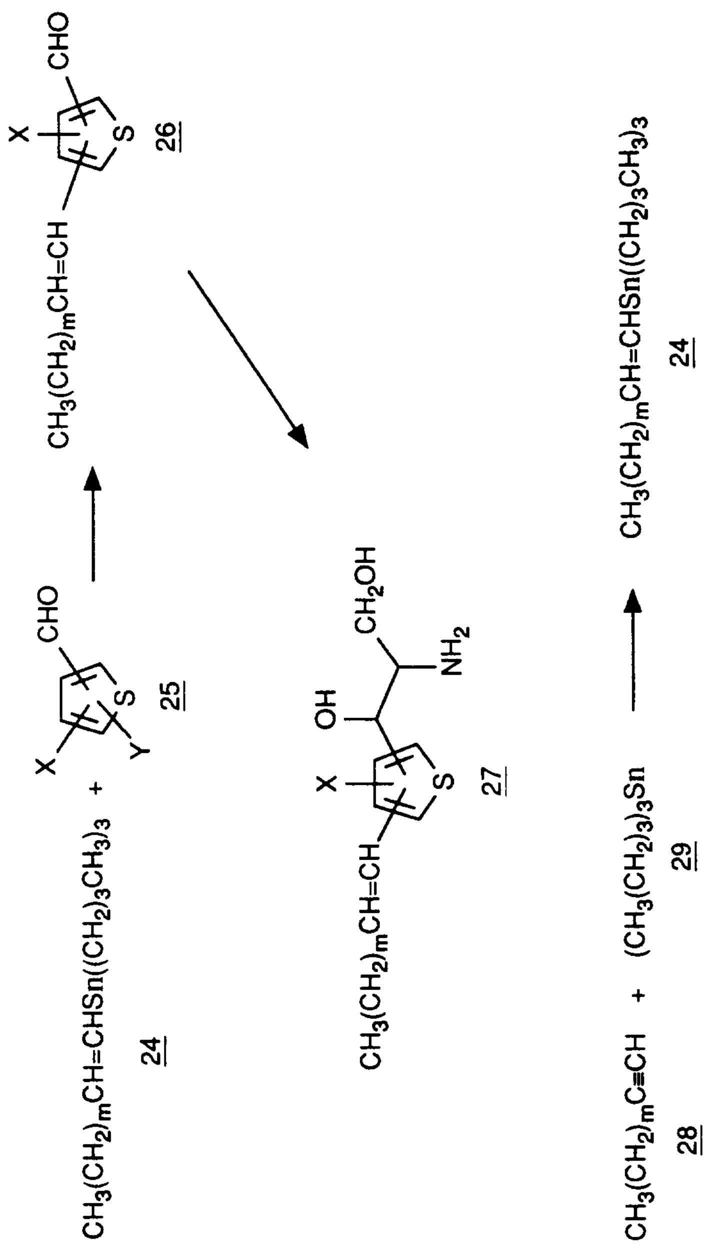

24 25 26 27 28 29 24

wherein X, Y, and m are as hereinbeforedescribed

## Claims

### Claims for the following Contracting States : AT, BE, CH, DE, DK, FR, GB, IT, LI, LU, NL, SE

**1.** A compound of the formula I

$RCH(OR^1)CH(NR^2R^3)R^4$    I

wherein R is

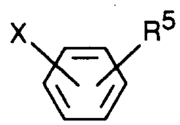,

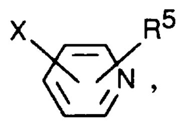, 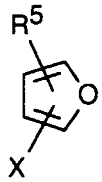, 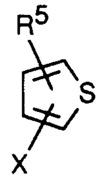, 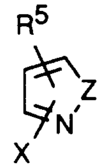, or 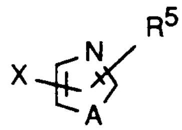

wherein $R^5$ is $CH_3(CH_2)_mC{\equiv}C$, $CH_3(CH_2)_mCH=CH$, $CH_3(CH_2)_mCH_2CH_2$,

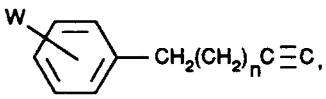 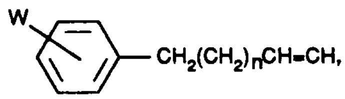 or 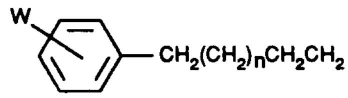

wherein m is 3 to 15, n is 0 to 12, and X is hydrogen, loweralkyl, loweralkoxy, halogen, or trifluoromethyl, and Z is S, O, or C=O; and A is S or O; $R^1$ is hydrogen or

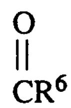

wherein $R^6$ is hydrogen, loweralkyl, loweralkoxy, or

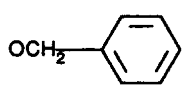 ;

$R^2$ is hydrogen or loweralkyl; $R^3$ is hydrogen, loweralkyl or

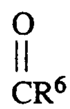

wherein $R^6$ is as above; $R^4$ is

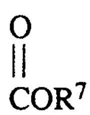

wherein $R^7$ is hydrogen, loweralkyl, or $CH_2OR^8$ wherein $R^8$ is hydrogen or

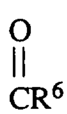

wherein $R^6$ is as above; $R^1$ and $R^8$ taken together with the oxygen to which they are attached form a group of the formula

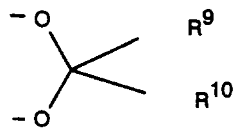

wherein $R^9$ and $R^{10}$ are independently hydrogen or loweralkyl; the optical isomers thereof, or the pharmaceutically acceptable salts thereof, the term lower referring to a group containing up to and including 8 carbon atoms

**2.** A compound as defined in claim 1, wherein R is

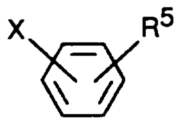 ,

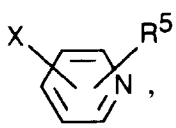 , 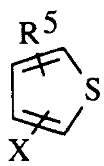 or 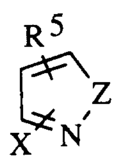 ,

wherein $R^5$ is as defined, X is hydrogen and Z is O.

**3.** A compound as defined in claim 2, wherein R is

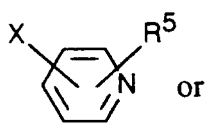 or 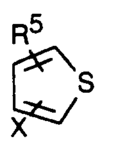

wherein X is hydrogen and $R^5$ is $CH_3(CH_2)_m$ C≡C or $CH_3(CH_2)_m$ $CH_2CH_2$ or

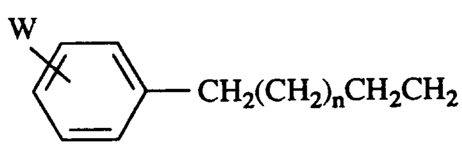 ,

wherein
m and n are as defined, $R^1$ is hydrogen or

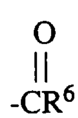 ,

where $R^6$ is loweralkyl,
$R^2$ is hydrogen,
$R^3$ is hydrogen or

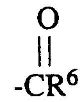

where $R^6$ is as above, and
$R^4$ is as defined.

**4.** A compound as defined in claim 3, where R is

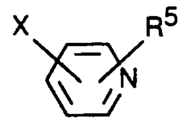,

where $R^1$, $R^2$ and $R^3$ are hydrogen and $R^5$ is $CH_3(CH_2)_mCH_2CH_2$ or

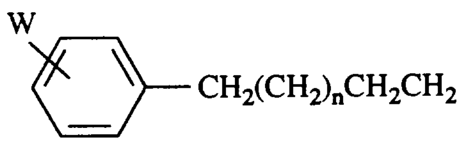,

where W and X are hydrogen and m and n are as defined.

**5.** The compound as defined in claim 1 which is erythro-2-amino-1-(6-decyl-2-pyridinyl)-1,3-dihydroxypropane, or a pharmaceutically acceptable salt thereof.

**6.** The compound as defined in claim 1, which is ethyl erythro-2-acetamido-3-[6-(1-dodecynyl)-2-pyridinyl]-3-hydroxy propionate, or a pharmaceutically acceptable salt thereof.

**7.** The compound as defined in claim 1, which is erythro-2-amino-1-(6-dodecyl-2-pyridinyl)-1,3-propanediol or a pharmaceutically acceptable salt thereof.

**8.** The compound as defined in claim 1 which is erythro-N-{1-6-(1-dodecynyl)-2-pyridinyl]-1,3-dihydroxy-2-propanyl}acetamide or a pharmaceutically acceptable salt thereof.

**9.** The compound as defined in claim 1 which is erythro-2-amino-1-[5-(1-dodecynyl)-2-thienyl]-1,3-propanediol, or a pharmaceuticaly acceptable salt thereof.

**10.** The compound as defined in claim 1 which is erythro-N-{1-[6-(1-decynyl)-2-pyridinyl]-1,3-dihydroxy-2-propanyl} acetamide or a pharmaceutically acceptable salt thereof.

**11.** A pharmaceutical composition which comprises as the active ingredient a compound as defined in claim 1 and a suitable adjuvant.

**12.** Use of a compound as defined in claim 1 for the preparation of a medicament having memory dysfunction relieving, anti inflammatory, cell proliferation reducing antibacterial and/or antifungal activity.

**13.** A compound of the formula

R'CHO

wherein R' is

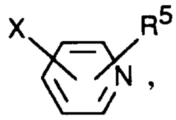 , 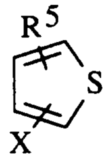 or 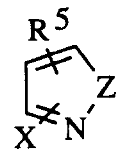 ,

wherein $R^5$ is $CH_3(CH_2)_mC{\equiv}C$, $CH_3(CH_2)_mCH=CH$, $CH_3(CH_2)_mCH_2CH_2$ or

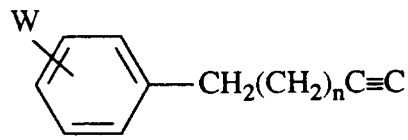 ,

where m is 3 to 15, n is 0 to 12, W and X are independently hydrogen, loweralkyl, loweralkoxy, halogen or trifluoromethyl and Z is O. the term "lower referring to a group containing up to 8 carbon atoms

**14.** A process for the preparation of a compound as defined in claim 1, which comprises

a) reacting a compound of the formula 4a

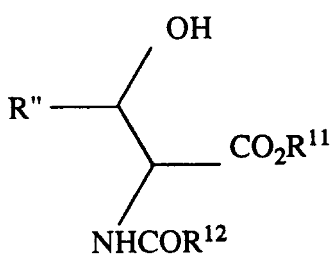

4a

wherein R'' is

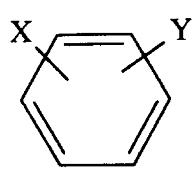 , 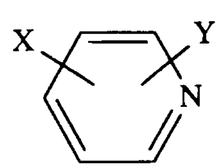 , 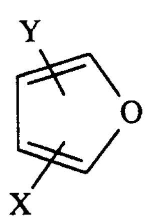 , 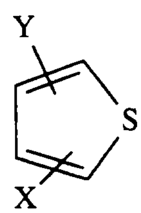 ,

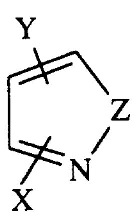 or 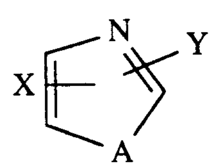

wherein X is as defined and Y is halogen, with an alkyne of the formula $CH_3(CH_2)_mC{\equiv}CH$ or

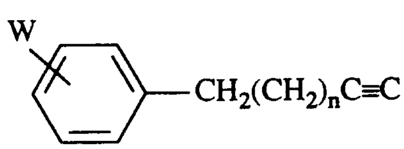 ,

wherein W, m an n are as defined, to form a compound of the formula I wherein R is as defined where $R^5$ is $CH_3(CH_2)_mC{\equiv}C$ or

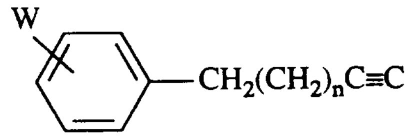

,

$R^1$ and $R^2$ are hydrogen, $R^3$ is $COR^6$ where $R^6$ is loweralkyl and $R^4$ is $CO_2R^7$ where $R^7$ is loweralkyl, or

b) reacting a compound of the formula 10a

RCHO

wherein R is as defined, where $R^5$ is $CH_3(CH_2)_mC{\equiv}C$ or

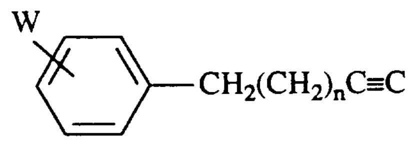

,

with a compound of the formula 3

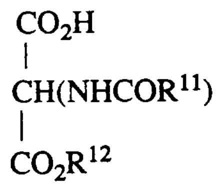

wherein $R^{11}$ and $R^{12}$ are loweralkyl, to form a compound of the formula I as obtained in step a) above,

c) optionally reducing a compound of the formula I as obtained in step a) or b) above, by means of an alkali borohydride to form a compound of the formula I wherein R is as defined, wherein $R^5$ is $CH_3(CH_2)_mC{\equiv}C$ or

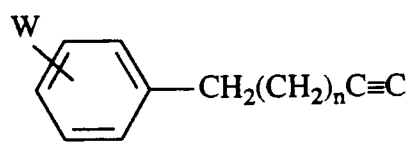

,

$R^1$ and $R^2$ are hydrogen, $R^3$ is $COR^6$ where $R^6$ is loweralkyl and $R^4$ is $CH_2OH$,

d) optionally hydrogenating a compound of the formula I as obtained in step a), b) or c) to form a compound of the formula I, where R is as defined where $R^5$ is $CH_3(CH_2)_m CH_2CH_2$ or

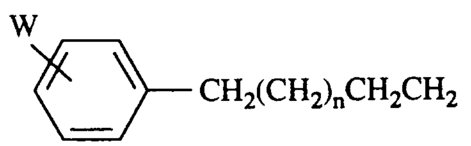

,

$R^1$ and $R^2$ are hydrogen, $R^3$ is $COR^6$ where $R^6$ is loweralkyl and $R^4$ is $CO_2R^7$, where $R^7$ is loweralkyl, or $R^4$ is $CH_2OH$,

e) optionally reducing a compound of the formula I, wherein R is as defined where $R^5$ is $CH_3(CH_2)_mCH_2CH_2$ or

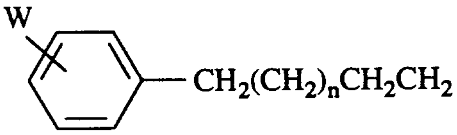

,

$R^1$ and $R^2$ are hydrogen, $R^3$ is $COR^6$ where $R^6$ is loweralkyl and $R^4$ is $CO_2R^7$ where $R^7$ is loweralkyl, by means of an alkali borohydride to form a compound of the formula I, where R, $R^5$, $R^1$, $R^3$ and $R^2$ are as hereinbefore described and $R^4$ is $CH_2OH$,

f) optionally hydrogenating a compound of the formula I, wherein R is as defined, where $R^5$ is $CH_3$-$(CH_2)_nC{\equiv}C$ or

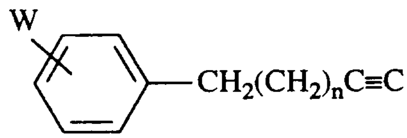

,

$R^1$ and $R^2$ are hydrogen, $R^3$ is $COR^6$ where $R^6$ is loweralkyl and $R^4$ is $CH_2OH$, to form a compound of the formula I wherein R, $R^1$, $R^2$, $R^3$ and $R^4$ are as hereinbefore described and $R^5$ is $CH_3(CH_2)$-$_nCH=CH$ or

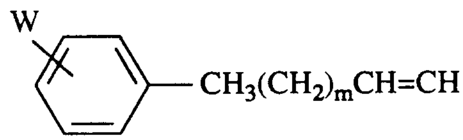

,

g) optionally hydrolyzing a compound of the formula I, wherein R is as defined, $R^1$ and $R^2$ are hydrogen, $R^3$ is $COR^6$ where $R^6$ is loweralkyl, and $R^4$ is $CH_2OH$, to form a compound of the formula I wherein R, $R^5$, $R^1$, $R^2$ and $R^4$ are as hereinbefore described and $R^3$ is hydrogen,

h) optionally reacting a compound of the formula I, wherein R is as defined, $R^1$, $R^2$ and $R^3$ are hydrogen and $R^4$ is $CH_2OH$, with a loweraldehyd in the presence of a reducing agent to form a compound of the formula I, wherein R and $R^5$ are as defined, $R^1$ is hydrogen, $R^4$ is $CH_2OH$ and $R^2$ and $R^3$ are loweralkyl,

i) optionally reacting a compound of the formula I, wherein R is as defined, $R^1$, $R^2$ and $R^3$ are hydrogen and $R^4$ is $-CH_2OH$, with N-benzyloxycarbonyloxy-succinimide of the formula 20

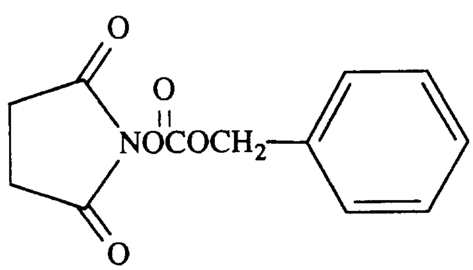

to form a compound of the formula I, where R and $R^5$ are as defined $R^1$ and $R^3$ are benzyloxycarbonyl and $R^4$ is $CH_2OR^8$ where $R^8$ is benzyloxycarbonyl,

j) optionally reacting a compound of the formula I wherein R is as defined, $R^1$ and $R^2$ are hydrogen, $R^3$ is $COR^6$ where $R^6$ is loweralkyl and $R^4$ is $CH_2OH$,

k) optionally acylating a compound of the formula I wherein R is as defined, $R^1$ and $R^2$ are hydrogen, $R^3$ is $COR^6$ where $R^6$ is loweralkyl and $R_4$ is $CH_2OH$, to form a compound of the formula I, wherein R, $R^5$, $R^1$, $R^2$ and $R^3$ are as hereinbefore described and $R^4$ is $CH_2OCOR^6$ where $R^6$ is loweralkyl,

l) optionally reacting a compound of the formula I wherein R is as defined, $R^1$ and $R^2$ are hydrogen, $R^3$ is $COR^6$ where $R^6$ is loweralkyl and $R^4$ is $CH_2OH$ with 2,2-dimethoxy-propane, to form a compound of the formula I, wherein R, $R^2$ and $R^3$ are as defined, $R^4$ is $CH_2OR^8$ and $R^1$ and $R^8$

taken together form a group of the formula

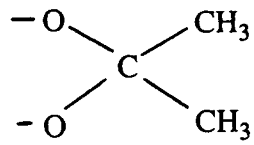 .

## Claims for the following Contracting States : ES, GR

**1.** A process for the preparation of a compound of the formula I

$RCH(OR^1)CH(NR^2R^3)R^4$ I

wherein R is

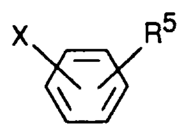 ,

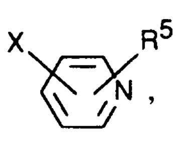 , 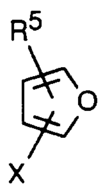 , 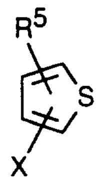 , 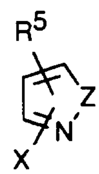 , or 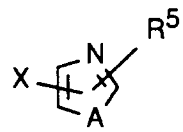

wherein $R^5$ is $CH_3(CH_2)_mC{\equiv}C$, $CH_3(CH_2)_mCH=CH$, $CH_3(CH_2)_mCH_2CH_2$,

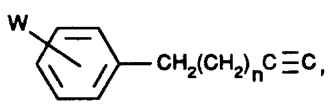 , 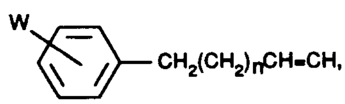 or 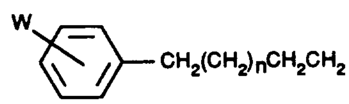

wherein m is 3 to 15, n is 0 to 12, and X is hydrogen, loweralkyl, loweralkoxy, halogen, or trifluoromethyl, and Z is S, O, or C = O; and A is S or O; $R^1$ is hydrogen or

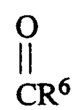

wherein $R^6$ is hydrogen, loweralkyl, loweralkoxy, or

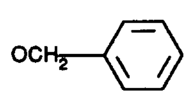 ;

$R^2$ is hydrogen or loweralkyl; $R^3$ is hydrogen, loweralkyl or

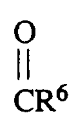

wherein $R^6$ is as above; $R^4$ is

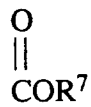

wherein $R^7$ is hydrogen, loweralkyl, or $CH_2OR^8$ wherein $R^8$ is hydrogen or

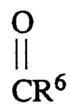

wherein $R^6$ is as above; $R^1$ and $R^8$ taken together with the oxygen to which they are attached form a group of the formula

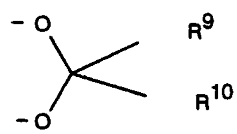

wherein $R^9$ and $R^{10}$ are independently hydrogen or loweralkyl; the optical isomers thereof, or the pharmaceutically acceptable salts thereof, the term lower referring to a group containing up to and including 8 carbon atoms, which comprises

a) reacting a compound of the formula 4a

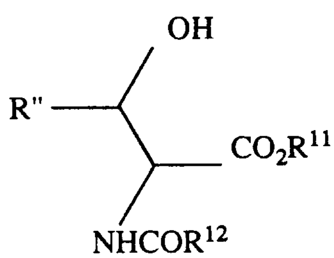

4a

wherein R" is

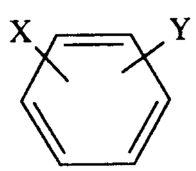 , 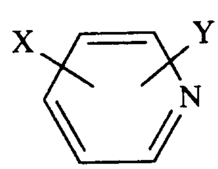 , 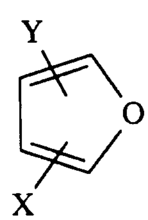 , 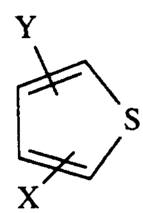 ,

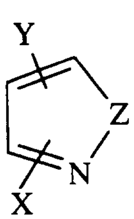 or 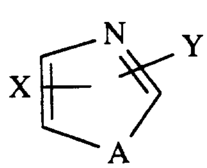

wherein X is as defined and Y is halogen, with an alkyne of the formula $CH_3(CH_2)_mC{\equiv}CH$ or

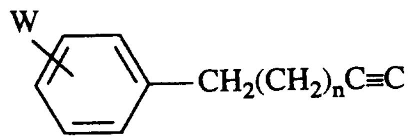,

wherein W, m an n are as defined, to form a compound of the formula I wherein R is as defined where $R^5$ is $CH_3(CH_2)_mC{\equiv}C$ or

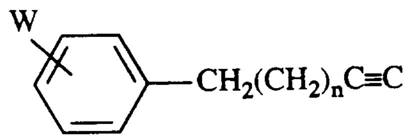,

$R^1$ and $R^2$ are hydrogen, $R^3$ is $COR^6$ where $R^6$ is loweralkyl and $R^4$ is $CO_2R^7$ where $R^7$ is loweralkyl, or
b) reacting a compound of the formula 10a

RCHO

wherein R is as defined, where $R^5$ is $CH_3(CH_2)_m$ $C{\equiv}C$ or

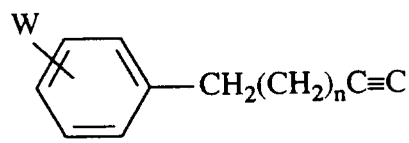,

with a compound of the formula 3

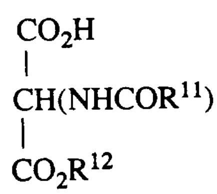

wherein $R^{11}$ and $R^{12}$ are loweralkyl, to form a compound of the formula I as obtained in step a) above,
c) optionally reducing a compound of the formula I as obtained in step a) or b) above, by means of an alkali borohydride to form a compound of the formula I wherein R is as defined, wherein $R^5$ is $CH_3(CH_2)_mC{\equiv}C$ or

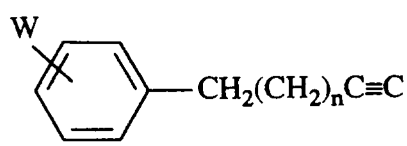,

$R^1$ and $R^2$ are hydrogen, $R^3$ is $COR^6$ where $R^6$ is loweralkyl and $R^4$ is $CH_2OH$,
d) optionally hydrogenating a compound of the formula I as obtained in step a), b) or c) to form a compound of the formula I, where R is as defined where $R^5$ is $CH_3(CH_2)_m$ $CH_2CH_2$ or

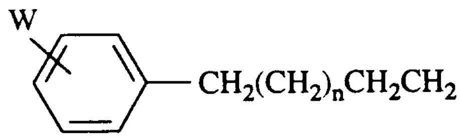,

$R^1$ and $R^2$ are hydrogen, $R^3$ is $COR^6$ where $R^6$ is loweralkyl and $R^4$ is $CO_2R^7$, where $R^7$ is loweralkyl or $R^4$ is $CH_2OH$,

e) optionally reducing a compound of the formula I, wherein R is as defined where $R^5$ is $CH_3(CH_2)_mCH_2CH_2$ or

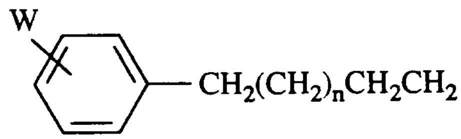,

$R^1$ and $R^2$ are hydrogen, $R^3$ is $COR^6$ where $R^6$ is loweralkyl and $R^4$ is $CO_2R^7$ where $R^7$ is loweralkyl, by means of an alkali borohydride to form a compound of the formula I, where R, $R^5$, $R^1$, $R^2$ and $R^3$ are as hereinbefore described and $R^4$ is $CH_2OH$,

f) optionally hydrogenating a compound of the formula I, wherein R is as defined, where $R^5$ is $CH_3(CH_2)_nC{\equiv}C$ or

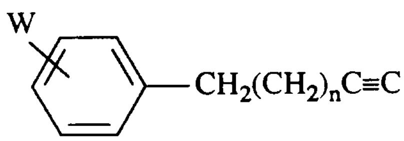,

$R^1$ and $R^2$ are hydrogen, $R^3$ is $COR^6$ where $R^6$ is loweralkyl and $R^4$ is $CH_2OH$, to form a compound of the formula I wherein R, $R^1$, $R^2$, $R^3$ and $R^4$ are as hereinbefore described and $R^5$ is $CH_3(CH_2)_n CH = CH$ or

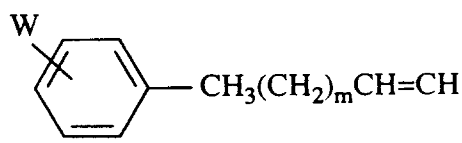,

g) optionally hydrolyzing a compound of the formula I, wherein R is as defined, $R^1$ and $R^2$ are hydrogen, $R^3$ is $COR^6$ where $R^6$ is loweralkyl, and $R^4$ is $CH_2OH$, to form a compound of the formula I wherein R, $R^5$, $R^1$, $R^2$ and $R^4$ are as hereinbefore described and $R^3$ is hydrogen,

h) optionally reacting a compound of the formula I, wherein R is as defined, $R^1$, $R^2$ and $R^3$ are hydrogen and $R^4$ is $CH_2OH$, with a loweraldehyd in the presence of a reducing agent to form a compound of the formula I, wherein R and $R^5$ are as defined, $R^1$ is hydrogen, $R^4$ is $CH_2OH$ and $R^2$ and $R^3$ are loweralkyl,

i) optionally reacting a compound of the formula I, wherein R is as defined, $R^1$, $R^2$ and $R^3$ are hydrogen and $R^4$ is $-CH_2OH$, with N-benzyloxycarbonyloxy-succinimide of the formula 20

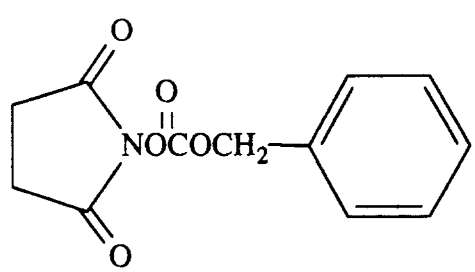

to form a compound of the formula I, where R and $R^5$ are as defined, $R^1$ and $R^3$ are benzyloxycarbonyl and $R^4$ is $CH_2OR^8$ where $R^8$ is benzyloxycarbonyl,
j) optionally reacting a compound of the formula I wherein R is as defined, $R^1$ and $R^2$ are hydrogen, $R^3$ is $COR^6$ where $R^6$ is loweralkyl and $R^4$ is $CH_2OH$,
k) optionally acylating a compound of the formula I wherein R is as defined, $R^1$ and $R^2$ are hydrogen, $R^3$ is $COR^6$ where $R^6$ is loweralkyl and $R^4$ is $CH_2OH$, to form a compound of the formula I, wherein R, $R^5$, $R^1$, $R^2$ and $R^3$ are as hereinbefore described and $R^4$ is $CH_2OCOR^6$ where $R^6$ is loweralkyl,
l) optionally reacting a compound of the formula I wherein R is as defined, $R^1$ and $R^2$ are hydrogen, $R^3$ is $COR^6$ where $R^6$ is loweralkyl and $R^4$ is $CH_2OH$ with 2,2-dimethoxy-propane, to form a compound of the formula I, wherein R, $R^2$ and $R^3$ are as defined, $R^4$ is $CH_2OR^8$ and $R^1$ and $R^8$ taken together form a group of the formula

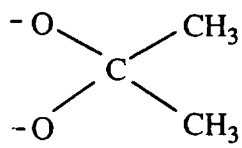

.

2. A process as defined in claim 1, wherein R is

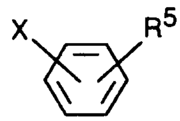

,

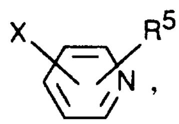 , 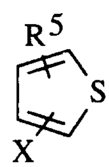 or 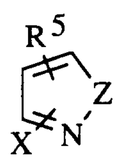 ,

wherein $R^5$ is as defined, X is hydrogen and Z is O.

3. A process as defined in claim 2, wherein R is

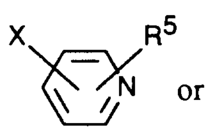 or 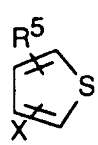

wherein X is hydrogen and $R^5$ is $CH_3(CH_2)_mC{\equiv}C$, or $CH_3(CH_2)_mCH_2CH_2$ or

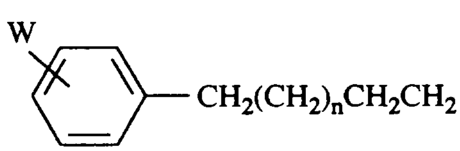

,

wherein m and n are as defined, $R^1$ is hydrogen or

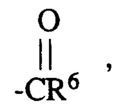

where $R^6$ is loweralkyl,
$R^2$ is hydrogen
$R^3$ is hydrogen or

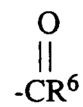

where $R^6$ is as above, and
$R^4$ is as defined.

**4.** A process as defined in claim 3, where R is

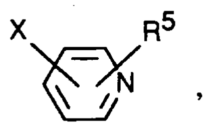

where $R^1$, $R^2$ and $R^3$ are hydrogen and $R^5$ is $CH_3(CH_2)_mCH_2CH_2$
or

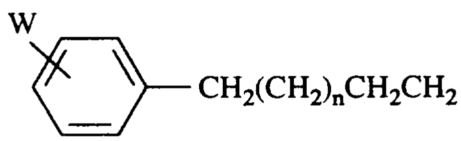

where W and X are hydrogen and m and n are as defined.

**5.** The process as defined in claim 1 wherein erythro-2-amino-1-(6-decyl-2-pyridinyl)-1,3-dihydroxypropane, or a pharmaceutically acceptable salt thereof is prepared.

**6.** The process as defined in claim 1, wherein ethyl erythro-2-acetamido-3-[6-(1-dodecynyl)-2-pyridinyl]-3-hydroxy propionate, or a pharmaceutically acceptable salt thereof is prepared.

**7.** The process as defined in claim 1, wherein erythro-2-amino-1-(6-dodecyl-2-pyridinyl)-1,3-propanediol or a pharmaceutically acceptable salt thereof is prepared.

**8.** The process as defined in claim 1 , wherein erythro-N-{1-[6-(1-dodecynyl)-2-pyridinyl]-1,3-dihydroxy-2-propanyl}acetamide or a pharmaceutically acceptable salt thereof is prepared.

**9.** The process as defined in claim 1, wherein erythro-2-amino-1-[5-(1-dodecynyl)-2-thienyl]-1,3-propanediol, or a pharmaceuticaly acceptable salt thereof is prepared.

**10.** The process as defined in claim 1, wherein erythro-N-{1-[6-(1-decynyl)-2-pyridinyl]-1,3-dihydroxy-2-propanyl}acetamide or a pharmaceutically acceptable salt thereof is prepared.

**11.** Use of a compound as defined in claim 1 for the preparation of a medicament having memory dysfunction relieving, anti inflammatory, cell proliferation reducing antibacterial and/or antifungal activity.

**12.** A compound of the formula 1a

R'CHO

wherein R' is

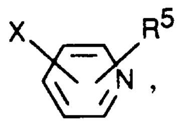 , 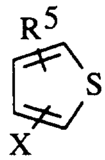 or 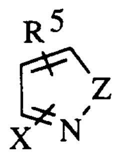

wherein $R^5$ is $CH_3(CH_2)_mC{\equiv}C$, $CH_3(CH_2)_mCH=CH$, $CH_3(CH_2)_mCH_2CH_2$ or

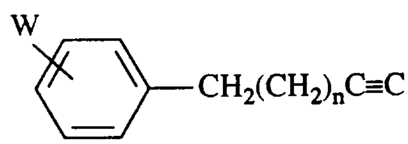 ,

where m is 3 to 15, n is 0 to 12, W and X are independently hydrogen, loweralkyl, loweralkoxy, halogen or trifluoromethyl and Z is O, the term lower referring to a group containing upto and including 8 carbon atoms

**Patentansprüche**
**Patentansprüche für folgende Vertragsstaaten : AT, BE, CH, DE, DK, FR, GB, IT, LI, LU, NL, SE**

**1.** Verbindung der Formel I

$RCH(OR^1)CH(NR^2R^3)R^4$ I

in welcher R

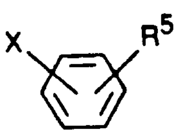 , 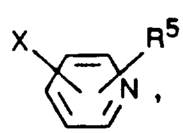 , 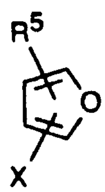 , 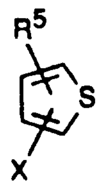 , 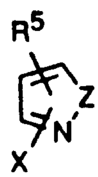 oder 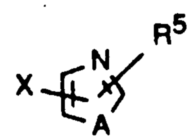

bedeutet, wobei $R^5$ $CH_3(CH_2)_mC{\equiv}C$, $CH_3(CH_2)_mCH=CH$, $CH_3(CH_2)_mCH_2CH_2$,

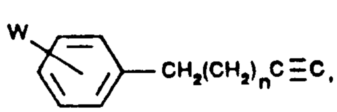 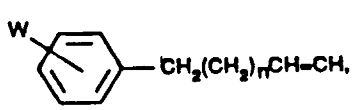 oder 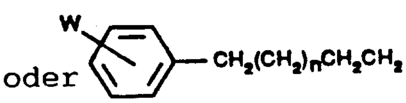

darstellt, wobei m für 3 bis 15, n für 0 bis 12 und X für Wasserstoff, Niederalkyl, Niederalkoxy, Halogen oder Trifluormethyl und Z für S, O oder C=O steht; und A für S oder O steht; $R^1$ Wasserstoff oder

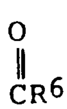

bedeutet, wobei $R^6$ für Wasserstoff, Niederalkyl, Niederalkoxy oder

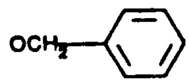

steht; $R^2$ Wasserstoff oder Niederalkyl bedeutet; $R^3$ Wasserstoff, Niederalkyl oder

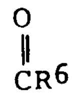

ist, wobei $R^6$ wie vorstehend angegeben definiert ist; $R^4$ für

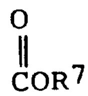

steht, wobei $R^7$ Wasserstoff, Niederalkyl oder $CH_2OR^8$ bedeutet, wobei $R^8$ Wasserstoff oder

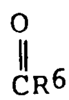

darstellt, wobei $R^6$ wie vorstehend angegeben definiert ist; $R^1$ und $R^8$ zusammen mit dem Sauerstoff, an den sie gebunden sind, eine Gruppe der Formel

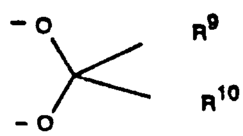

bilden, in welcher $R^9$ und $R^{10}$ unabhängig voneinander Wasserstoff oder Niederalkyl, deren optische Isomere oder deren pharmazeutisch verträgliche Salze darstellen, und wobei der Begriff "Nieder" sich auf eine Gruppe mit bis zu und einschließlich 8 Kohlenstoffatomen bezieht.

2. Verbindung gemäß Anspruch 1, wobei R

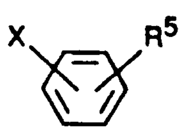 , 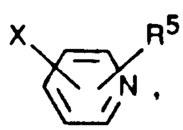 , 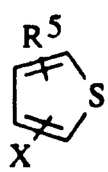 oder 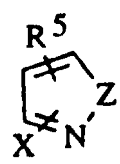

darstellt, wobei $R^5$ wie vorstehend angegeben definiert ist, X für Wasserstoff und Z für O steht.

3. Verbindung gemäß Anspruch 2, wobei
R

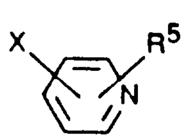 oder 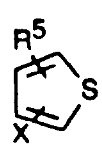

bedeutet, wobei X für Wasserstoff und $R^5$ für $CH_3(CH_2)_mC{\equiv}C$ oder $CH_3(CH_2)_mCH_2CH_2$ oder

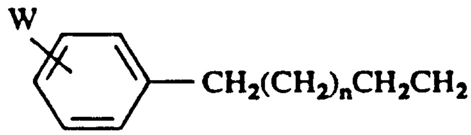

steht, wobei
m und n wie vorstehend angegeben definiert sind,
$R^1$ Wasserstoff oder

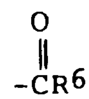

bedeutet, wobei $R^6$ für Niederalkyl steht,
$R^2$ Wasserstoff ist,
$R^3$ Wasserstoff oder

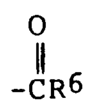

bedeutet, wobei $R^6$ wie vorstehend angegeben definiert ist, und
$R^4$ die weiter oben angewiesene Bedeutung zukommt.

4. Verbindung gemäß Anspruch 3, wobei R

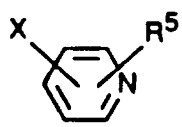

darstellt, wobei $R^1$, $R^2$ und $R^3$ für Wasserstoff stehen und $R^5$ für $CH_3(CH_2)_mCH_2CH_2$ oder

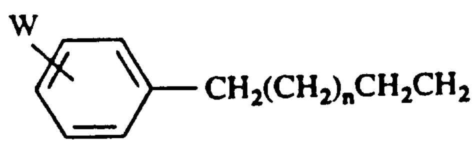

steht, wobei W und X Wasserstoff darstellen und m und n wie vorstehend angegeben definiert sind.

5. Verbindung gemäß Anspruch 1, bei der es sich um Erythro-2-amino-1-(6-decyl-2-pyridinyl)-1,3-dihydroxypropan oder ein pharmazeutisch verträgliches Salz davon handelt.

6. Verbindung gemäß Anspruch 1, bei der es sich um Ethylerythro-2-acetamido-3-[6-(1-dodecynyl)-2-pyridinyl]-3-hydroxypropionat oder ein pharmazeutisch verträgliches Salz davon handelt.

7. Verbindung gemäß Anspruch 1, bei der es sich um Erythro-2-amino-1-(6-dodecyl-2-pyridinyl)-1,3-propandiol oder ein pharmazeutisch verträgliches Salz davon handelt.

8. Verbindung gemäß Anspruch 1, bei der es sich um Erythro-N-{1-[6-(1-dodecynyl)-2-pyridinyl]-1,3-dihydroxy-2-propanyl}acetamid oder ein pharmazeutisch verträgliches Salz davon handelt.

**9.** Verbindung gemäß Anspruch 1, bei der es sich um Erythro-2-amino-1-[5-(1-dodecynyl)-2-thienyl]-1,3-propandiol oder ein pharmazeutisch verträgliches Salz davon handelt.

**10.** Verbindung gemäß Anspruch 1, bei der es sich um Erythro-N-{1-[6-(1-decynyl)-2-pyridinyl]-1,3-dihydroxy-2-propanyl}acetamid oder ein pharmazeutisch verträgliches Salz davon handelt.

**11.** Pharmazeutische Zusammensetzung, die als Wirkstoff eine Verbindung gemäß Anspruch 1 und einen geeigneten Hilfsstoff enthält.

**12.** Verwendung einer Verbindung gemäß Anspruch 1 zur Herstellung eines Medikaments zur Erleichterung von Gedächtnisstörungen, mit entzündungshemmender, die Zellproliferation verringernder, antibakterieller und/oder antimykotischer Wirksamkeit.

**13.** Verbindung der Formel

R'CHO

in welcher R'

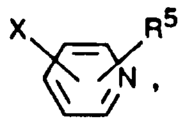,
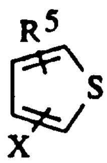
oder
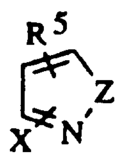

bedeutet, wobei $R^5$ $CH_3(CH_2)_mC{\equiv}C$, $CH_3(CH_2)_mCH=CH$, $CH_3(CH_2)_mCH_2CH_2$ oder

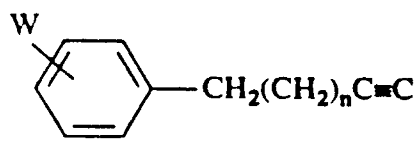

darstellt, wobei m für 3 bis 15, n für 0 bis 12, und W und X unabhängig voneinander für Wasserstoff, Niederalkyl, Niederalkoxy, Halogen oder Trifluormethyl und Z für O stehen; der Begriff "Nieder" bezeichnet eine Gruppe mit bis zu 8 Kohlenstoffatomen.

**14.** Verfahren zur Herstellung einer Verbindung nach der Definition von Anspruch 1, das folgende Schritte umfaßt:
a) Umsetzen einer Verbindung der Formel 4a

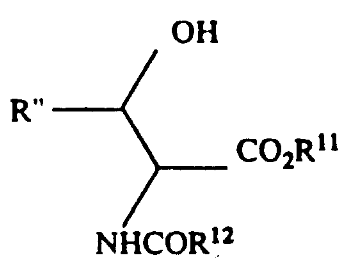

4a

in welcher R"

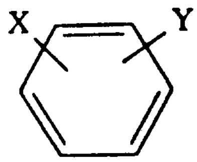, 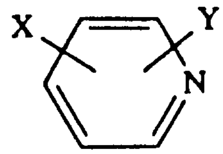, 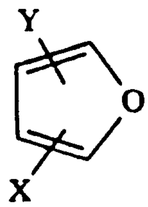, 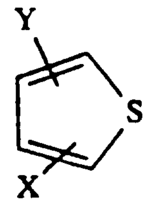,

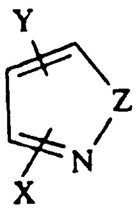 oder 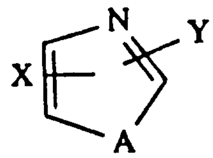

bedeutet, wobei X wie vorstehend angegeben definiert ist und Y ein Halogen darstellt, mit einem Alkyn der Formel $CH_3(CH_2)_mC{\equiv}CH$ oder

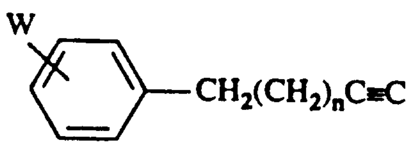 ,

wobei W, m und n wie vorstehend angegeben definiert sind, zur Bildung einer Verbindung der Formel I, in welcher R die weiter oben angewiesene Bedeutung zukommt, $R^5$ für $CH_3(CH_2)_mC{\equiv}C$ oder

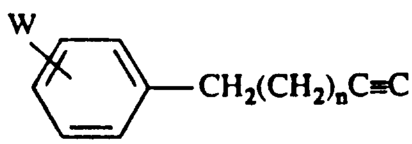

steht, $R^1$ und $R^2$ Wasserstoff und $R^3$ $COR^6$ darstellen, wobei $R^6$ Niederalkyl ist, und $R^4$ $CO_2R^7$ entspricht, wobei $R^7$ Niederalkyl ist, oder
b) Umsetzen einer Verbindung der Formel 10a

RCHO

in welcher R wie vorstehend angegeben definiert ist, wobei $R^5$ für $CH_3(CH_2)_mC{\equiv}C$ oder

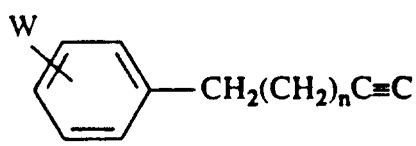

steht, mit einer Verbindung nach Formel 3

$CO_2H$
|
$CH(NHCOR^{11})$
|
$CO_2R^{12}$,

in welcher $R^{11}$ und $R^{12}$ für Niederalkyl stehen, zur Bildung einer Verbindung der Formel I, wie sie

nach dem vorstehend beschriebenen Schritt a) erhalten wird,

c) wahlweise Reduzieren einer Verbindung der Formel I, wie sie nach dem vorstehend beschriebenen Schritt a) oder b) erhalten wird, durch Einsatz eines Alkaliborhydrids zur Bildung einer Verbindung der Formel I, in welcher R die weiter oben angewiesene Bedeutung zukommt und $R^5$ für $CH_3(CH_2)_mC{\equiv}C$ oder

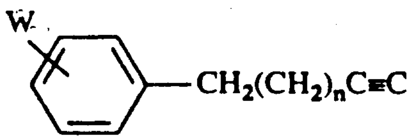

steht, $R^1$ und $R^2$ für Wasserstoff, $R^3$ für $COR^6$ mit $R^6$ als Niederalkyl und $R^4$ für $CH_2OH$ stehen,

d) wahlweise Hydrogenieren einer Verbindung der Formel I, wie sie in Schritt a), b) oder c) erhalten wurde, zur Bildung einer Verbindung der Formel I, in welcher R die weiter oben angewiesene Bedeutung zukommt, und $R^5$ für $CH_3(CH_2)_mCH_2CH_2$ oder

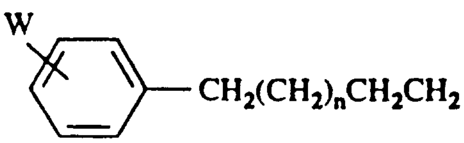

steht, $R^1$ und $R^2$ Wasserstoff darstellen und $R^3$ für $COR^6$ steht, wobei $R^6$ Niederalkyl ist, und $R^4$ $CO_2R^7$ bedeutet, wobei $R^7$ Niederalkyl ist, oder $R^4$ $CH_2OH$ bedeutet,

e) wahlweise Reduzieren einer Verbindung der Formel I, in welcher R wie vorstehend angegeben definiert ist, wobei $R^5$ $CH_3(CH_2)_mCH_2CH_2$ oder

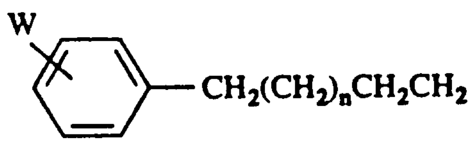

bedeutet, $R^1$ und $R^2$ Wasserstoff sind, $R^3$ für $COR^6$ steht, wobei $R^6$ Niederalkyl ist, und $R^4$ $CO_2R^7$ bedeutet, wobei $R^7$ Niederalkyl ist, mit Hilfe eines Alkaliborhydrids zur Bildung einer Verbindung der Formel I, in welcher R, $R^5$, $R^1$, $R^3$ und $R^2$ wie vorstehend beschrieben definiert sind und $R^4$ für $CH_2OH$ steht,

f) wahlweise Hydrogenieren einer Verbindung der Formel I, in welcher R wie vorstehend beschrieben definiert ist, wobei $R^5$ für $CH_3(CH_2)_nC{\equiv}C$ oder

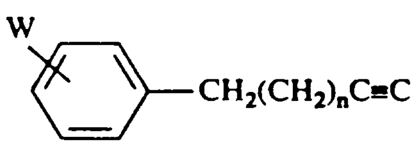

steht, $R^1$ und $R^2$ für Wasserstoff, $R^3$ für $COR^6$, wobei $R^6$ Niederalkyl ist, und $R^4$ für $CH_2OH$ stehen, zur Bildung einer Verbindung der Formel I, in welcher R, $R^1$, $R^2$, $R^3$ und $R^4$ wie vorstehend beschrieben definiert sind und $R^5$ für $CH_3(CH_2)_nCH=CH$ oder

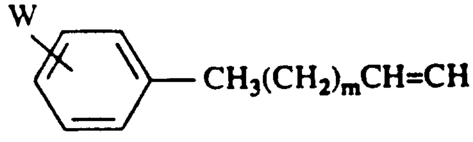

steht,

g) wahlweise Hydrolyse einer Verbindung der Formel I, in welcher R wie vorstehend angegeben definiert ist, $R^1$ und $R^2$ Wasserstoff sind, $R^3$ für $COR^6$ steht, wobei $R^6$ Niederalkyl ist und $R^4$ $CH_2OH$ entspricht, zur Bildung einer Verbindung Formel I, in welcher R, $R^5$, $R^1$, $R^2$ und $R^4$ wie vorstehend beschrieben definiert sind und $R^3$ Wasserstoff bedeutet,

h) wahlweise Umsetzen einer Verbindung der Formel I, in welcher R wie vorstehend angegeben definiert ist, $R^1$, $R^2$ und $R^3$ Wasserstoff sind, und $R^4$ für $CH_2OH$ steht, mit einem Niederaldehyd in Gegenwart eines Reduktionsmittel zur Bildung einer Verbindung der Formel I, in welcher R und $R^5$ die weiter oben angewiesene Bedeutung zukommt, $R^1$ für Wasserstoff, $R^4$ für $CH_2OH$ und $R^2$ und $R^3$ für Niederalkyl stehen,

i) wahlweise Umsetzen einer Verbindung der Formel I, in welcher R wie vorstehend angegeben definiert ist, $R^1$, $R^2$ und $R^3$ für Wasserstoff und $R^4$ für $-CH_2OH$ stehen, mit N-Benzyloxycarbonyloxy-succinimid der Formel 20

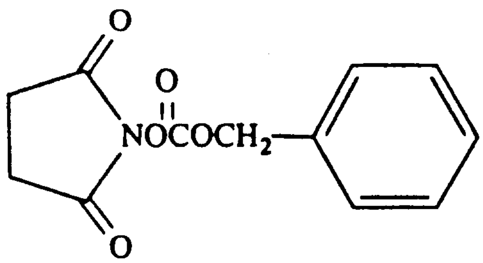

zur Bildung einer Verbindung der Formel I, in welcher R und $R^5$ wie vorstehend angegeben definiert sind, $R^1$ und $R^3$ für Benzyloxycarbonyl und $R^4$ für $CH_2OR^8$ stehen, wobei $R^8$ Benzyloxycarbonyl bedeutet,

j) wahlweise Umsetzen einer Verbindung der Formel I, in welcher R wie vorstehend angegeben definiert ist, $R^1$ und $R^2$ für Wasserstoff, $R^3$ für $COR^6$ mit $R^6$ als Niederalkyl und $R^4$ für $CH_2OH$ stehen,

k) wahlweise Acylieren einer Verbindung der Formel I, in welcher R wie vorstehend angegeben definiert ist, $R^1$ und $R^2$ für Wasserstoff stehen, $R^3$ $COR^6$ bedeutet, wobei $R^6$ Niederalkyl ist, und $R^4$ $CH_2OH$ entspricht, zur Bildung einer Verbindung der Formel I, in welcher R, $R^5$, $R^1$, $R^2$ und $R^3$ wie vorstehend beschrieben definiert sind und $R^4$ für $CH_2OCOR^6$ steht, wobei $R^6$ Niederalkyl ist,

l) wahlweise Umsetzen einer Verbindung der Formel I, in welcher R wie vorstehend angegeben definiert ist, $R^1$ und $R^2$ für Wasserstoff stehen, $R^3$ $COR^6$ bedeutet, wobei $R^6$ Niederalkyl ist, und $R^4$ für $CH_2OH$ steht, mit 2,2-Dimethoxypropan zur Bildung einer Verbindung der Formel I, in welcher R, $R^2$ und $R^3$ wie vorstehend angegeben definiert sind, $R^4$ für $CH_2OR^8$ steht und $R^1$ und $R^8$ zusammen eine Gruppe der Formel

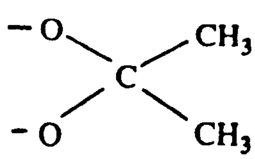

bilden.

**Patentansprüche für folgende Vertragsstaaten : ES, GR**

1. Verfahren zur Herstellung einer Verbindung der Formel I

$RCH(OR^1)CH(NR^2R^3)R^4$ I

in welcher R

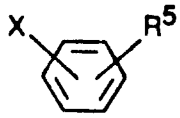 , 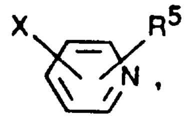 , 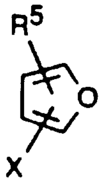 , 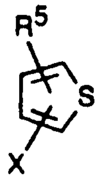 , 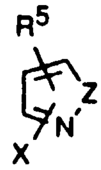 oder 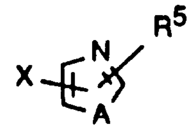

bedeutet, wobei $R^5$ $CH_3(CH_2)_mC{\equiv}C$, $CH_3(CH_2)_mCH=CH$, $CH_3(CH_2)_mCH_2CH_2$,

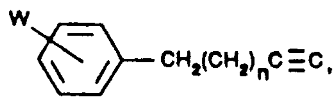 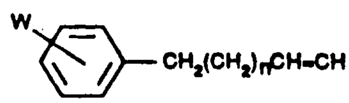 oder 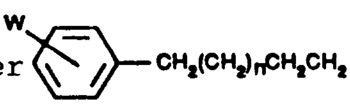

darstellt, wobei m für 3 bis 15, n für 0 bis 12 und X für Wasserstoff, Niederalkyl, Niederalkoxy, Halogen oder Trifluormethyl und Z für S, O oder C=O steht; und A für S oder O steht; $R^1$ Wasserstoff oder

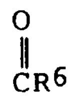

bedeutet, wobei $R^6$ für Wasserstoff, Niederalkyl, Niederalkoxy oder

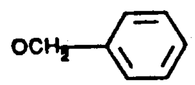

steht; $R^2$ Wasserstoff oder Niederalkyl bedeutet; $R^3$ Wasserstoff, Niederalkyl oder

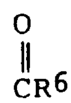

ist, wobei $R^6$ wie vorstehend angegeben definiert ist; $R^4$ für

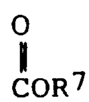

steht, wobei $R^7$ Wasserstoff, Niederalkyl oder $CH_2OR^8$ bedeutet, wobei $R^8$ Wasserstoff oder

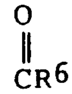

darstellt, wobei $R^6$ wie vorstehend angegeben definiert ist; $R^1$ und $R^8$ zusammen mit dem Sauerstoff, an den sie gebunden sind, eine Gruppe der Formel

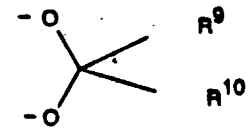

bilden, in welcher $R^9$ und $R^{10}$ unabhängig voneinander Wasserstoff oder Niederalkyl, deren optische Isomere oder deren pharmazeutisch verträgliche Salze darstellen, und wobei der Begriff "Nieder" sich auf eine Gruppe mit bis zu und einschließlich 8 Kohlenstoffatomen bezieht, das folgende Schritte umfaßt:

a) Umsetzen einer Verbindung der Formel 4a

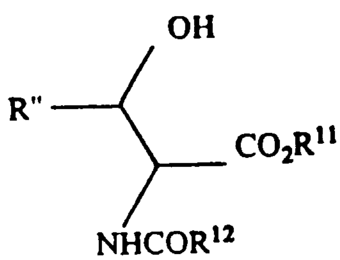

4a

in welcher R''

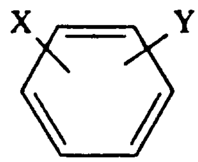 , 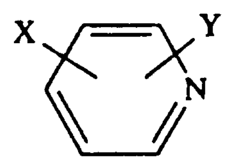 , 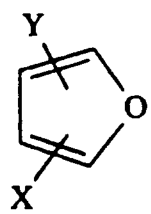 ,

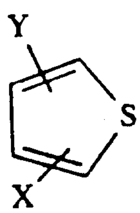 , 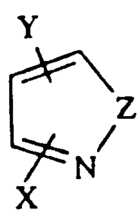 oder 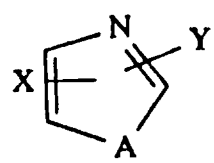

bedeutet, wobei X wie vorstehend angegeben definiert ist und Y ein Halogen darstellt, mit einem Alkyn der Formel $CH_3(CH_2)_mC{\equiv}CH$ oder

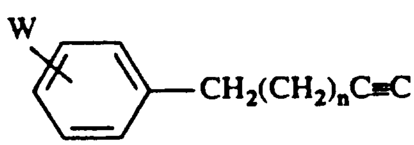 ,

wobei W, m und n wie vorstehend angegeben definiert sind, zur Bildung einer Verbindung der Formel I, in welcher R die weiter oben angewiesene Bedeutung zukommt, $R^5$ für $CH_3(CH_2)_mC{\equiv}C$ oder

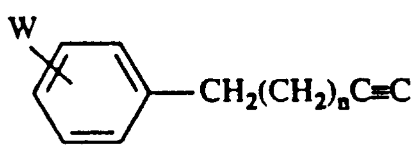

steht, $R^1$ und $R^2$ Wasserstoff und $R^3$ $COR^6$ darstellen, wobei $R^6$ Niederalkyl ist, und $R^4$ $CO_2R^7$ entspricht, wobei $R^7$ Niederalkyl ist, oder
b) Umsetzen einer Verbindung der Formel 10a

RCHO

in welcher R wie vorstehend angegeben definiert ist, wobei $R^5$ für $CH_3(CH_2)_mC{\equiv}C$ oder

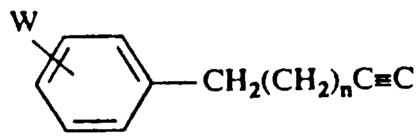

steht, mit einer Verbindung nach Formel 3

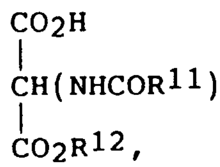

in welcher $R^{11}$ und $R^{12}$ für Niederalkyl stehen, zur Bildung einer Verbindung der Formel I, wie sie nach dem vorstehend beschriebenen Schritt a) erhalten wird,
c) wahlweise Reduzieren einer Verbindung der Formel I, wie sie nach dem vorstellend beschriebenen Schritt a) oder b) erhalten wird, durch Einsatz eines Alkaliborhydrids zur Bildung einer Verbindung der Formel I, in welcher R die weiter oben angewiesene Bedeutung zukommt und $R^5$ für $CH_3(CH_2)_mC{\equiv}C$ oder

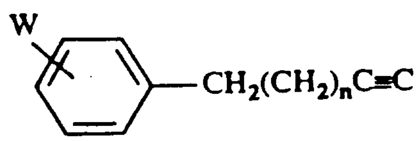

steht, $R^1$ und $R^2$ für Wasserstoff, $R^3$ für $COR^6$ mit $R^6$ als Niederalkyl und $R^4$ für $CH_2OH$ stehen,
d) wahlweise Hydrogenieren einer Verbindung der Formel I, wie sie in Schritt a), b) oder c) erhalten wurde, zur Bildung einer Verbindung der Formel I, in welcher R die weiter oben angewiesene Bedeutung zukommt, und $R^5$ für $CH_3(CH_2)_mCH_2CH_2$ oder

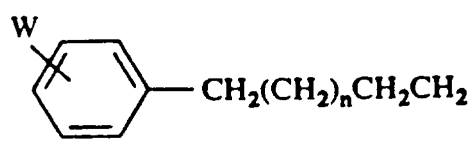

steht, $R^1$ und $R^2$ Wasserstoff darstellen und $R^3$ für $COR^6$ steht, wobei $R^6$ Niederalkyl ist, und $R^4$ $CO_2R^7$ bedeutet, wobei $R^7$ Niederalkyl ist, oder $R^4$ $CH_2OH$ bedeutet,
e) wahlweise Reduzieren einer Verbindung der Formel I, in welcher R wie vorstehend angegeben definiert ist, wobei $R^5$ $CH_3(CH_2)_mCH_2CH_2$ oder

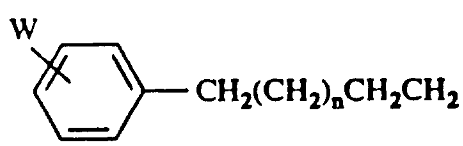

bedeutet und $R^1$ und $R^2$ Wasserstoff sind, $R^3$ für $COR^6$ steht, wobei $R^6$ Niederalkyl ist, und $R^4$ $CO_2R^7$ bedeutet, wobei $R^7$ Niederalkyl ist, mit Hilfe eines Alkaliborhydrids zur Bildung einer Verbindung der Formel I, in welcher R, $R^5$, $R^1$, $R^3$ und $R^2$ wie vorstehend beschrieben definiert sind und $R^4$ für $CH_2OH$ steht,

f) wahlweise Hydrogenieren einer Verbindung der Formel I, in welcher R wie vorstehend beschrieben definiert ist, wobei $R^5$ für $CH_3(CH_2)_nC{\equiv}C$ oder

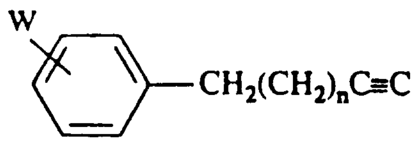

steht, $R^1$ und $R^2$ für Wasserstoff, $R^3$ für $COR^6$, wobei $R^6$ Niederalkyl ist, und $R^4$ für $CH_2OH$ stehen, zur Bildung einer Verbindung der Formel I, in welcher R, $R^1$, $R^2$, $R^3$ und $R^4$ wie vorstehend beschrieben definiert sind und $R^5$ für $CH_3(CH_2)_nCH=CH$ oder

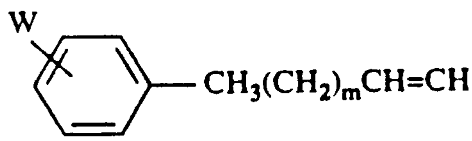

steht,

g) wahlweise Hydrolyse einer Verbindung der Formel I, in welcher R wie vorstehend angegeben definiert ist, $R^1$ und $R^2$ Wasserstoff sind, $R_3$ für $COR_6$ steht, wobei $R_6$ Niederalkyl ist und $R^4$ $CH_2OH$ entspricht, zur Bildung einer Verbindung Formel I, in welcher R, $R^5$, $R^1$, $R^2$ und $R^4$ wie vorstehend beschrieben definiert sind und $R^3$ Wasserstoff bedeutet,

h) wahlweise Umsetzen einer Verbindung der Formel I, in welcher R wie vorstehend angegeben definiert ist, $R^1$, $R^2$ und $R^3$ Wasserstoff sind, und $R^4$ für $CH_2OH$ steht, mit einem Niederaldehyd in Gegenwart eines Reduktionsmittel zur Bildung einer Verbindung der Formel I, in welcher R und $R^5$ die weiter oben angewiesene Bedeutung zukommt, $R^1$ für Wasserstoff, $R^4$ für $CH_2OH$ und $R^2$ und $R^3$ für Niederalkyl stehen,

i) wahlweise Umsetzen einer Verbindung der Formel I, in welcher R wie vorstehend angegeben definiert ist, $R^1$, $R^2$ und $R^3$ für Wasserstoff und $R^4$ für $-CH_2OH$ stehen, mit N-Benzyloxycarbonyloxy-succinimid der Formel 20

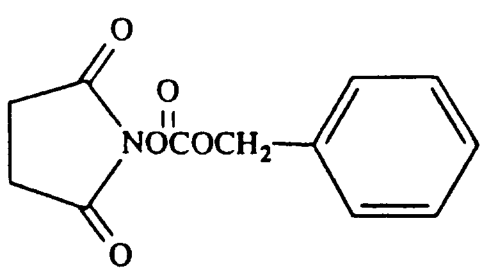

zur Bildung einer Verbindung der Formel I, in welcher R und $R^5$ wie vorstehend angegeben definiert sind, $R^1$ und $R^3$ für Benzyloxycarbonyl und $R^4$ für $CH_2OR^8$ stehen, wobei $R^8$ Benzyloxycarbonyl bedeutet,

j) wahlweise Umsetzen einer Verbindung der Formel I, in welcher R wie vorstehend angegeben definiert ist, $R^1$ und $R^2$ für Wasserstoff, $R_3$ für $COR_6$ mit $R_6$ als Niederalkyl und $R_4$ für $CH_2OH$ stehen,

k) wahlweise Acylieren einer Verbindung der Formel I, in welcher R wie vorstehend angegeben definiert ist, $R^1$ und $R^2$ für Wasserstoff stehen, $R_3$ $COR_6$ bedeutet, wobei $R_6$ Niederalkyl ist, und $R^4$ $CH_2OH$ entspricht, zur Bildung einer Verbindung der Formel I, in welcher R, $R^5$, $R^1$, $R^2$ und $R^3$ wie vorstehend beschrieben definiert sind und $R^4$ für $CH_2OCOR^6$ steht, wobei $R^6$ Niederalkyl ist,

l) wahlweise Umsetzen einer Verbindung der Formel I, in welcher R wie vorstehend angegeben definiert ist, $R^1$ und $R^2$ für Wasserstoff stehen, $R^3$ $COR^6$ bedeutet, wobei $R^6$ Niederalkyl ist, und $R^4$ für $CH_2OH$ steht, mit 2,2-Dimethoxypropan zur Bildung einer Verbindung der Formel I, in welcher R, $R^2$ und $R^3$ wie vorstehend angegeben definiert sind, $R^4$ für $CH_2OR^8$ steht und $R^1$ und $R^8$ zusammen eine Gruppe der Formel

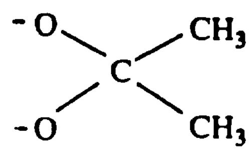

bilden.

**2.** Verfahren gemäß Anspruch 1, wobei R

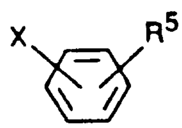 , 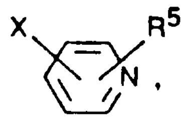 , 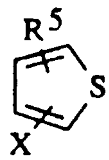 oder 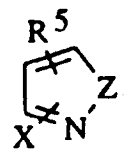

darstellt, wobei $R^5$ wie vorstehend angegeben definiert ist, X für Wasserstoff und Z für O steht.

**3.** Verfahren gemäß Anspruch 2, wobei
R

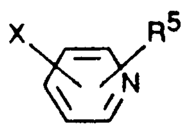 oder 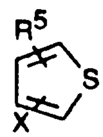

bedeutet, wobei X für Wasserstoff und $R^5$ für $CH_3(CH_2)_mC{\equiv}C$ oder $CH_3(CH_2)_mCH_2CH_2$ oder

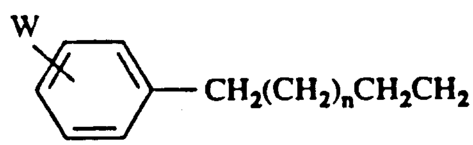

steht, wobei
m und n wie vorstehend angegeben definiert sind,
$R^1$ Wasserstoff oder

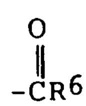

bedeutet, wobei $R^6$ für Niederalkyl steht,
$R^2$ Wasserstoff ist,
$R^3$ Wasserstoff oder

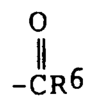

bedeutet, wobei $R^6$ wie vorstehend angegeben definiert ist, und $R^4$ die weiter oben angewiesene Bedeutung zukommt.

**4.** Verfahren gemäß Anspruch 3, wobei R

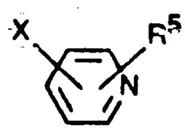

darstellt, wobei $R^1$, $R^2$ und $R^3$ fur Wasserstoff stehen und $R^5$ für $CH_3(CH_2)_mCH_2CH_2$ oder

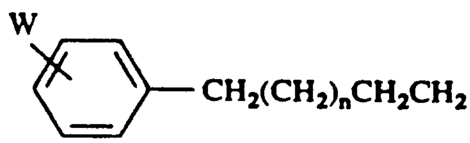

steht, wobei W und X Wasserstoff darstellen und m und n wie vorstehend angegeben definiert sind.

**5.** Verfahren gemäß Anspruch 1, bei dem Erythro-2-amino-1-(6-decyl-2-pyridinyl)-1,3-dihydroxypropan oder ein pharmazeutisch verträgliches Salz davon hergestellt wird.

**6.** Verfahren gemäß Anspruch 1, bei dem Ethylerythro-2-acetamido-3-[6-(1-dodecynyl)-2-pyridinyl]-3-hydroxypropionat oder ein pharmazeutisch verträgliches Salz davon hergestellt wird.

**7.** Verfahren gemäß Anspruch 1, bei dem Erythro-2-amino-1-(6-dodecyl-2-pyridinyl)-1,3-propandiol oder ein pharmazeutisch verträgliches Salz davon hergestellt wird.

**8.** Verfahren gemäß Anspruch 1, bei dem Erythro-N-{1-[6-(1-dodecynyl)-2-pyridinyl]-1,3-dihydroxy-2-propanyl}acetamid oder ein pharmazeutisch verträgliches Salz davon hergestellt wird.

**9.** Verfahren gemäß Anspruch 1, bei dem Erythro-2-amino-1-[5-(1-dodecynyl)2-thienyl]-1,3-propandiol oder ein pharmazeutisch verträgliches Salz davon hergestellt wird.

**10.** Verfahren gemäß Anspruch 1, bei dem Erythro-N-{1-[6-(1-decynyl)-2-pyridinyl]-1,3-dihydroxy-2-propanyl}acetamid oder ein pharmazeutisch verträgliches Salz davon hergestellt wird.

**11.** Verwendung einer Verbindung gemäß Anspruch 1 zur Herstellung eines Medikaments zur Erleichterung von Gedächtnisstörungen, mit entzündungshemmender, die Zellproliferation verringernder, antibakterieller und/oder antimykotischer Wirksamkeit.

**12.** Verfahren der Formel 1a

R'CHO

in welcher R'

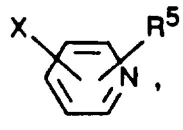 , 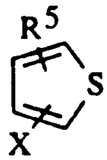 oder 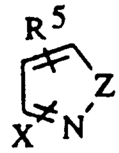

bedeutet, wobei $R^5$ $CH_3(CH_2)_mC{\equiv}C$, $CH_3(CH_2)_mCH{=}CH$, $CH_3(CH_2)_mCH_2CH_2$ oder

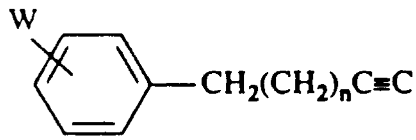

darstellt, wobei m für 3 bis 15, n für 0 bis 12, und W und X unabhängig voneinander für Wasserstoff, Niederalkyl, Niederalkoxy, Halogen oder Trifluormethyl und Z für O stehen; der Begriff "Nieder" bezeichnet eine Gruppe mit bis zu und einschließlich 8 Kohlenstoffatomen.

## Revendications

**Revendications pour les Etats contractants suivants : AT, BE, CH, DE, DK, FR, GB, IT, LI, LU, NL, SE**

**1.** Composé de formule I

$RCH(OR^1)CH(NR^2R^3)R^4$ I

dans laquelle R est

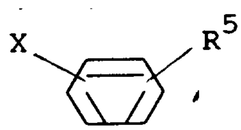 ,

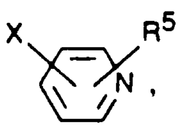 , 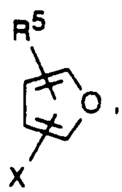 , 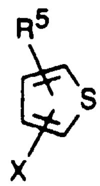 , 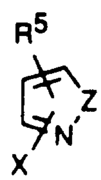 , ou 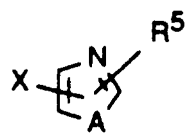

$R^5$ étant $CH_3(CH_2)_mC{\equiv}C$, $CH_3(CH_2)_mCH{=}CH$, $CH_3(CH_2)_mCH_2CH_2$,

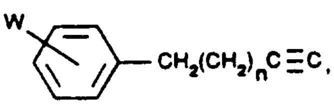 , 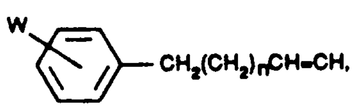 ou 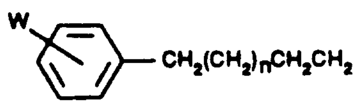

m allant de 3 à 15, n allant de 0 à 12 et X étant un atome d'hydrogène ou d'halogène ou un groupe alkyle inférieur, alcoxy inférieur ou trifluorométhyle, et Z étant S, O ou C=O; et A étant S ou O; $R^1$ est un atome d'hydrogène ou

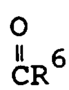 ,

$R^6$ étant un atome d'hydrogène ou un groupe alkyle inférieur, alcoxy inférieur ou

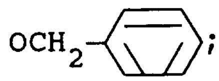

$R^2$ est un atome d'hydrogène ou un groupe alkyle inférieur; $R^3$ est un atome d'hydrogène, un groupe alkyle inférieur ou

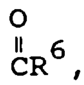

$R^6$ étant tel que défini plus haut, $R^4$ est

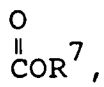

$R^7$ étant un atome d'hydrogène ou un groupe alkyle inférieur ou $CH_2OR^8$ , $R^8$ étant un atome d'hydrogène ou

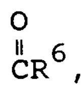

$R^6$ étant tel que défini plus haut; $R^1$ et $R^8$ forment ensemble, avec l'atome d'oxygène sur lequel ils sont fixés, un groupe de formule

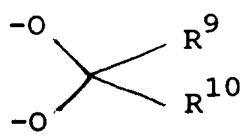

dans laquelle $R^9$ et $R^{10}$ sont indépendamment un atome d'hydrogène ou un groupe alkyle inférieur; isomères optiques de celui-ci, ou sels pharmaceutiquement acceptables de celui-ci, le terme d'inférieur désignant un groupe contenant jusqu'à et y compris 8 atomes de carbone.

2. Composé selon la revendication 1, dans lequel R est

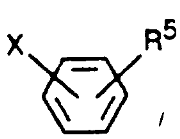 , 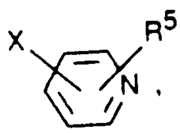 , 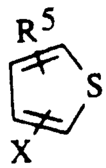 ou 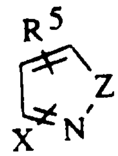 ,

$R^5$ étant tel que défini, X étant un atome d'hydrogène et Z étant O.

3. Composé selon la revendication 2, dans lequel R est

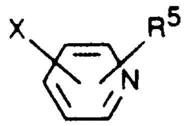 ou 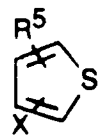 ,

X étant un atome d'hydrogène et $R^5$ étant $CH_3(CH_2)_mC{\equiv}C$ ou $CH_3(CH_2)_mCH_2CH_2$ ou

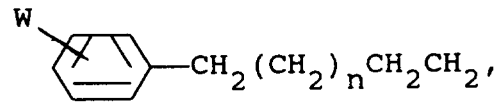

m et n étant tels que définis,

$R^1$ est un atome d'hydrogène ou un groupe

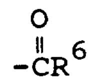

dans lequel $R^6$ est un groupe alkyle inférieur,

$R^2$ est un atome d'hydrogène,

$R^3$ est un atome d'hydrogène ou un groupe

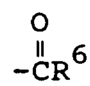

dans lequel $R^6$ est tel que défini plus haut, et

$R^4$ est est tel que défini.

**4.** Composé selon la revendication 3, dans lequel R est

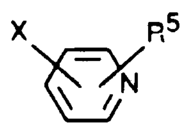 ,

$R^1$, $R^2$ et $R^3$ sont des atomes d'hydrogène et $R^5$ est $CH_3(CH_2)_mCH_2CH_2$ ou

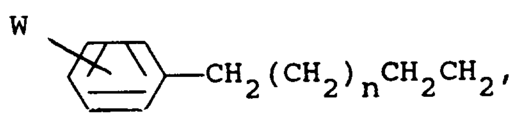

W et X étant des atomes d'hydrogène et m et n étant tels que définis.

**5.** Composé selon la revendication 1, qui est l'érythro-2-amino-1-(6-décyl-2-pyridinyl)-1,3-dihydroxypropane ou un sel pharmaceutiquement acceptable de celui-ci.

**6.** Composé selon la revendication 1, qui est l'érythro-2-acétamido-3-[6-(1-dodécynyl)-2-pyridinyl]-3-hydroxypropionate d'éthyle ou un sel pharmaceutiquement acceptable de celui-ci.

**7.** Composé selon la revendication 1, qui est l'érythro-2-amino-1-(6-dodécyl-2-pyridinyl)-1,3-propanediol ou un sel pharmaceutiquement acceptable de celui-ci.

**8.** Composé selon la revendication 1, qui est l'érythro-N-{1-[6-(1-dodécynyl)-2-pyridinyl]-1,3-dihydroxy-2-propanyl}acétamide ou un sel pharmaceutiquement acceptable de celui-ci.

**9.** Composé selon la revendication 1, qui est l'érythro-2-amino-1-[5-(1-dodécynyl)-2-thiényl]-1,3-propanediol ou un sel pharmaceutiquement acceptable de celui-ci.

**10.** Composé selon la revendication 1, qui est l'érythro-N-{1-[6-(1-décynyl)-2-pyridinyl]-1,3-dihydroxy-2-propanyl]acétamide ou un sel pharmaceutiquement acceptable de celui-ci.

**11.** Composition pharmaceutique comprenant, en tant que composant actif, un composé selon la revendication 1 et un adjuvant approprié.

**12.** Utilisation d'un composé selon la revendication 1, pour la fabrication d'un médicament ayant une activité faisant disparaître des dysfonctionnements de la mémoire, anti-inflammatoire, de diminution de la prolifération cellulaire, antibactérienne et/ou antifongique.

**13.** Composé de formule

R'CHO

dans laquelle R' est

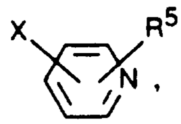 , 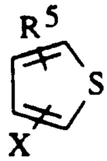 ou 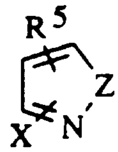 ,

$R^5$ étant $CH_3(CH_2)_mC{\equiv}C$, $CH_3(CH_2)_mCH=CH$, $CH_3(CH_2)_mCH_2CH_2$ ou

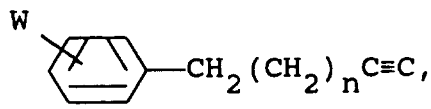

m allant de 3 à 15, n allant de 0 à 12, W et X étant indépendamment un atome d'hydrogène ou d'halogène ou un groupe alkyle inférieur, alcoxy inférieur ou trifluorométhyle, et Z étant O, le terme d'inférieur désignant un groupe contenant jusqu'à 8 atomes de carbone.

**14.** Procédé pour la préparation d'un composé selon la revendication 1, comprenant

a) la mise en réaction d'un composé de formule 4a

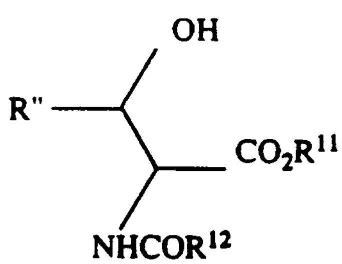

4a

dans laquelle R'' est

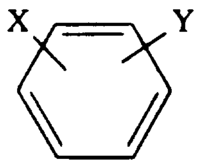 , 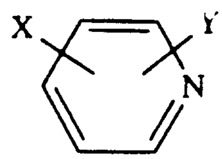 , 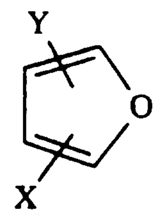 , 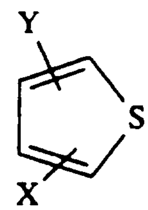 ,

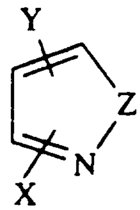 ou 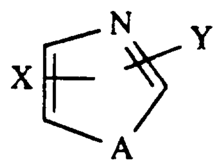

X étant tel que défini et Y étant un atome d'halogène, avec un alcyne de formule $CH_3(CH_2)_mC{\equiv}CH$ ou

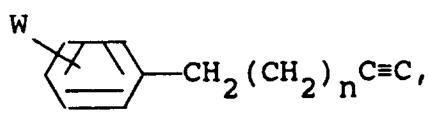

W, m et n étant tels que définis, pour l'obtention d'un composé de formule I dans laquelle R est tel que défini, $R^5$ étant $CH_3(CH_2)_mC{\equiv}C$ ou

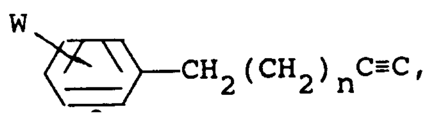

$R^1$ et $R^2$ sont des atomes d'hydrogène, $R^3$ est $COR^6$, $R^6$ étant un groupe alkyle inférieur et $R^4$ est $CO_2R^7$, $R^7$ étant un groupe alkyle inférieur, ou
b) la mise en réaction d'un composé de formule 10a

RCHO

dans laquelle R est tel que défini, $R^5$ étant $CH_3(CH_2)_mC{\equiv}C$ ou

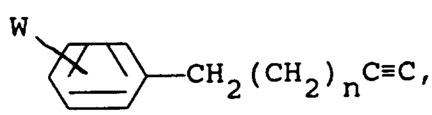

avec un composé de formule 3

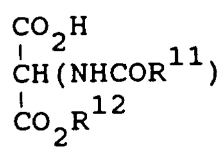

dans laquelle $R^{11}$ et $R^{12}$ sont des groupes alkyle inférieur, pour la formation d'un composé de formule I tel qu'obtenu dans l'étape a) ci-dessus,
c) éventuellement la réduction d'un composé de formule I tel qu'obtenu dans l'étape a) ou b) ci-dessus, au moyen d'un borohydrure de métal alcalin, pour la formation d'un composé de formule I dans laquelle R est tel que défini, $R^5$ étant $CH_3(CH_2)_mC{\equiv}C$ ou

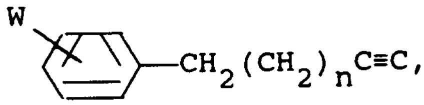

$R^1$ et $R^2$ sont des atomes d'hydrogène, $R^3$ est $COR^6$, $R^6$ étant un groupe alkyle inférieur, et $R^4$ est $CH_2OH$,

d) éventuellement l'hydrogénation d'un composé de formule I tel qu'obtenu dans l'étape a), b) ou c), pour l'obtention d'un composé de formule I dans lequel R est tel que défini, $R^5$ étant $CH_3(CH_2)_mCH_2CH_2$ ou

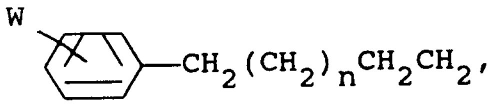

$R^1$ et $R^2$ sont des atomes d'hydrogène, $R^3$ est $COR^6$, $R^6$ étant un groupe alkyle inférieur et $R^4$ est $CO_2R^7$, $R^7$ étant un groupe alkyle inférieur, ou $R^4$ est $CH_2OH$,

e) éventuellement la réduction d'un composé de formule I dans lequel R est tel que défini, $R^5$ étant $CH_3(CH_2)_mCH_2CH_2$ ou

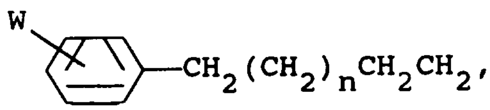

$R^1$ et $R^2$ sont des atomes d'hydrogène, $R^3$ est $COR^6$, $R^6$ étant un groupe alkyle inférieur, et $R^4$ est $CO_2R^7$ , $R^7$ étant un groupe alkyle inférieur, au moyen d'un borohydrure de métal alcalin, pour l'obtention d'un composé de formule I dans lequel R, $R^5$, $R^1$, $R^3$ et $R^2$ sont tels que décrits précédemment et $R^4$ est $CH_2OH$,

f) éventuellement l'hydrogénation d'un composé de formule I dans lequel R est tel que défini, $R^5$ étant $CH_3(CH_2)_nC{\equiv}C$ ou

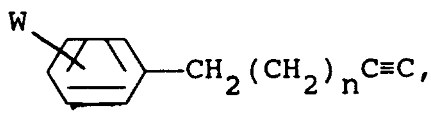

$R^1$ et $R^2$ sont des atomes d'hydrogène, $R^3$ est $COR^6$, $R^6$ étant un groupe alkyle inférieur, et $R^4$ est $CH_2OH$, pour la formation d'un composé de formule I dans lequel R, $R^1$, $R^2$, $R^3$ et $R^4$ sont tels que décrits précédemment et $R^5$ est $CH_3(CH_2)_nCH=CH$ ou

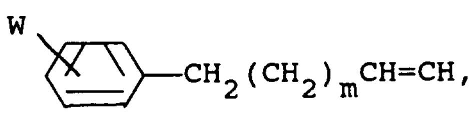

g) éventuellement l'hydrolyse d'un composé de formule I dans lequel R est tel que défini, $R^1$ et $R^2$ sont des atomes d'hydrogène, $R^3$ est $COR^6$, $R^6$ étant un groupe alkyle inférieur, et $R^4$ est $CH_2OH$, pour la formation d'un composé de formule I dans lequel R, $R^5$, $R^1$, $R^2$ et $R^4$ sont tels que décrits précédemment et $R^3$ est un atome d'hydrogène,

h) éventuellement la mise en réaction d'un composé de formule I dans lequel R est tel que défini, $R^1$, $R^2$ et $R^3$ sont des atomes d'hydrogène et $R^4$ est $CH_2OH$, avec un aldéhyde inférieur en présence d'un réducteur, pour l'obtention d'un composé de formule I dans lequel R et $R^5$ sont tels que définis, $R^1$ est un atome d'hydrogène, $R^4$ est $CH_2OH$ et $R^2$ et $R^3$ sont des groupes alkyle inférieur,

i) éventuellement la mise en réaction d'un composé de formule I dans lequel R est tel que défini, $R^1$, $R^2$ et $R^3$ sont des atomes d'hydrogène et $R^4$ est $-CH_2OH$, avec le N-benzyloxycarbonyloxy-succinimide de formule 20

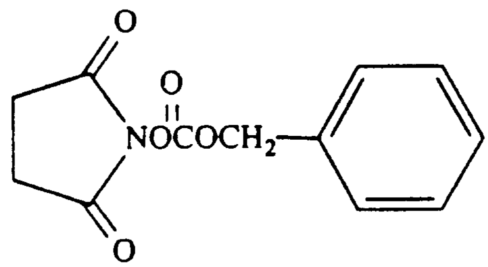

pour la formation d'un composé de formule I dans lequel R et $R^5$ sont tels que définis, $R^1$ et $R^3$ sont le groupe benzyloxycarbonyle et $R^4$ est $CH_2OR^8$, $R^8$ étant le groupe benzyloxycarbonyle,
j) éventuellement la mise en réaction d'un composé de formule I dans lequel R est tels que défini, $R^1$ et $R^2$ sont des atomes d'hydrogène, $R^3$ est $COR^6$ , $R^6$ étant un groupe alkyle inférieur, et $R^4$ est $CH_2OH$,
k) éventuellement l'acylation d'un composé de formule I dans lequel R est tel que défini, $R^1$ et $R^2$ sont des atomes d'hydrogène, $R^3$ est $COR^6$, $R^6$ étant un groupe alkyle inférieur, et $R^4$ est $CH_2OH$, pour l'obtention d'un composé de formule I dans lequel R, $R^5$, $R^1$ $R^2$ et $R^3$ sont tels que définis précédemment, et $R^4$ est $CH_2OCOR^6$ , $R^6$ etant un groupe alkyle inférieur,
l) éventuellement la mise en réaction d'un composé de formule I dans lequel R est tel que défini, $R^1$ et $R^2$ sont des atomes d'hydrogène, $R^3$ est $COR^6$, $R^6$ étant un groupe alkyle inférieur, $R^4$ est $CH_2OH$, avec du 2,2-diméthoxypropane, pour l'obtention d'un composé de formule I dans lequel R, $R^2$ et $R^3$ sont tels que définis, $R^4$ est $CH_2OR^8$ et $R^1$ et $R^8$, considérés ensemble, forment un groupe de formule

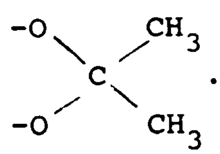 .

**Revendications pour les Etats contractants suivants : ES, GR**

**1.** Procédé pour la préparation d'un composé de formule I

$RCH(OR^1)CH(NR^2R^3)R^4$ I

dans laquelle R est

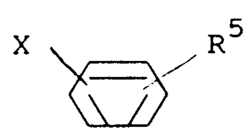

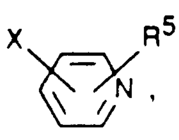 , 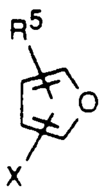 , 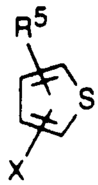 , 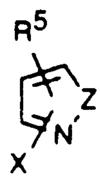 , ou 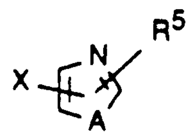 .

$R^5$ étant $CH_3(CH_2)_mC{\equiv}C$, $CH_3(CH_2)_mCH=CH$, $CH_3(CH_2)_mCH_2CH_2$,

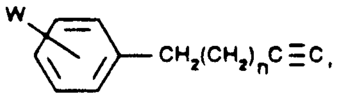, 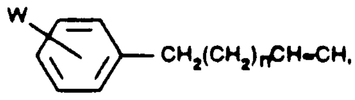 ou 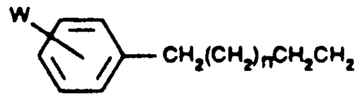

m allant de 3 à 15, n allant de 0 à 12 et X étant un atome d'hydrogène ou d'halogène ou un groupe alkyle inférieur, alcoxy inférieur ou trifluorométhyle, et Z étant S, O ou C=O; et A étant S ou O; $R^1$ est un atome d'hydrogène ou

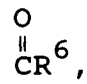

$R^6$ étant un atome d'hydrogène ou un groupe alkyle inférieur, alcoxy inférieur ou

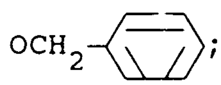

$R^2$ est un atome d'hydrogène ou un groupe alkyle inférieur; $R^3$ est un atome d'hydrogène, un groupe alkyle inférieur ou

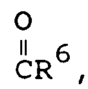

$R^6$ étant tel que défini plus haut, $R^4$ est

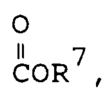

$R^7$ étant un atome d'hydrogène ou un groupe alkyle inférieur ou $CH_2OR^8$ , $R^8$ étant un atome d'hydrogène ou

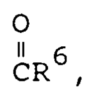

$R^6$ étant tel que défini plus haut; $R^1$ et $R^8$ forment ensemble, avec l'atome d'oxygène sur lequel ils sont fixés, un groupe de formule

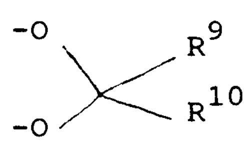

dans laquelle $R^9$ et $R^{10}$ sont indépendamment un atome d'hydrogène ou un groupe alkyle inférieur; de ses isomères optiques ou de ses sels pharmaceutiquement acceptables, le terme d'inférieur désignant un groupe contenant jusqu'à et y compris 8 atomes de carbone, comprenant.

a) la mise en réaction d'un composé de formule 4a

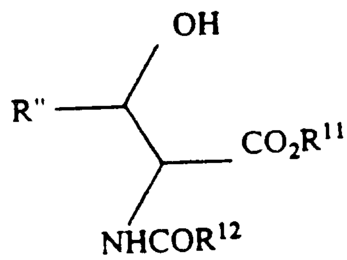

4a

dans laquelle R'' est

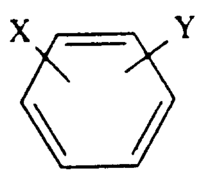
,
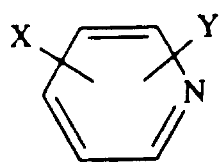
,
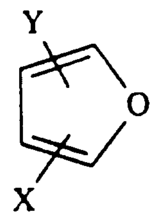
,
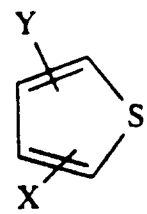
,

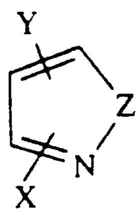
ou
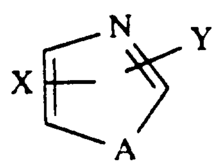

X étant tel que défini et Y étant un atome d'halogène, avec un alcyne de formule $CH_3(CH_2)_mC{\equiv}CH$ ou

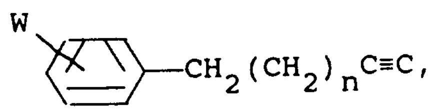

W, m et n étant tels que définis, pour l'obtention d'un composé de formule I dans laquelle R est tel que défini, $R^5$ étant $CH_3(CH_2)_mC{\equiv}C$ ou

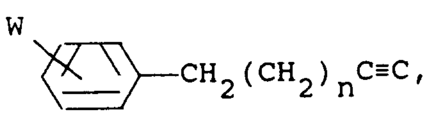

$R^1$ et $R^2$ sont des atomes d'hydrogène, $R^3$ est $COR^6$, $R^6$ étant un groupe alkyle inférieur et $R^4$ est $CO_2R^7$ , $R^7$ étant un groupe alkyle inférieur, ou

b) la mise en réaction d'un composé de formule 10a

RCHO

dans laquelle R est tel que défini, $R^5$ étant $CH_3(CH_2)_mC{\equiv}C$ ou

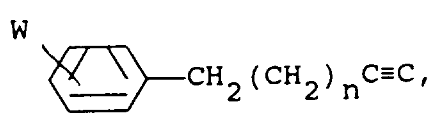

avec un composé de formule 3

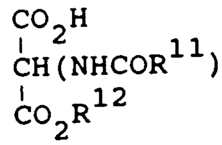

dans laquelle $R^{11}$ et $R^{12}$ sont des groupes alkyle inférieur, pour la formation d'un composé de formule I tel qu'obtenu dans l'étape a) ci-dessus,

c) éventuellement la réduction d'un composé de formule I tel qu'obtenu dans l'étape a) ou b) ci-dessus, au moyen d'un borohydrure de métal alcalin, pour la formation d'un composé de formule I dans laquelle R est tel que défini, $R^5$ étant $CH_3(CH_2)_mC{\equiv}C$ ou

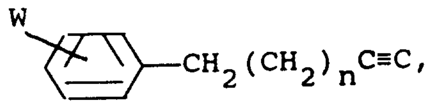

$R^1$ et $R^2$ sont des atomes d'hydrogène, $R^3$ est $COR^6$, $R^6$ étant un groupe alkyle inférieur, et $R^4$ est $CH_2OH$,

d) éventuellement l'hydrogénation d'un composé de formule I tel qu'obtenu dans l'étape a), b) ou c), pour l'obtention d'un composé de formule I dans lequel R est tel que défini, $R^5$ étant $CH_3(CH_2)_mCH_2CH_2$ ou

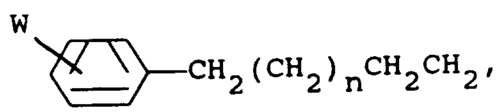

$R^1$ et $R^2$ sont des atomes d'hydrogène, $R^3$ est $COR^6$, $R^6$ étant un groupe alkyle inférieur et $R^4$ est $CO_2R^7$ , $R^7$ étant un groupe alkyle inférieur, ou $R^4$ est $CH_2OH$,

e) éventuellement la réduction d'un composé de formule I dans lequel R est tel que défini, $R^5$ étant $CH_3(CH_2)_mCH_2CH_2$ ou

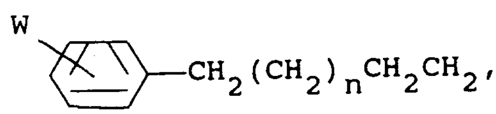

$R^1$ et $R^2$ sont des atomes d'hydrogène, $R^3$ est $COR^6$, $R^6$ étant un groupe alkyle inférieur, et $R^4$ est $CO_2R^7$ , $R^7$ étant un groupe alkyle inférieur, au moyen d'un borohydrure de métal alcalin, pour l'obtention d'un composé de formule I dans lequel R, $R^5$, $R^1$, $R^2$ et $R^3$ sont tels que décrits précédemment et $R^4$ est $CH_2OH$,

f) éventuellement l'hydrogénation d'un composé de formule I dans lequel R est tel que défini, $R^5$ étant $CH_3(CH_2)_nC{\equiv}C$ ou

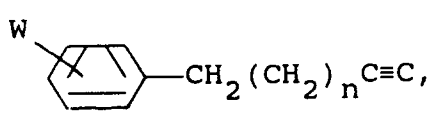

$R^1$ et $R^2$ sont des atomes d'hydrogène, $R^3$ est $COR^6$, $R^6$ étant un groupe alkyle inférieur, et $R^4$ est $CH_2OH$, pour la formation d'un composé de formule I dans lequel R, $R^1$, $R^2$, $R^3$ et $R^4$ sont tels que décrits précédemment et $R^5$ est $CH_3(CH_2)_nCH=CH$ ou

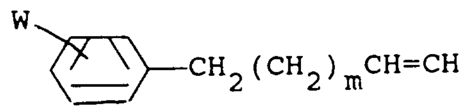

g) éventuellement l'hydrolyse d'un composé de formule I dans lequel R est tel que défini, $R^1$ et $R^2$ sont des atomes d'hydrogène, $R^3$ est $COR^6$, $R^6$ étant un groupe alkyle inférieur, et $R^4$ est $CH_2OH$, pour la formation d'un composé de formule I dans lequel R, $R^5$, $R^1$, $R^2$ et $R^4$ sont tels que décrits précédemment et $R^3$ est un atome d'hydrogène,

h) éventuellement la mise en réaction d'un composé de formule I dans lequel R est tel que défini, $R^1$, $R^2$ et $R^3$ sont des atomes d'hydrogène et $R^4$ est $CH_2OH$, avec un aldéhyde inférieur en présence d'un réducteur, pour l'obtention d'un composé de formule I dans lequel R et $R^5$ sont tels que définis, $R^1$ est un atome d'hydrogène, $R^4$ est $CH_2OH$ et $R^2$ et $R^3$ sont des groupes alkyle inférieur,

i) éventuellement la mise en réaction d'un composé de formule I dans lequel R est tel' que défini, $R^1$, $R^2$ et $R^3$ sont des atomes d'hydrogène et $R^4$ est $-CH_2OH$, avec le N-benzyloxycarbonyloxy-succinimide de formule 20

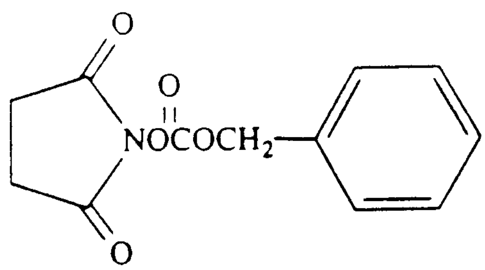

pour la formation d'un composé de formule I dans lequel R et $R^5$ sont tels que définis, $R^1$ et $R^3$ sont le groupe benzyloxycarbonyle et $R^4$ est $CH_2OR^8$ $R^8$ étant le groupe benzyloxycarbonyle,

j) éventuellement la mise en réaction d'un composé de formule I dans lequel R est tels que défini, $R^1$ et $R^2$ sont des atomes d'hydrogène, $R^3$ est $COR^6$ $R^6$ étant un groupe alkyle inférieur, et $R^4$ est $CH_2OH$,

k) éventuellement l'acylation d'un composé de formule I dans lequel R est tel que défini, $R^1$ et $R^2$ sont des atomes d'hydrogène, $R^3$ est $COR^6$, $R^6$ étant un groupe alkyle inférieur, et $R^4$ est $CH_2OH$, pour l'obtention d'un composé de formule I dans lequel R, $R^5$, $R^1$, $R^2$ et $R^3$ sont tels que définis précédemment, et $R^4$ est $CH_2OCOR^6$, $R^6$ étant un groupe alkyle inférieur,

l) éventuellement la mise en réaction d'un composé de formule I dans lequel R est tel que défini, $R^1$ et $R^2$ sont des atomes d'hydrogène, $R^3$ est $COR^6$, $R^6$ étant un groupe alkyle inférieur, $R^4$ est $CH_2OH$, avec du 2,2-diméthoxypropane, pour l'obtention d'un composé de formule I dans lequel R, $R^2$ et $R^3$ sont tels que définis, $R^4$ est $CH_2OR^8$ et $R^1$ et $R^8$, considérés ensemble, forment un groupe de formule

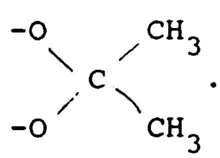

**2.** Procédé selon la revendication 1, dans lequel R est

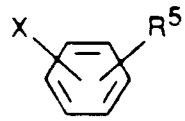 , 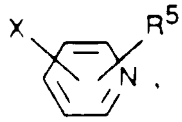 , 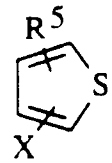 ou 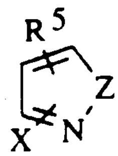 ,

$R^5$ étant tel que défini, X étant un atome d'hydrogène et Z étant O.

**3.** Procédé selon la revendication 2, dans lequel R est

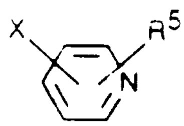 ou 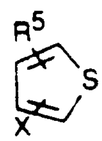 ,

X étant un atome d'hydrogène et $R^5$ étant $CH_3(CH_2)_mC{\equiv}C$ ou $CH_3(CH_2)_mCH_2CH_2$ ou

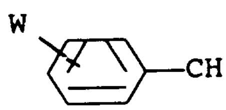 $CH_2(CH_2)_nCH_2CH_2$,

m et n étant tels que définis,
$R^1$ est un atome d'hydrogène ou un groupe

$$-\overset{\overset{O}{\|}}{C}R^6$$

dans lequel $R^6$ est un groupe alkyle inférieur,
$R^2$ est un atome d'hydrogène,
$R^3$ est un atome d'hydrogène ou un groupe

$$-\overset{\overset{O}{\|}}{C}R^6$$

dans lequel $R^6$ est tel que défini plus haut, et
$R^4$ est tel que défini.

**4.** Procédé selon la revendication 3, dans lequel R est

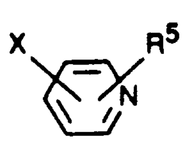 ,

, $R^1$, $R^2$ et $R^3$ sont des atomes d'hydrogène et $R^5$ est $CH_3(CH_2)_mCH_2CH_2$ ou

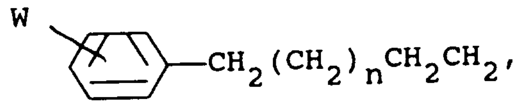

W et X étant des atomes d'hydrogène et m et n étant tels que définis.

5. Procédé selon la revendication 1, dans lequel on prépare de l'érythro-2-amino-1-(6-décyl-2-pyridinyl)-1,3-dihydroxypropane ou un sel pharmaceutiquement acceptable de celui-ci.

6. Procédé selon la revendication 1, dans lequel on prépare de l'érythro-2-acétamido-3-[6-(1-dodécynyl)-2-pyridinyl]-3-hydroxypropionate d'éthyle ou un sel pharmaceutiquement acceptable de celui-ci.

7. Procédé selon la revendication 1, dans lequel on prépare de l'érythro-2-amino-1-(6-dodécyl-2-pyridinyl)-1,3-propanediol ou un sel pharmaceutiquement acceptable de celui-ci.

8. Procédé selon la revendication 1, dans lequel on prépare de l'érythro-N-{1-[6(1-dodécynyl)-2-pyridinyl]-1,3-dihydroxy-2-propanyl}acétamide ou un sel pharmaceutiquement acceptable de celui-ci.

9. Procédé selon la revendication 1, dans lequel on prépare de l'érythro-2-amino-1-[5-(1-dodécynyl)-2-thiényl]-1,3-propanediol ou un sel pharmaceutiquement acceptable de celui-ci.

10. Procédé selon la revendication 1, dans lequel on prépare de l'érythro-N-{1-[6-(1-décynyl)-2-pyridinyl]-1,3-dihydroxy-2-propanyl]acétamide ou un sel pharmaceutiquement acceptable de celui-ci.

11. Utilisation d'un composé tel que défini dans la revendication 1, pour la fabrication d'un médicament ayant une activité faisant disparaître des dysfonctionnements de la mémoire, anti-inflammatoire, de diminution de la prolifération cellulaire, antibactérienne et/ou antifongique.

12. Composé de formule 1a

R'CHO

dans laquelle R' est

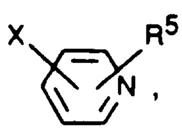 , 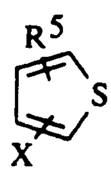 ou 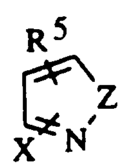 ,

$R^5$ étant $CH_3(CH_2)_mC{\equiv}C$, $CH_3(CH_2)_mCH{\equiv}CH$, $CH_3(CH_2)_mCH_2CH_2$ ou

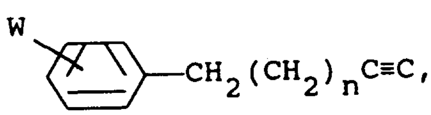

m allant de 3 à 15, n allant de 0 à 12, W et X étant indépendamment un atome d'hydrogène ou d'halogène ou un groupe alkyle inférieur, alcoxy inférieur ou triflourométhyle, et Z étant O.